# EUROPÄISCHE PATENTSCHRIFT

(11) **EP 0 973 752 B1**
(45) Veröffentlichungstag und Bekanntmachung des Hinweises auf die Patenterteilung: **11.05.2005**
(21) Anmeldenummer: 98908103.9
(22) Anmeldetag: 20.02.1998
(51) Int. Cl.: C07D 239/54, A01N 43/54, C07D 249/12, C07D 249/16

(54) **HETEROZYKLISCH SUBSTITUIERTE AROMATISCHE AMINOVERBINDUNGEN MIT HERBIZIDER WIRKUNG**
HETEROCYCLICALLY SUBSTITUTED AROMATIC AMINO COMPOUNDS WITH A HERBICIDAL EFFECT
COMPOSES AMINES AROMATIQUES SUBSTITUES A ACTION HERBICIDE

(30) Priorität: 05.03.1997 DE 19708928
(43) Veröffentlichungstag der Anmeldung: 26.01.2000
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); FINDEISEN, Kurt, D-51375 Leverkusen (DE); KLUTH, Joachim, D-40764 Langenfeld (DE); LINKER, Karl-Heinz, D-51377 Leverkusen (DE); MÜLLER, Klaus-Helmut, D-40593 Düsseldorf (DE); SCHALLNER, Otto, D-40789 Monheim (DE); DOLLINGER, Markus, D-51381 Leverkusen (DE)
(86) Internationale Anmeldenummer: PCT/EP1998/000972
(87) Internationale Veröffentlichungsnummer: WO 1998/039304

(56) Entgegenhaltungen:
- EP-A- 0 011 693
- EP-A- 0 255 047
- EP-A- 0 558 999
- WO-A-87/03782
- WO-A-93/11097
- WO-A-94/04511
- WO-A-94/14817
- WO-A-96/07323
- WO-A-96/16043
- WO-A-96/18618
- WO-A-97/05120
- JP-A- 6 345 743
- US-A- 3 922 162
- US-A- 4 213 773
- US-A- 4 249 934
- US-A- 4 906 286
- US-A- 5 084 084
- US-A- 5 108 486
- US-A- 5 136 868

## Beschreibung

Die Erfindung betrifft neue substituierte aromatische Aminoverbindungen. Verfahren zu ihrer Herstellung und ihre Verwendung als Herbizide.

Einige substituierte aromatische Aminoverbindungen, wie z.B. N-[2-(1,5-Dihydro-1-methyl-5-thioxo-3-trifluormethyl-4H-1,2,4-triazol-4-yl)-5-fluor-phenyl]-benzamid und N-[5-Chlor-2-(1,5-dihydro-1-methyl-5-thioxo-3-trifluormethyl-4H-1,2,4-triazol-4-yl)-phenyl]-acetamid sind bereits aus der Patentliteratur als potentielle Herbizide bekannt geworden (vgl. US 5108486). Diese Verbindungen haben jedoch keine besondere Bedeutung erlangt.

Weitere substituierte aromatische Aminoverbindungen, wie z.B. N-[5-Chlor-2-(2,5-dihydro-3,4-dimethyl-2,5-dioxo-1H-pyrrol-1-yl)-phenyl]-acetamid (vgl. Indian J. Chem, Sect. B, 29B (1990), 659-660 - zitiert in Chem. Abstracts 113:21 1906), sowie N-[2-(5-diethylamino-3-t-butyl-1H-1,2,4-triazol-1-yl)-5-trifluormethyl-phenyl]-2,2-dimethyl-propanamid und N-[2-(5-diethylamino-3-t-butyl-1H-1,2,4-triazol-1-yl)-5-trifluormethyl-phenyl]-acetamid (vgl. JP 02091062 - zitiert in Chem. Abstracts 113:97612) sind ebenfalls bereits bekannt. Über eine herbizide Wirksamkeit dieser Verbindungen ist jedoch nichts bekannt geworden.

Es wurden nun neue substituierte aromatische Aminoverbindungen der allgemeinen Formel (I), 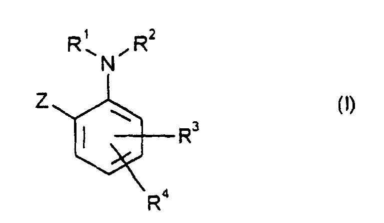 in welcher
- R¹: für Wasserstoff, Hydroxy, Amino, für Alkyl, Alkoxy, Alkylamino oder Dialkylamino mit jeweils 1 bis 6 Kohlenstoffatomen in den Alkylgruppen, oder für eine der nachstehenden Gruppierungen steht,

-CQ¹-R⁵, -CQ¹-Q²-R⁶,
- R²: für eine der nachstehenden Gruppierungen steht,

-CQ¹-R⁵, -CQ¹-Q²-R⁶,
- R³: für Wasserstoff, Hydroxy, Amino, Carboxy, Cyano, Carbamoyl, Thiocarbamoyl, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfmyl oder Alkylsulfonyl mit jeweils 1 bis 6 Kohlenstoffatomen in den Alkylgruppen, oder für eine der nachstehenden Gruppierungen steht,

-SO₂-NH-R⁵, -NH-SO₂-R⁷, -N(SO₂-R⁷)₂, -N(SO₂-R⁷)(CO-R⁵),
- R⁴: für Wasserstoff, Hydroxy, Amino, Carboxy, Cyano, Carbamoyl, Thiocarbamoyl, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in den Alkylgruppen, oder für eine der nachstehenden Gruppierungen steht,

-SO₂-NH-R⁵, -NH-SO₂-R⁷, -N(SO₂-R⁷)₂, -N(SO₂-R⁷)(CO-R⁵),
- n: für die Zahlen 0, 1 oder 2 steht,
- Q¹: für O oder S steht und
- Q²: für O, S, NH oder N-Alkyl mit 1 bis 4 Kohlenstoffatomen steht,
- R⁵: für Wasserstoff, für gegebenenfalls durch Cyano, Halogen oder C₁-C₄-Alkoxy substituiertes Alkyl mit 1 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Aryl oder Arylalkyl mit 6 oder 10 Kohlenstoffatomen in der Arylgruppe und gegebenenfalls 1 bis 4 Kohlenstoffatomen im Alkylteil, oder für gegebenenfalls durch Halogen, C₁-C₄-Alkyl, C₁-C₄-Halogenalkyl, C₁-C₄-Alkoxy oder C₁-C₄-Halogenalkoxy substituiertes Heterocyclyl steht, wobei als Heterocyclylgruppen Furyl, Tetrahydrofuryl, Thienyl, Pyridyl und Pyrimidinyl bevorzugt sind,
- R⁶: für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, oder für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Aryl oder Arylalkyl mit 6 oder 10 Kohlenstoffatomen in der Arylgruppe und gegebenenfalls 1 bis 4 Kohlenstoffatomen im Alkylteil steht,
- R⁷: für gegebenenfalls durch Halogen substituiertes Alkyl mit 1 bis 6 Kohlenstoffatomen, für jeweils gegebenenfalls durch Halogen substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl, C₁-C₄-Alkyl-sulfonyl oder C₁-C₄-Alkoxy-carbonyl substituiertes Aryl oder Arylalkyl mit 6 oder 10 Kohlenstoffatomen in der Arylgruppe und gegebenenfalls 1 bis 4 Kohlenstoffatomen im Alkylteil, oder für gegebenenfalls durch Halogen, C₁-C₄-Alkyl, C₁-C₄-Halogenalkyl, C₁-C₄-Alkoxy oder C₁-C₄-Halogenalkoxy substituiertes Heterocyclyl steht, wobei als Heterocyclylgruppen Pyridyl und Pyrimidinyl bevorzugt sind, und
- Z: für eine der nachstehenden heterocyclischen Gruppierungen steht. 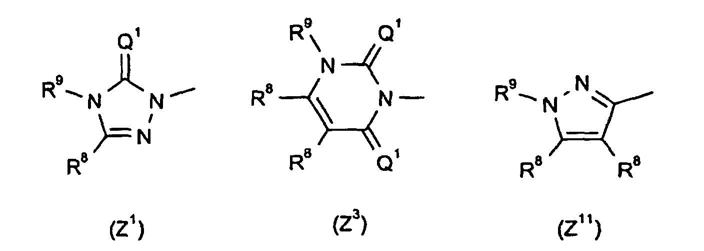 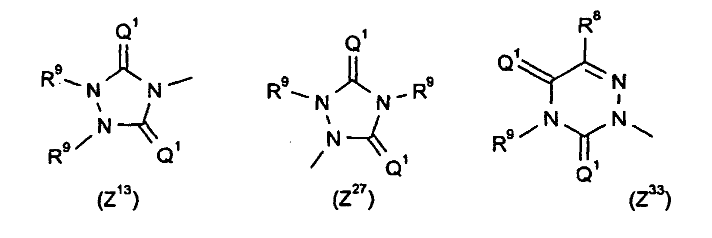 wobei jeweils
Q¹ und Q² die oben angegebenen Bedeutungen haben,
R⁸ für Wasserstoff, Amino, Nitro, Cyano, Carboxy, Carbamoyl, Thiocarbamoyl, Halogen, für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkoxy substituiertes Alkoxy oder Alkoxycarbonyl mit jeweils 1 bis 6 Kohlenstoffatomen in den Alkylgruppen, für jeweils gegebenenfalls durch Halogen substituiertes Alkenyloxy oder Alkinyloxy mit jeweils 3 bis 6 Kohlenstoffatomen, für gegebenenfalls durch Cyano, Halogen oder C₁-C₄-Alkoxy substituiertes Alkylthio mit 1 bis 6 Kohlenstoffatomen, für jeweils gegebenenfalls durch Halogen substituiertes Alkenylthio oder Alkinylthio mit jeweils 3 bis 6 Kohlenstoffatomen, für jeweils gegebenenfalls durch Cyano, Halogen oder C₁-C₄-Alkoxy substituiertes Alkylamino oder Dialkylamino mit jeweils 1 bis 6 Kohlenstoffatomen in den Alkylgruppen, oder für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht, und
- R⁹: für Wasserstoff, Hydroxy, Amino, Cyano, für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für gegebenenfalls durch Cyano, Halogen oder C₁-C₄-Alkoxy substituiertes Alkoxycarbonyl, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Cyano, Nitro, Halogen, C₁-C₄-Alkyl, C₁-C₄-Halogenalkyl, C₁-C₄-Alkoxy und/oder C₁-C₄-Halogenalkoxy substituiertes Phenyl oder Phenyl-C₁-C₄-alkyl steht,
wobei gegebenenfalls zwei benachbarte Reste - R⁸ und R⁸, R⁹ und R⁹ oder R⁸ und R⁹ - zusammen für Alkandiyl (Alkylen) oder Oxaalkandiyl mit jeweils 2 bis 6 Kohlenstoffatomen stehen, und
wobei die einzelnen Reste R⁸ und R⁹ - soweit sie mehr als einmal in der gleichen heterocyclischen Gruppierung stehen, die gleiche oder verschiedene Bedeutungen im Rahmen der obigen Definition haben können,
gefunden.

Man erhält die neuen substituierten aromatischen Aminoverbindungen der allgemeinen Formel (I), wenn man aromatische Aminoverbindungen der allgemeinen Formel (II), 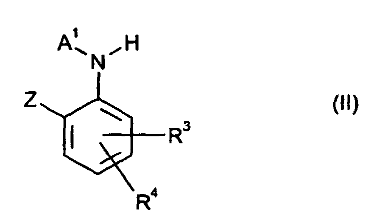 in welcher
- R³, R⁴ und Z: die oben angegebenen Bedeutungen haben und
- A¹: für Wasserstoff, Hydroxy, Amino, Alkyl, Alkoxy, Alkylamino oder Dialkylamino steht,
mit elektrophilen Verbindungen der allgemeinen Formel (III),

X-R² (III)

in welcher
- R²: die oben angegebene Bedeutung hat und
- X: für Halogen steht,
gegebenenfalls in Gegenwart eines Säureakzeptors und gegebenenfalls in Gegenwart eines Verdünnungsmittels, umsetzt
und die auf diese Weise erhaltenen Verbindungen der allgemeinen Formel (I), gegebenenfalls nach üblichen Methoden, in andere Verbindungen der allgemeinen Formel (I) gemäß der obigen Definition umwandelt (vgl. die Herstellungsbeispiele).

Die neuen substituierten aromatischen Aminoverbindungen der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkandiyl, Alkenyl oder Alkinyl - auch in Verbindung mit Heteroatomen, wie in Alkoxy, Alkylthio oder Alkylamino - jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I),
in welcher
- R¹: für Wasserstoff, Hydroxy, Amino, für Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino oder Dimethylamino, oder für eine der nachstehenden Gruppierungen steht,

-CQ¹-R⁵, -CQ¹-Q²-R⁶,
- R²: für eine der nachstehenden Gruppierungen steht,

-CQ¹-R⁵, -CQ¹-Q²-R⁶,
- R³: für Wasserstoff, Hydroxy, Amino, Carboxy, Cyano, Carbamoyl, Thiocarbamoyl, Nitro, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methoxycarbonyl, Ethoxycarbonyl, Methylthio, Ethylthio, n- oder i-Propylthio, n-, i-, s- oder t-Butylthio, Methylsulfinyl, Ethylsulfinyl, Methylsulfonyl oder Ethylsulfonyl, oder für eine der nachstehenden Gruppierungen steht,

-SO₂-NH-R⁵, -NH-SO₂-R⁷, -N(SO₂-R⁷)₂, -N(SO₂-R⁷)(CO-R⁵),
- R⁴: für Wasserstoff, Hydroxy, Amino, Carboxy, Cyano, Carbamoyl, Thiocarbamoyl, Nitro, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methoxycarbonyl, Ethoxycarbonyl, Methylthio, Ethylthio, n- oder i-Propylthio, n-, i-, s- oder t-Butylthio, Methylsulfinyl, Ethylsulfinyl, Methylsulfonyl oder Ethylsulfonyl, oder für eine der nachstehenden Gruppierungen steht,

-SO₂-NH-R⁵, -NH-SO₂-R⁷, -N(SO₂-R⁷)₂, -N(SO₂-R⁷)(CO-R⁵),
- n: für die Zahlen 0, 1 oder 2 steht,
- Q¹: für O oder S steht und
- Q²: für O, S, NH oder N-Methyl steht,
- R⁵: für Wasserstoff, für gegebenenfalls durch Cyano, Fluor, Chlor, Brom, Methoxy oder Ethoxy substituiertes Methyl, Ethyl, n- oder i-Propyl, n-, i-, s- oder t-Butyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Phenyl oder Benzyl, oder für gegebenenfalls durch Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Furyl, Tetrahydrofuryl, Thienyl oder Pyridyl steht,
- R⁶: für gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Phenyl oder Benzyl steht,
- R⁷: für gegebenenfalls durch Fluor, Chlor oder Brom substituiertes Methyl, Ethyl, n- oder i-Propyl, n-, i-, s- oder t-Butyl, für jeweils gegebenenfalls durch Fluor, Chlor oder Brom substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, n-, i-, s- oder t-Butylthio, Methylsulfinyl, Ethylsulfmyl, Methylsulfonyl, Ethylsulfonyl, Methoxycarbonyl oder Ethoxy-carbonyl substituiertes Phenyl oder Benzyl, oder für gegebenenfalls durch Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Pyridyl oder Pyrimidinyl steht und
- Z: für eine der nachstehenden heterocyclischen Gruppierungen steht. 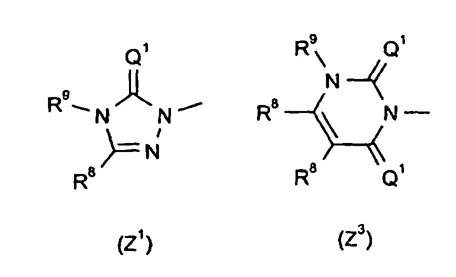 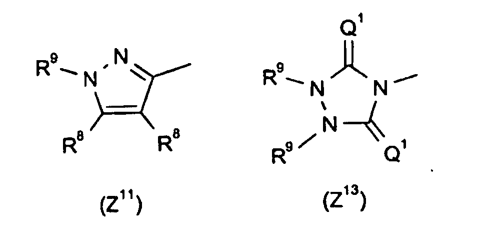 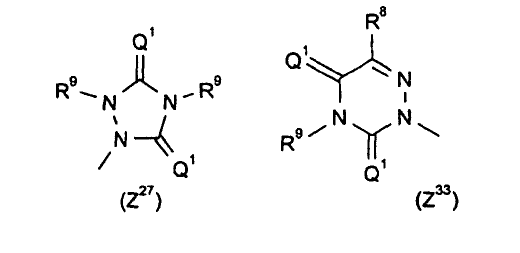
wobei jeweils
- Q¹ und Q²: die oben angegebenen Bedeutungen haben,
- R⁸: für Wasserstoff, Amino, Nitro, Cyano, Carboxy, Carbamoyl, Thiocarbamoyl, Fluor, Chlor, Brom, für gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, Methoxycarbonyl oder Ethoxycarbonyl, für jeweils gegebenenfalls durch Fluor oder Chlor substituiertes Propenyloxy, Butenyloxy, Propinyloxy oder Butinyloxy, für gegebenenfalls durch Cyano, Fluor, Chlor, Methoxy oder Ethoxy substituiertes Methylthio, Ethylthio, n- oder i-Propylthio, n-, i-, s- oder t-Butylthio, für jeweils gegebenenfalls durch Fluor oder Chlor substituiertes Propenylthio, Butenylthio, Propinylthio oder Butinylthio, für jeweils gegebenenfalls durch Cyano, Fluor, Chlor, Methoxy oder Ethoxy substituiertes Methylamino, Ethylamino, n- oder i-Propylamino, n-, i-, s- oder t-Butylamino, Dimethylamino oder Diethylamino, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht, und
- R⁹: für Wasserstoff, Hydroxy, Amino, Cyano, für gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für gegebenenfalls durch Cyano, Fluor, Chlor, Methoxy oder Ethoxy substituiertes Methoxycarbonyl oder Ethoxycarbonyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Cyano, Nitro, Fluor, Chlor, Brom, Methyl, Ethyl, n- oder i-Propyl, n-, i-, s-
oder t-Butyl, Trifluormethyl, Methoxy, Ethoxy, n- oder i-Propoxy, n-, i-, s- oder t-Butoxy, Difluormethoxy und/oder Trifluormethoxy substituiertes Phenyl oder Benzyl steht,
wobei gegebenenfalls zwei benachbarte Reste - R⁸ und R⁸, R⁹ und R⁹ oder R⁸ und R⁹ - zusammen für Ethan-1,2-diyl (Dimethylen), Propan-1,3-diyl (Trimethylen), Butan-1,4-diyl (Tetramethylen) oder 1-Oxa-butan-1,4-diyl stehen, und
wobei die einzelnen Reste R⁸ und R⁹ - soweit sie mehr als einmal in der gleichen heterocyclischen Gruppierung stehen, die gleiche oder verschiedene Bedeutungen im Rahmen der obigen Definition haben können.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

### Gruppe 1

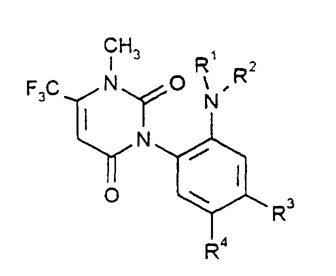

R¹, R², R³ und R⁴ haben dabei die in der nachstehenden Auflistung angegebenen Bedeutungen:

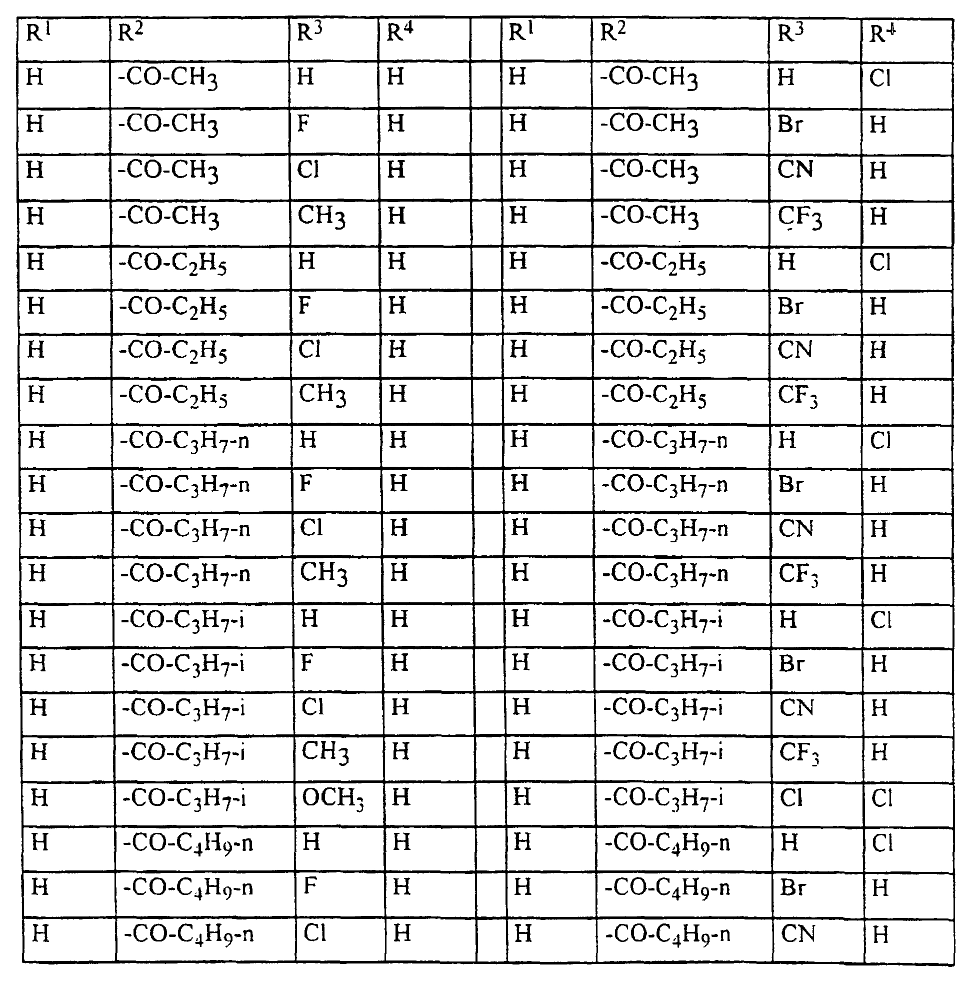

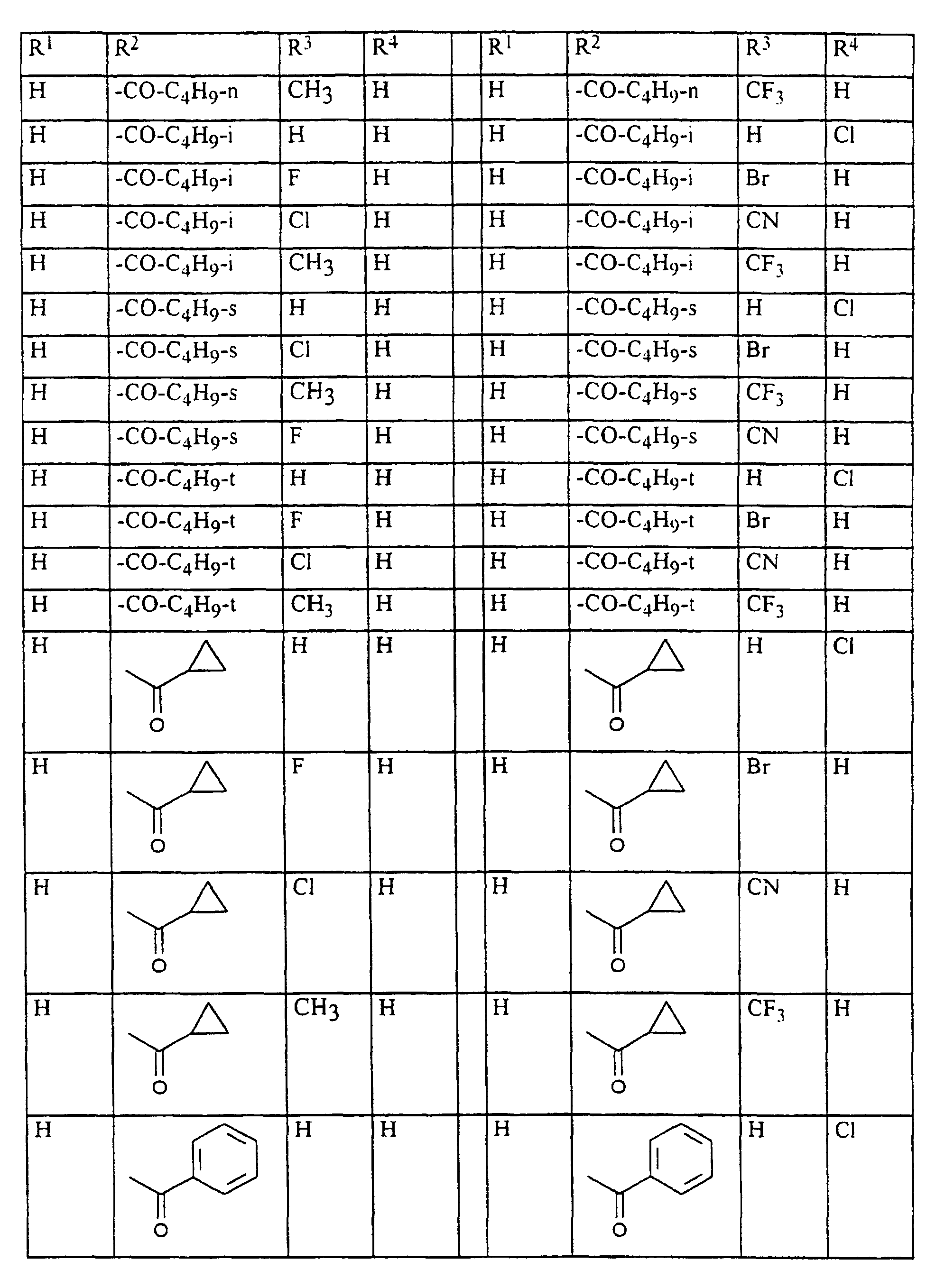

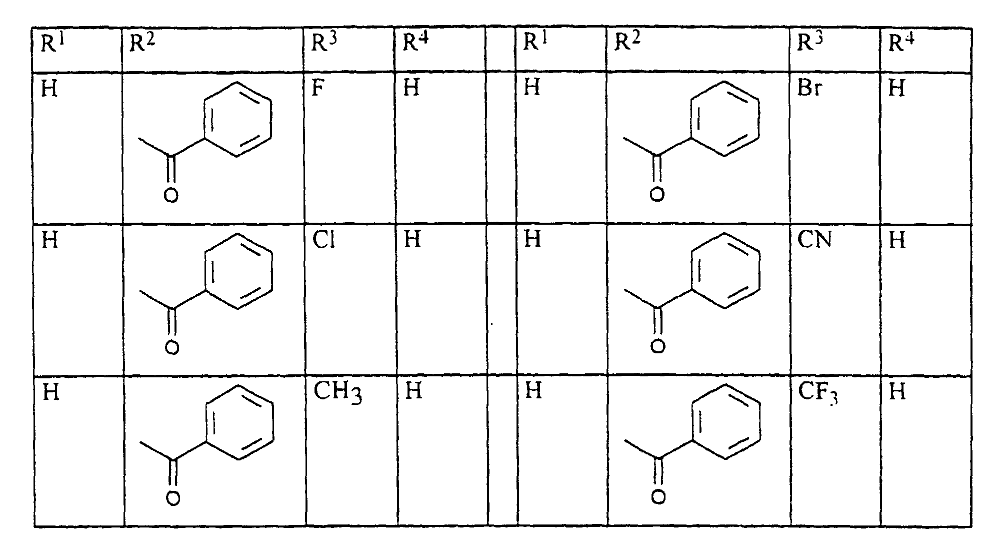

### Gruppe 2

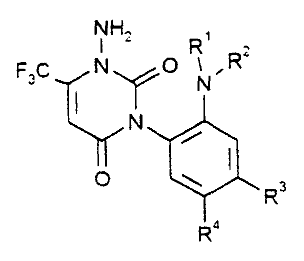

R¹, R² R³ und R⁴ haben hierbei beispielhaft die oben in Gruppe 1 angegebenen Bedeutungen.

### Gruppe 3

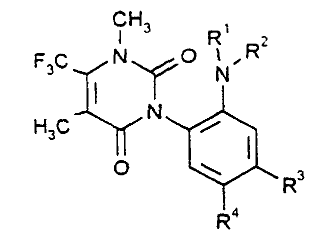

R¹, R² R³ und R⁴ haben hierbei beispielhaft die oben in Gruppe 1 angegebenen Bedeutungen.

### Gruppe 4

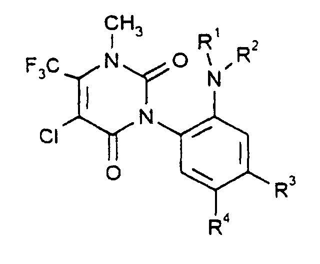

R¹, R² R³ und R⁴ haben hierbei beispielhaft die oben in Gruppe 1 angegebenen Bedeutungen.

### Gruppe 5

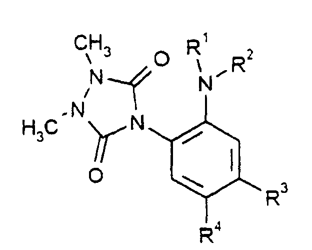

R¹, R² R³ und R⁴ haben hierbei beispielhaft die oben in Gruppe 1 angegebenen Bedeutungen.

### Gruppe 6

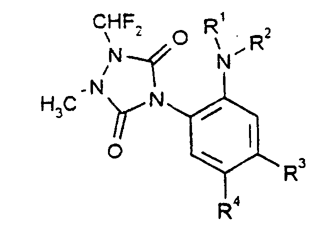

R¹, R² R³ und R⁴ haben hierbei beispielhaft die oben in Gruppe 1 angegebenen Bedeutungen.

### Gruppe 7

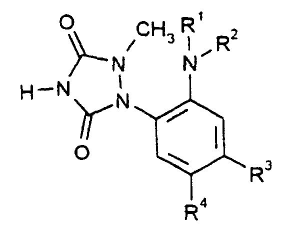

R¹, R² R³ und R⁴ haben hierbei beispielhaft die oben in Gruppe 1 angegebenen Bedeutungen.

### Gruppe 8

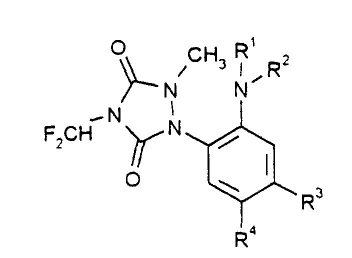

R¹, R² R³ und R⁴ haben hierbei beispielhaft die oben in Gruppe 1 angegebenen Bedeutungen.

### Gruppe 9

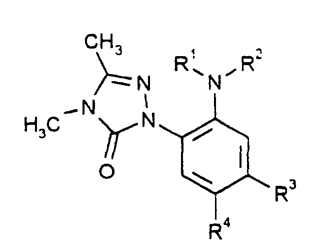

R¹, R² R³ und R⁴ haben hierbei beispielhaft die oben in Gruppe 1 angegebenen Bedeutungen.

### Gruppe 10

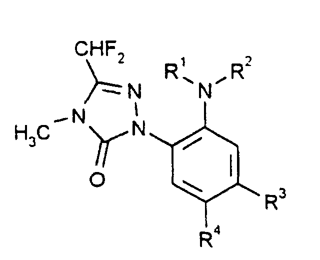

R¹, R² R³ und R⁴ haben hierbei beispielhaft die oben in Gruppe 1 angegebenen Bedeutungen.

### Gruppen 11

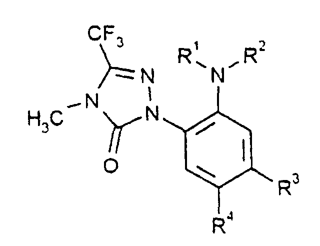

R¹, R² R³ und R⁴ haben hierbei beispielhaft die oben in Gruppe 1 angegebenen Bedeutungen.

### Gruppe 12

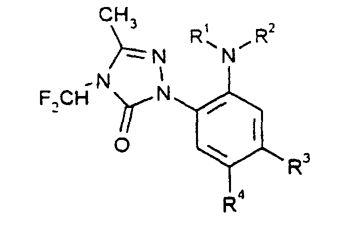

R¹, R² R³ und R⁴ haben hierbei beispielhaft die oben in Gruppe 1 angegebenen Bedeutungen.

### Gruppe 13

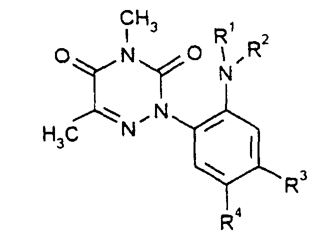

R¹, R² R³ und R⁴ haben hierbei beispielhaft die oben in Gruppe 1 angegebenen Bedeutungen.

### Gruppe 14

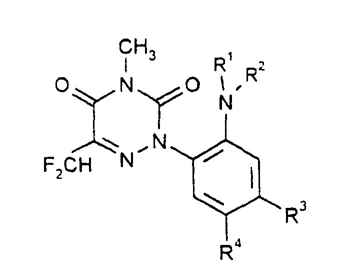

R¹, R² R³ und R⁴ haben hierbei beispielhaft die oben in Gruppe 1 angegebenen Bedeutungen.

### Gruppe 15

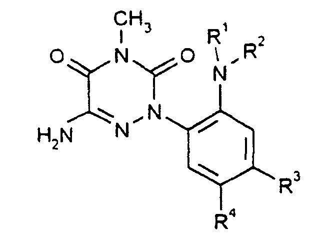

R¹, R² R³ und R⁴ haben hierbei beispielhaft die oben in Gruppe 1 angegebenen Bedeutungen.

Verwendet man beispielsweise 2-(2-Amino-4-cyano-phenyl)-4-methyl-5-difluormethyl-2,4-dihydro-3H-1,2,4-triazol-3-on und Acetylchlorid als Ausgangsstoffe, so kann der Reaktionsablauf beim erfindungsgemäßen Verfahren durch das folgende Formelschema skizziert werden:

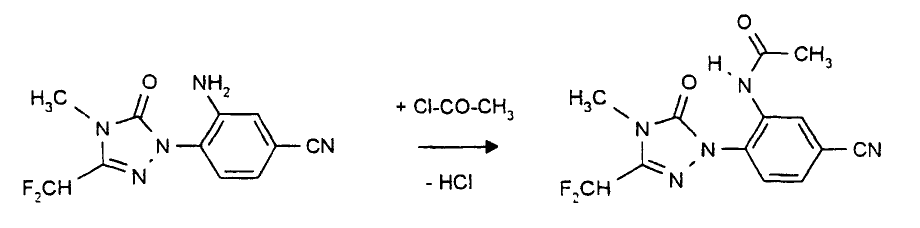

Die beim erfindungsgemäßen Verfahren zur Herstellung von Verbindungen der Formel (I) als Ausgangsstoffe zu verwendenden aromatischen Aminoverbindungen sind durch die Formel (II) allgemein definiert. In der Formel (II) haben R³, R⁴ und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³, R⁴ und Z angegeben wurden; A¹ steht vorzugsweise für Wasserstoff, Hydroxy, Amino, C₁-C₆-Alkyl, C₁-C₆-Alkoxy, C₁-C₆-Alkylamino oder Di-(C₁-C₆-alkyl)-amino, insbesondere für Wasserstoff oder Methyl.

Die Ausgangsstoffe der allgemeinen Formel (II) sind noch nicht aus der Literatur bekannt; sie sind als neue Stoffe auch Gegenstand der vorliegenden Anmeldung.

Man erhält die neuen aromatischen Aminoverbindungen der allgemeinen Formel (II), wenn man aromatische Nitroverbindungen der allgemeinen Formel (IV), 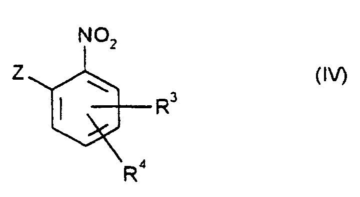 in welcher
R³, R⁴ und Z die oben angegebene Bedeutung haben,
mit Reduktionsmitteln, wie z.B. mit Wasserstoff in Gegenwart eines Katalysators, wie z.B. Raney-Nickel, oder mit Zinn(II)-chlorid-Dihydrat, oder mit Eisen in Gegenwart einer Säure, wie z.B. Salzsäure, jeweils in Gegenwart eines Verdünnungsmittels, wie z.B. Wasser, Methanol oder Ethanol, bei Temperaturen zwischen 0°C und 120°C umsetzt (vgl. die Herstellungsbeispiele).

Die als Vorprodukte benötigten aromatischen Nitroverbindungen der allgemeinen Formel (IV) sind bekannt und/oder können nach bekannten Verfahren hergestellt werden (vgl. US 3489761, DE 2413938, GB 2123420, US 4496390, WO 8702357, EP 617026, Herstellungsbeispiele).

Die beim erfindungsgemäßen Verfahren zur Herstellung von Verbindungen der Formel (I) weiter als Ausgangsstoffe zu verwendenden elektrophilen Verbindungen sind durch die Formel (III) allgemein definiert. In der Formel (III) hat R² vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R² angegeben wurde; X steht vorzugsweise für Fluor, Chlor, Brom oder Iod, insbesondere für Chlor oder Brom.

Die Ausgangsstoffe der allgemeinen Formel (III) sind bekannte organische Synthesechemikalien.

Das erfindungsgemäße Verfahren zur Herstellung der Verbindungen der Formel (I) wird vorzugsweise unter Verwendung eines Säureakzeptors durchgeführt. Als Säureakzeptoren für das erfindungsgemäße Verfahren kommen im allgemeinen die üblichen anorganischen oder organischen Basen oder Säurebindemittel in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin. N.N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Das erfindungsgemäße Verfahren zur Herstellung der Verbindungen der Formel (I) wird vorzugsweise unter Verwendung eines Verdünnungsmittels durchgerührt. Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgerührt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phteum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum. Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen, wie z.B. in Weizen, sowohl im Vorauflauf- als auch im NachauflaufVerfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit. Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofumesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Thiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-Verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

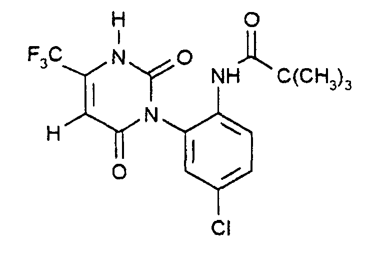

Eine Mischung aus 1,6 g (5,23 mMol) 1-(2-Amino-5-chlor-phenyl)-3,6-dihydro-2,6-dioxo-4-trifluormethyl-1(2H)-pyrimidin, 0,72 g (5,23 mMol) Pivalinsäurechlorid. 1,21 g (12 mMol) Triethylamin und 30 ml Acetonitril wird 45 Minuten bei Raumtemperatur (ca. 20°C) gerührt und anschließend im Wasserstrahlvakuum eingeengt. Der Rückstand wird dann mit 1N-Salzsäure, Diethylether und Petrolether verrührt und das hierbei kristallin anfallende Produkt wird durch Absaugen isoliert.

Man erhält 1,65 g (81% der Theorie) 1-(5-Chlor-2-pivaloylamino-phenyl)-3,6-dihydro-2,6-dioxo-4-trifluormethyl-1(2H)-pyrimidin vom Schmelzpunkt 276°C.

### Beispiel 2

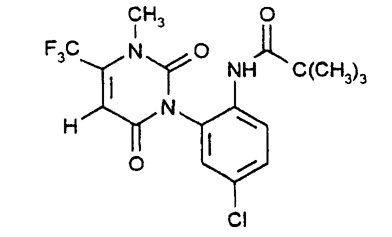

Eine Mischung aus 1,17 g (3 mMol) 1-(5-Chlor-2-pivaloylamino-phenyl)-3,6-dihydro-2,6-dioxo-4-trifluormethyl-1(2H)-pyrimidin, 0,42 g (3 mMol) Dimethylsulfat, 0,46 g (3 mMol) Kaliumcarbonat und 20 ml Aceton wird 45 Minuten unter Rückfluß erhizt und anschließend im Wasserstrahlvakuum eingeengt. Dann wird der Rückstand mit 1N-Salzsäure, Essigsäureethylester und Diethylether verrührt. Das hierbei kristallin angefallene Produkt wird durch Absaugen isoliert.

Man erhält 1,05 g (88% der Theorie) 1-(5-Chlor-2-pivaloylamino-phenyl)-3,6-dihydro-2,6-dioxo-3-methyl-4-trifluormethyl-1(2H)-pyrimidin vom Schmelzpunkt 267°C.

Analog zu den Herstellungsbeispielen 1 und 2 sowie entsprechend der allgemeinen Beschreibung des erfindungsgemäßen Herstellungsverfahrens können beispielsweise auch die in den nachstehenden Tabellen aufgeführten Verbindungen der Formel (I) hergestellt werden.

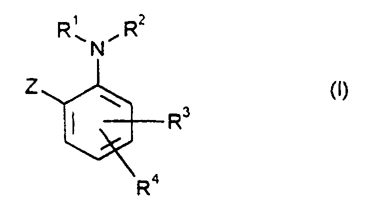

Ein großer Teil der erfindungsgemäßen Wirkstoffe kann durch die nachstehende allgemeine Formel (Ia) wiedergegeben werden:

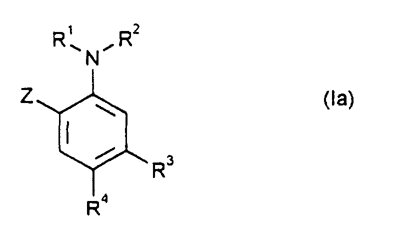

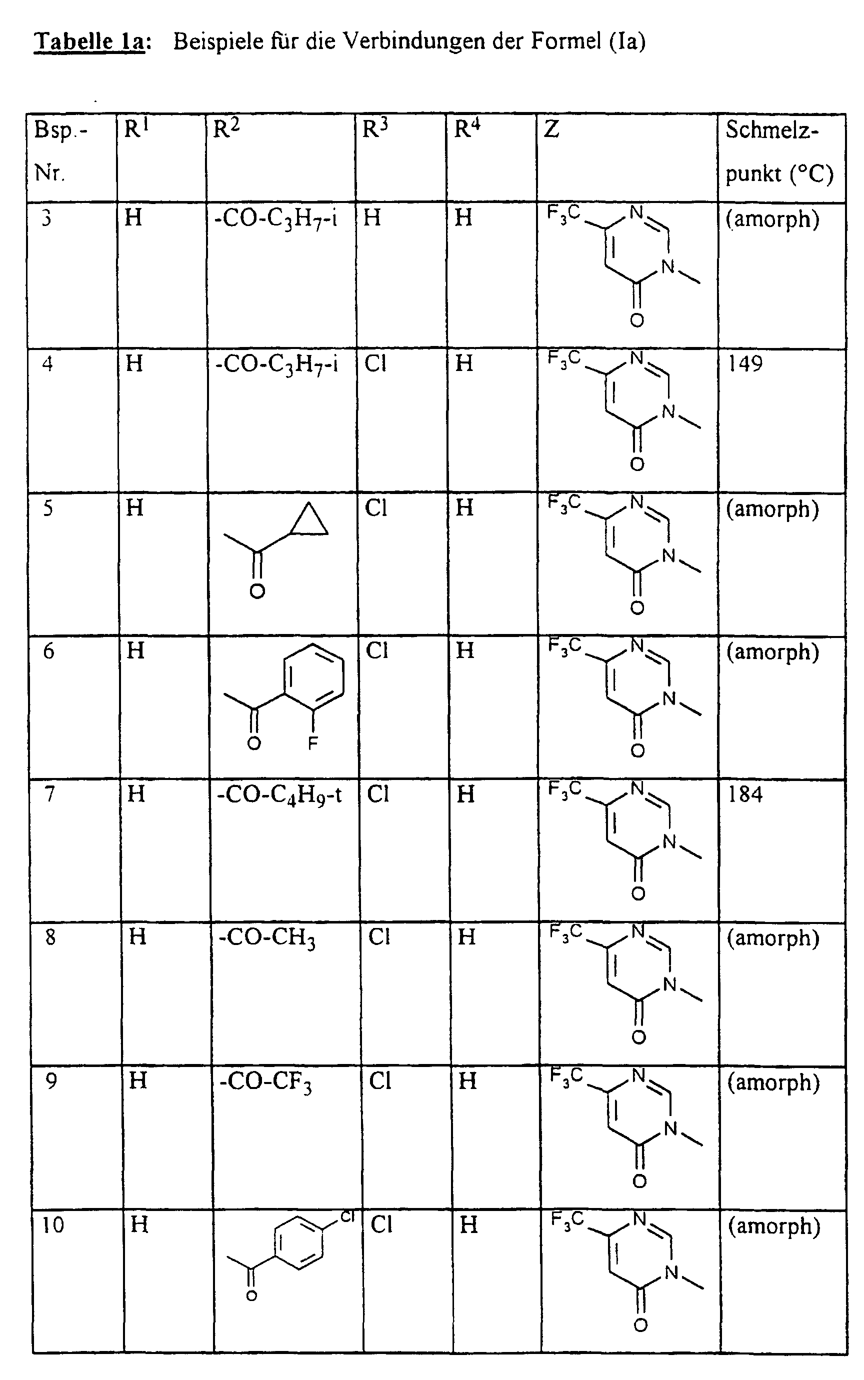

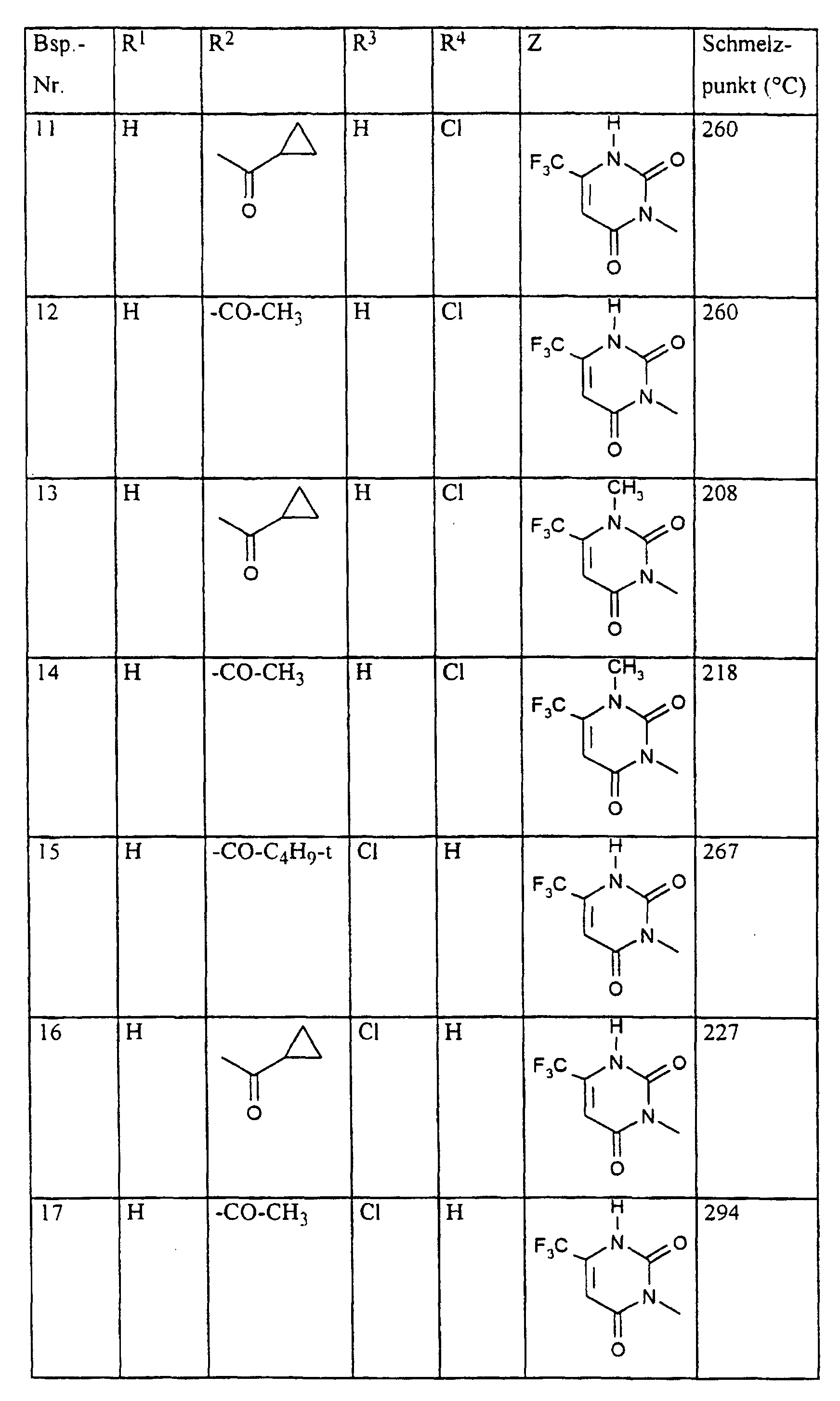

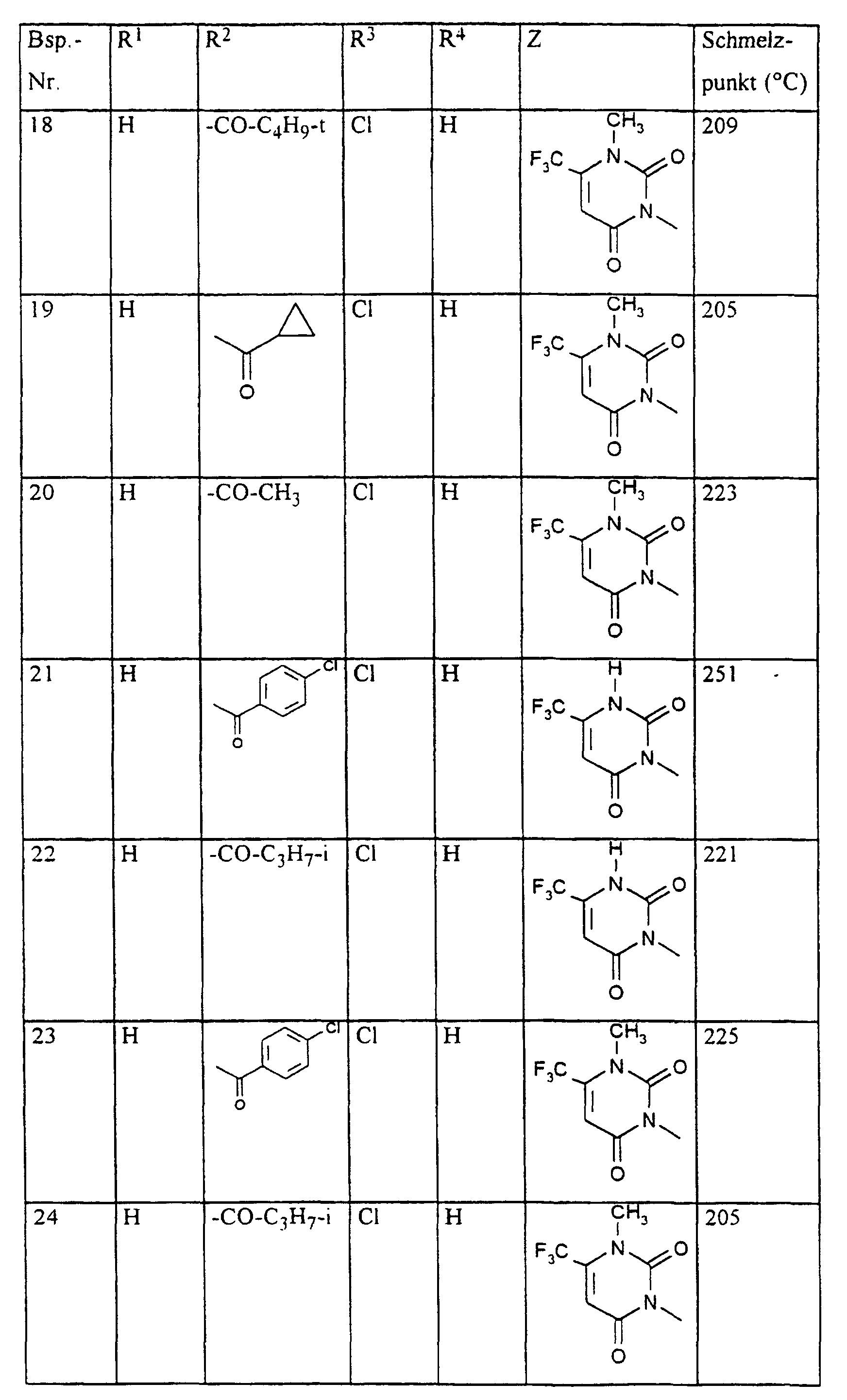

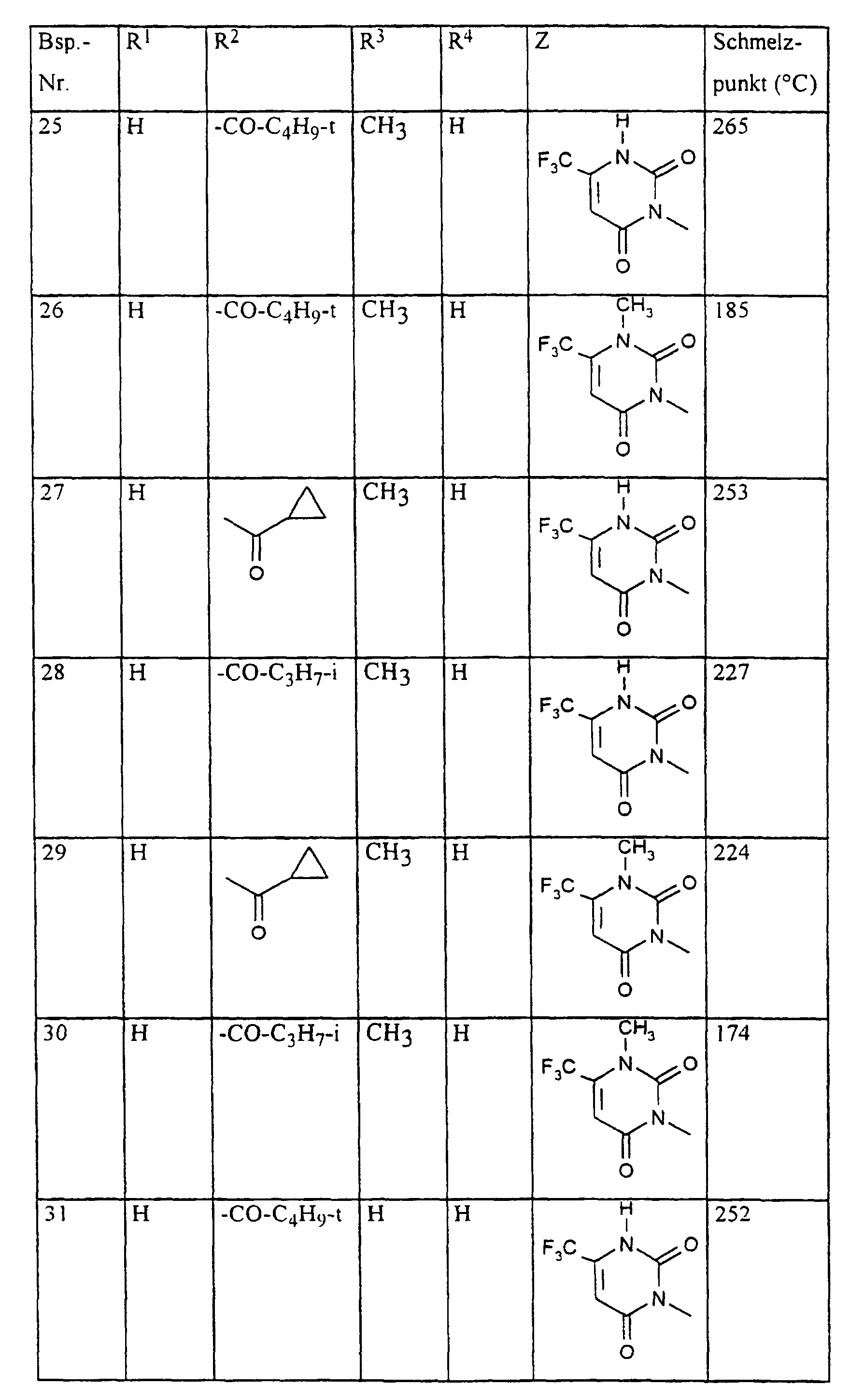

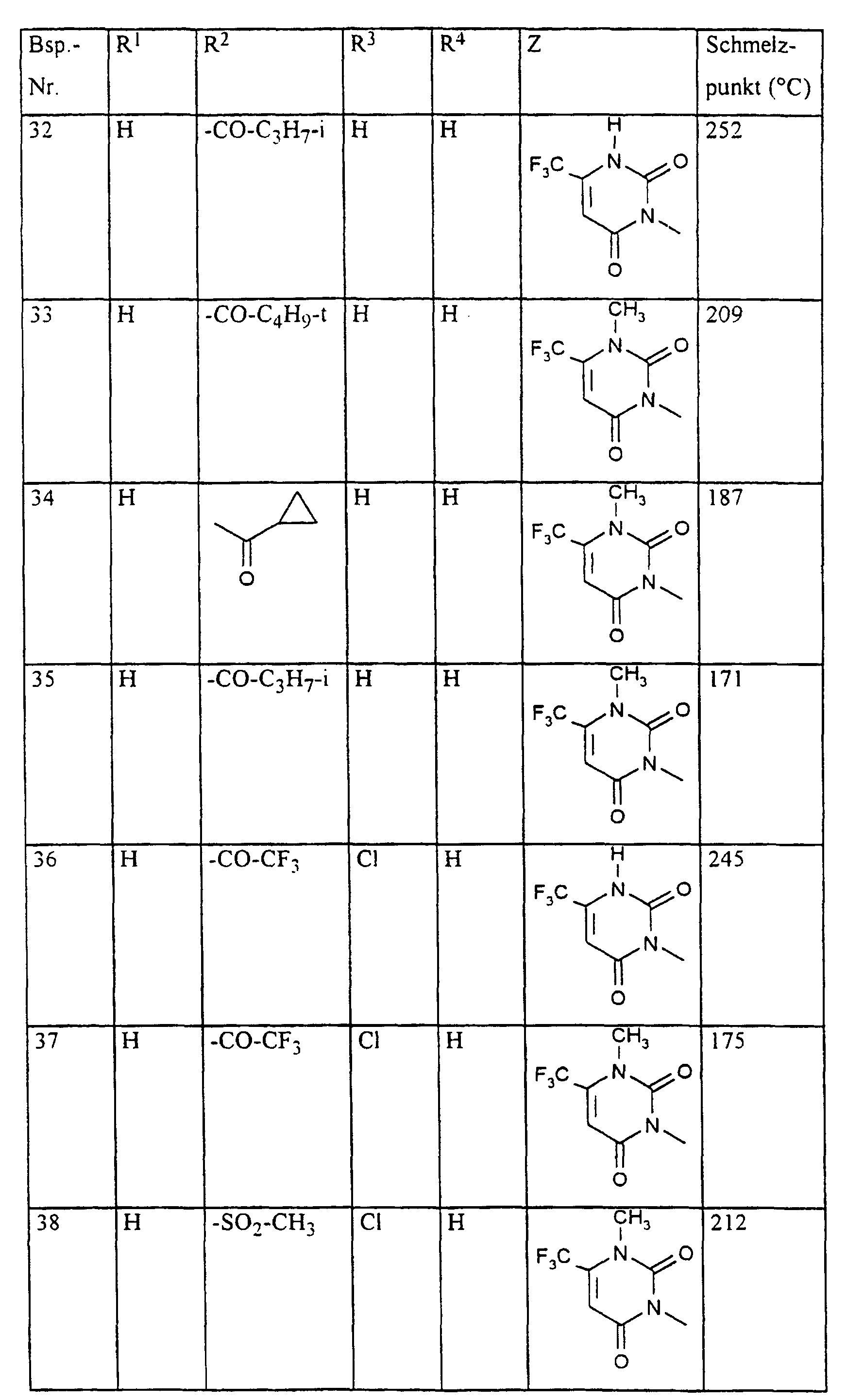

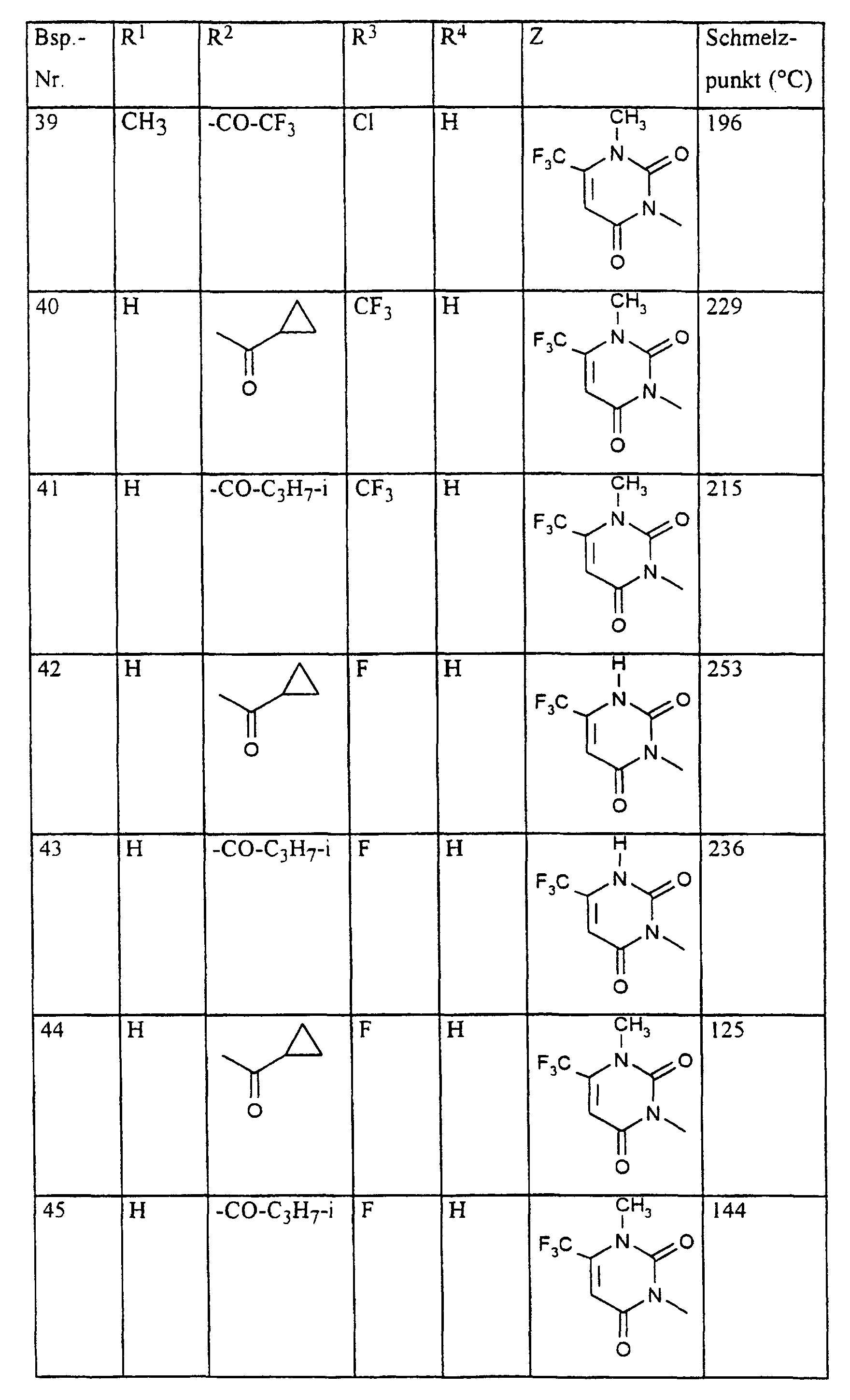

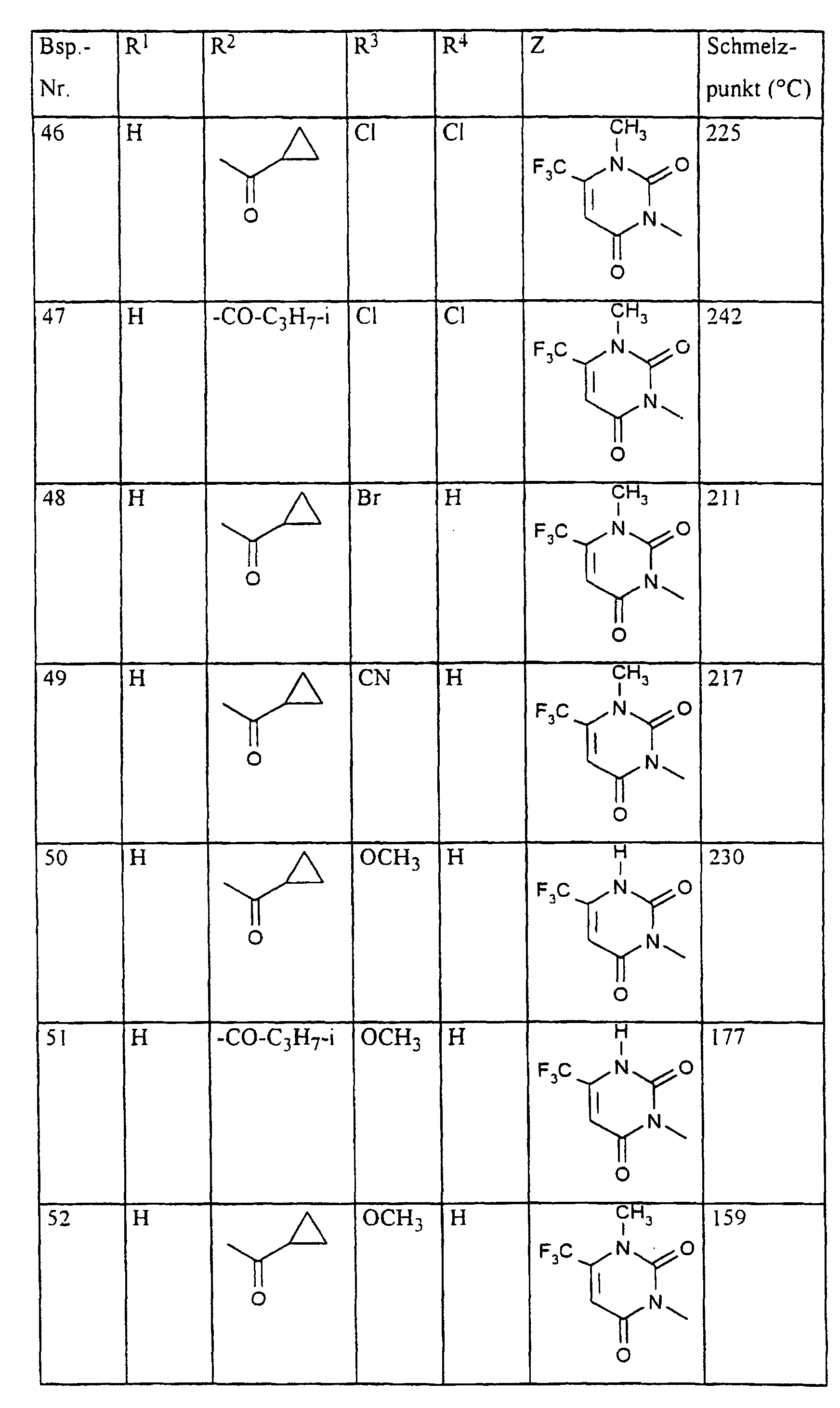

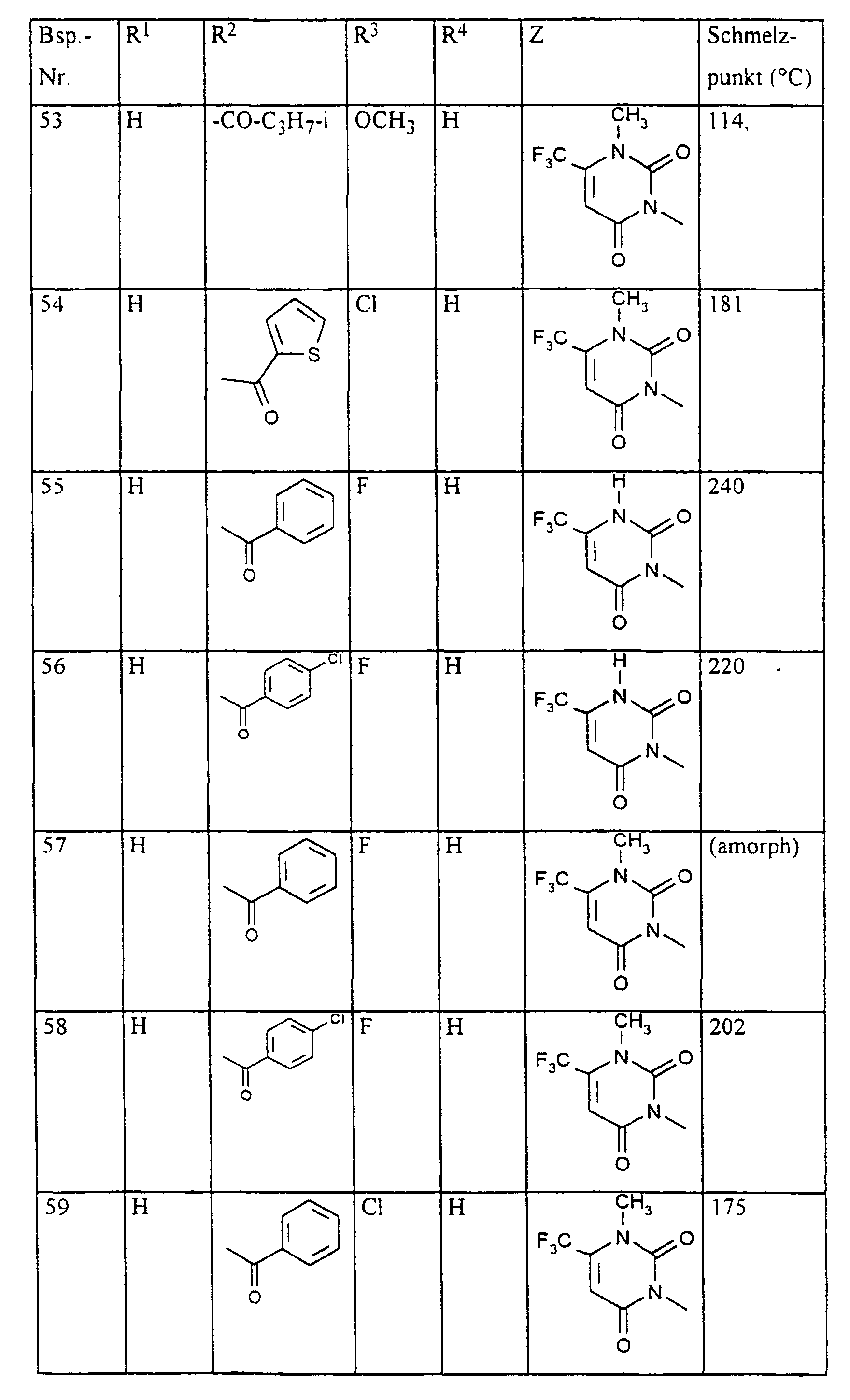

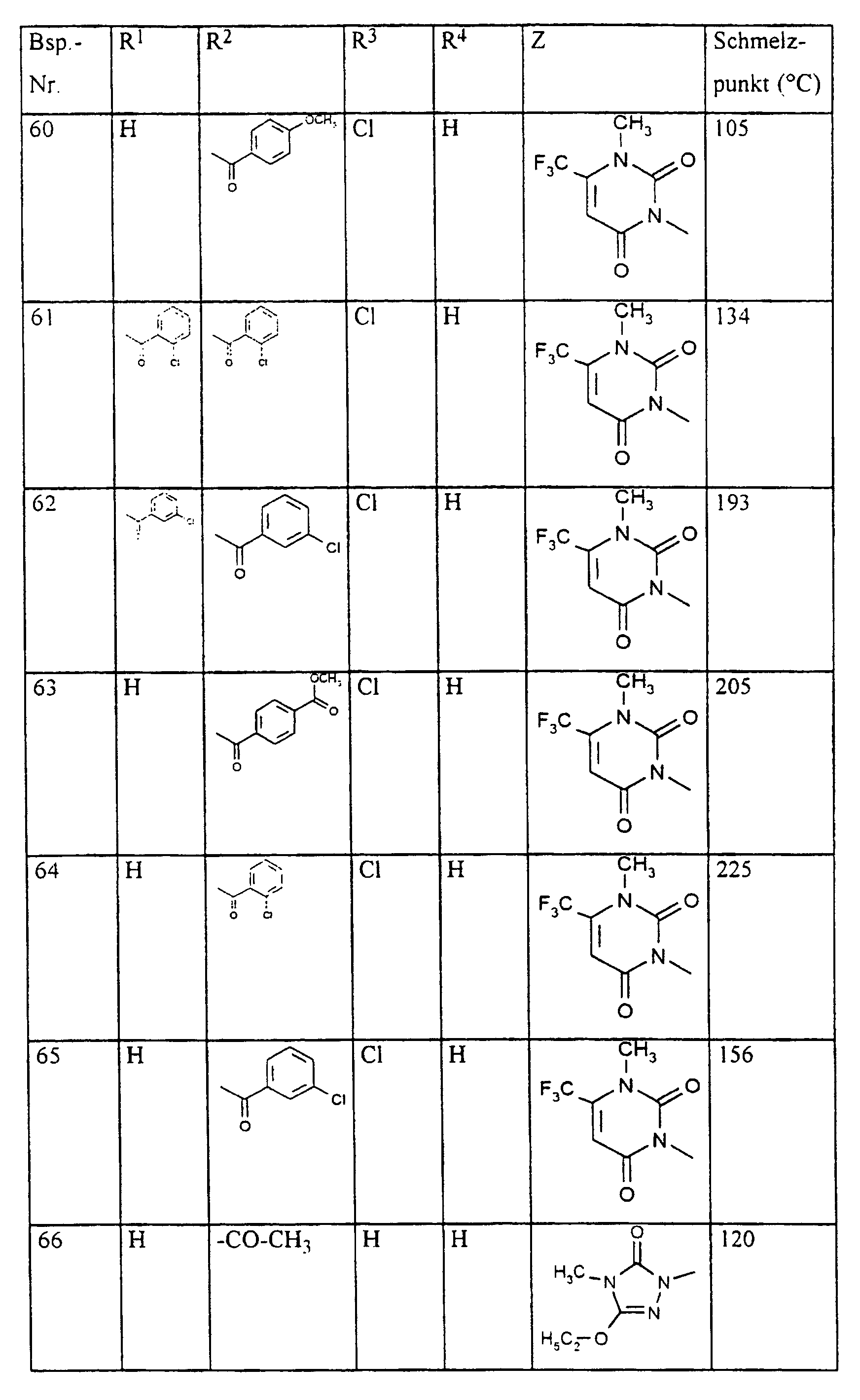

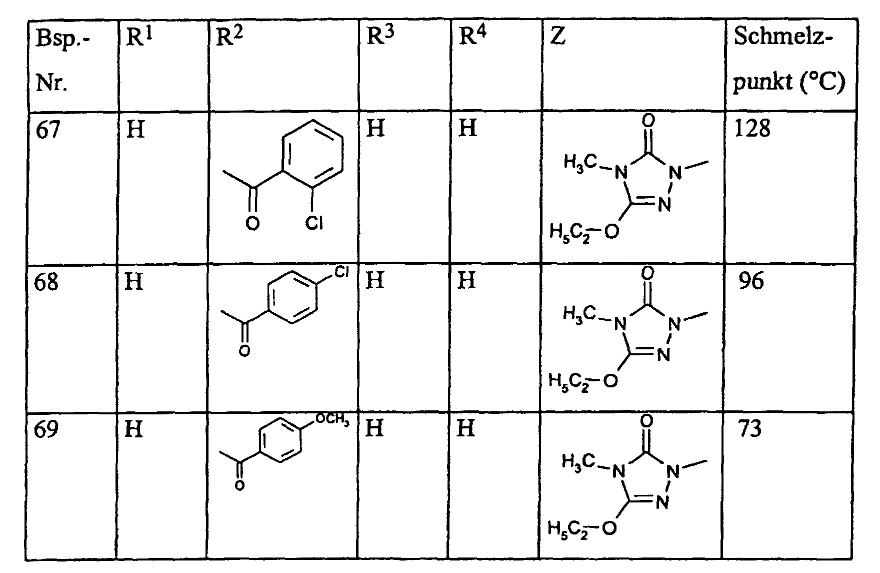

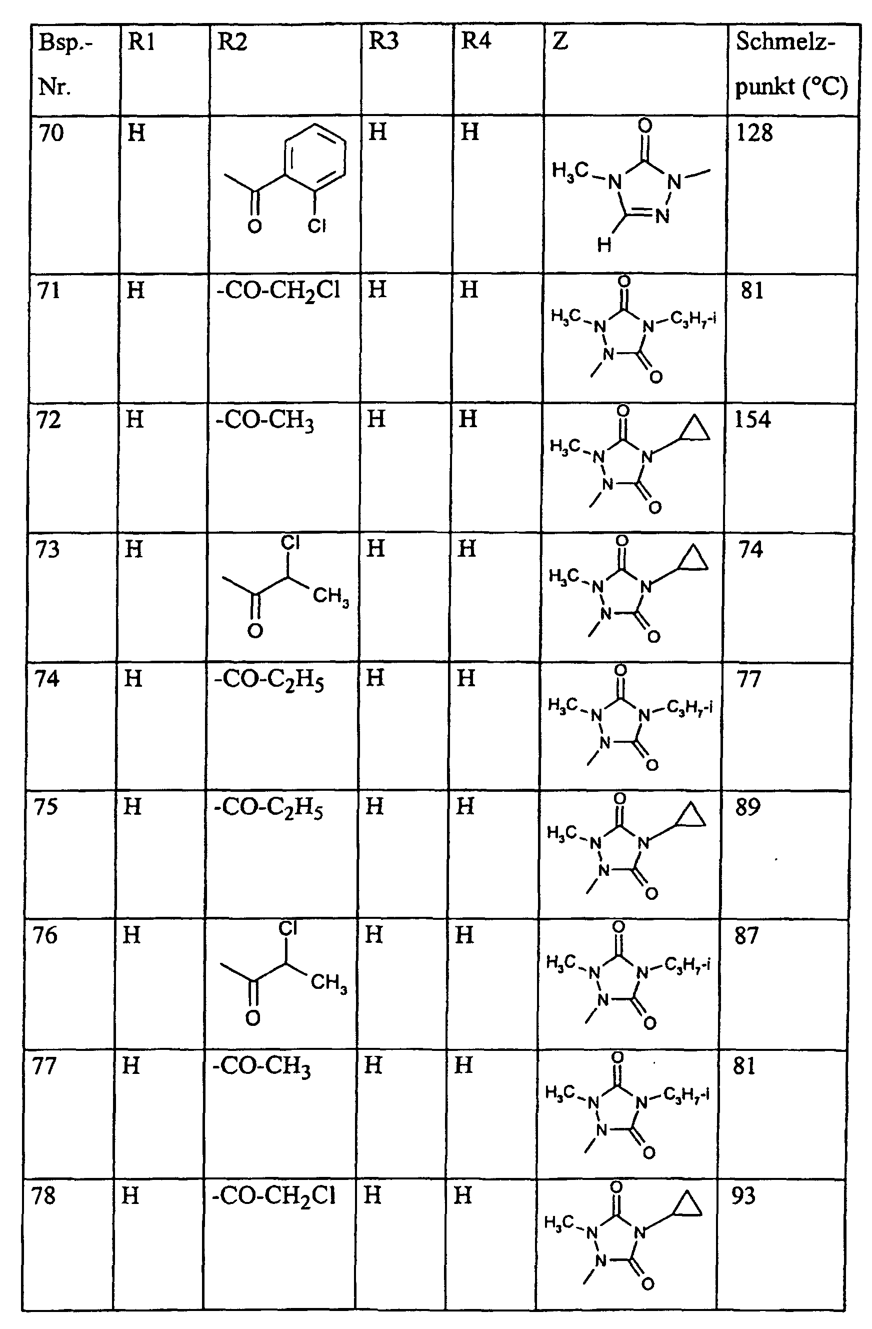

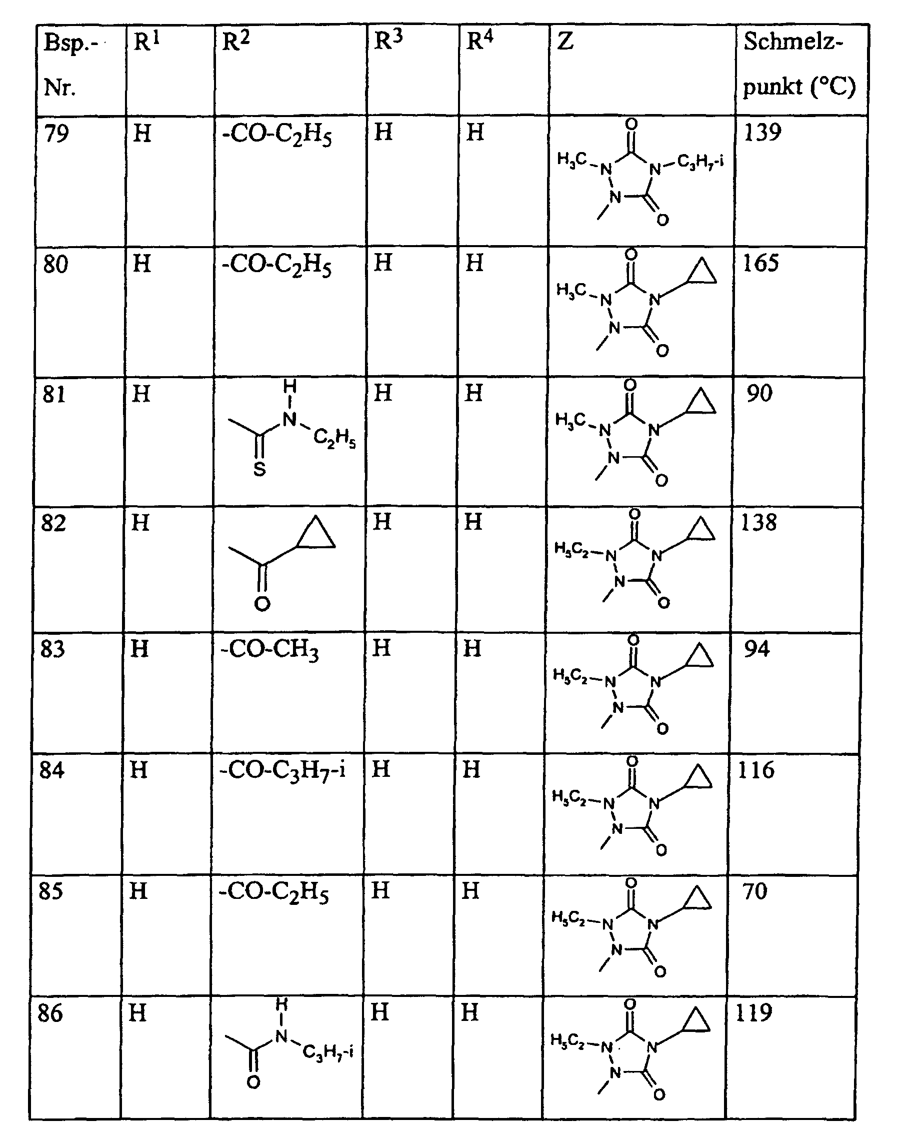

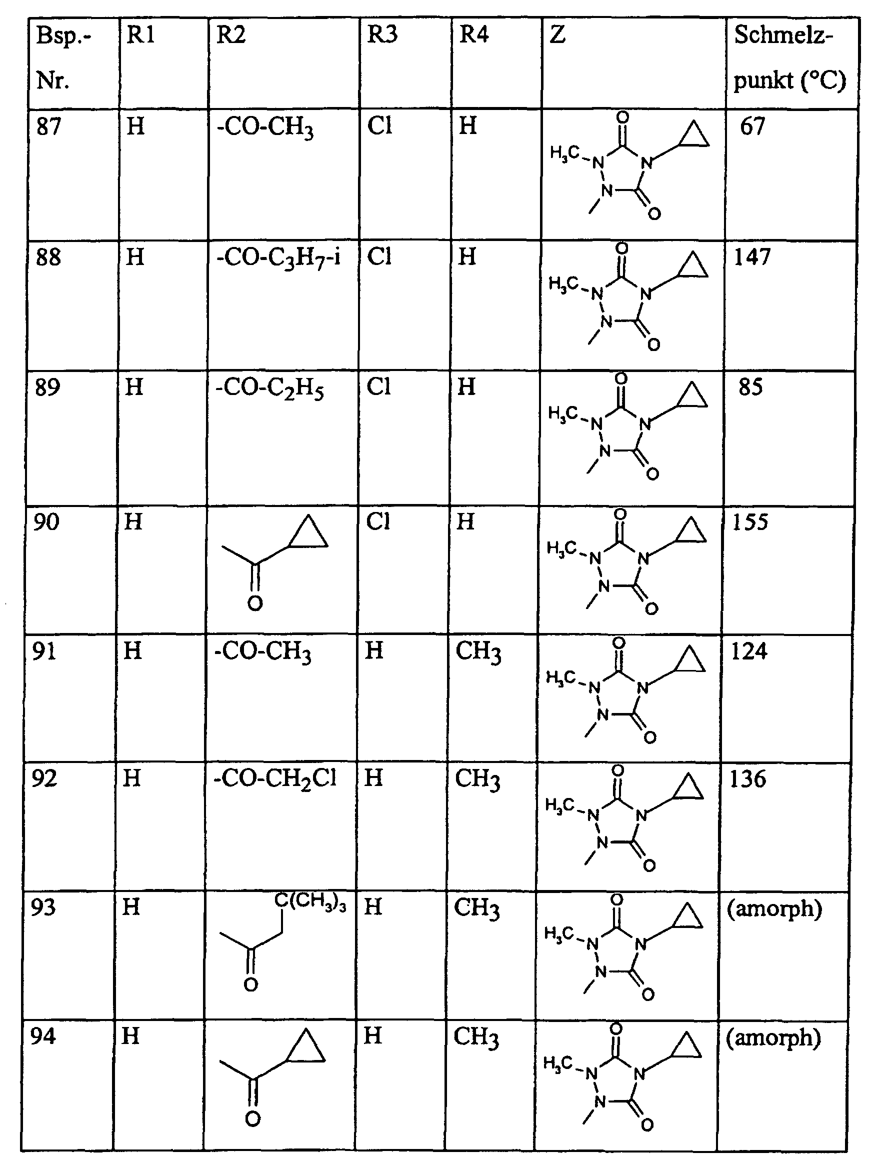

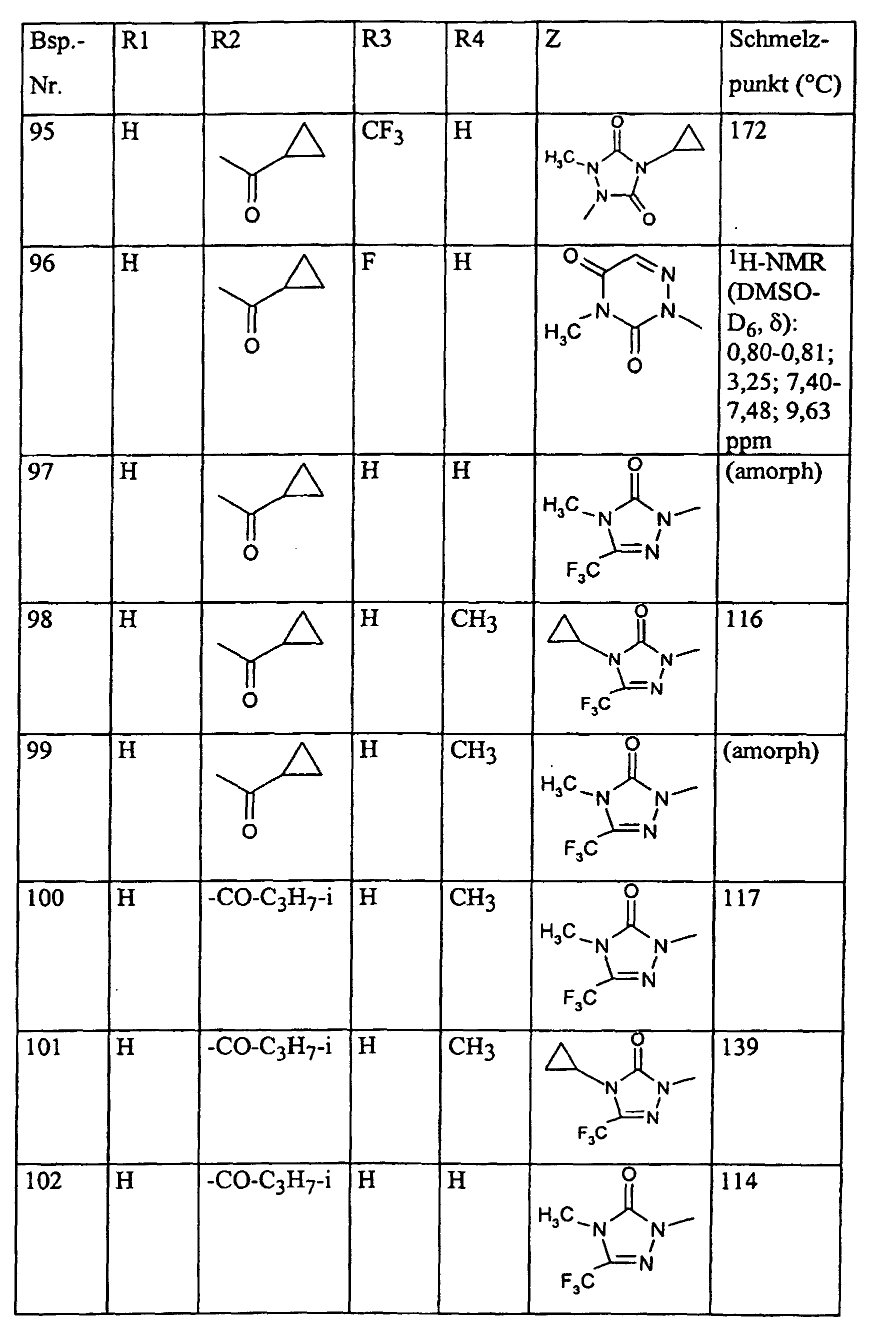

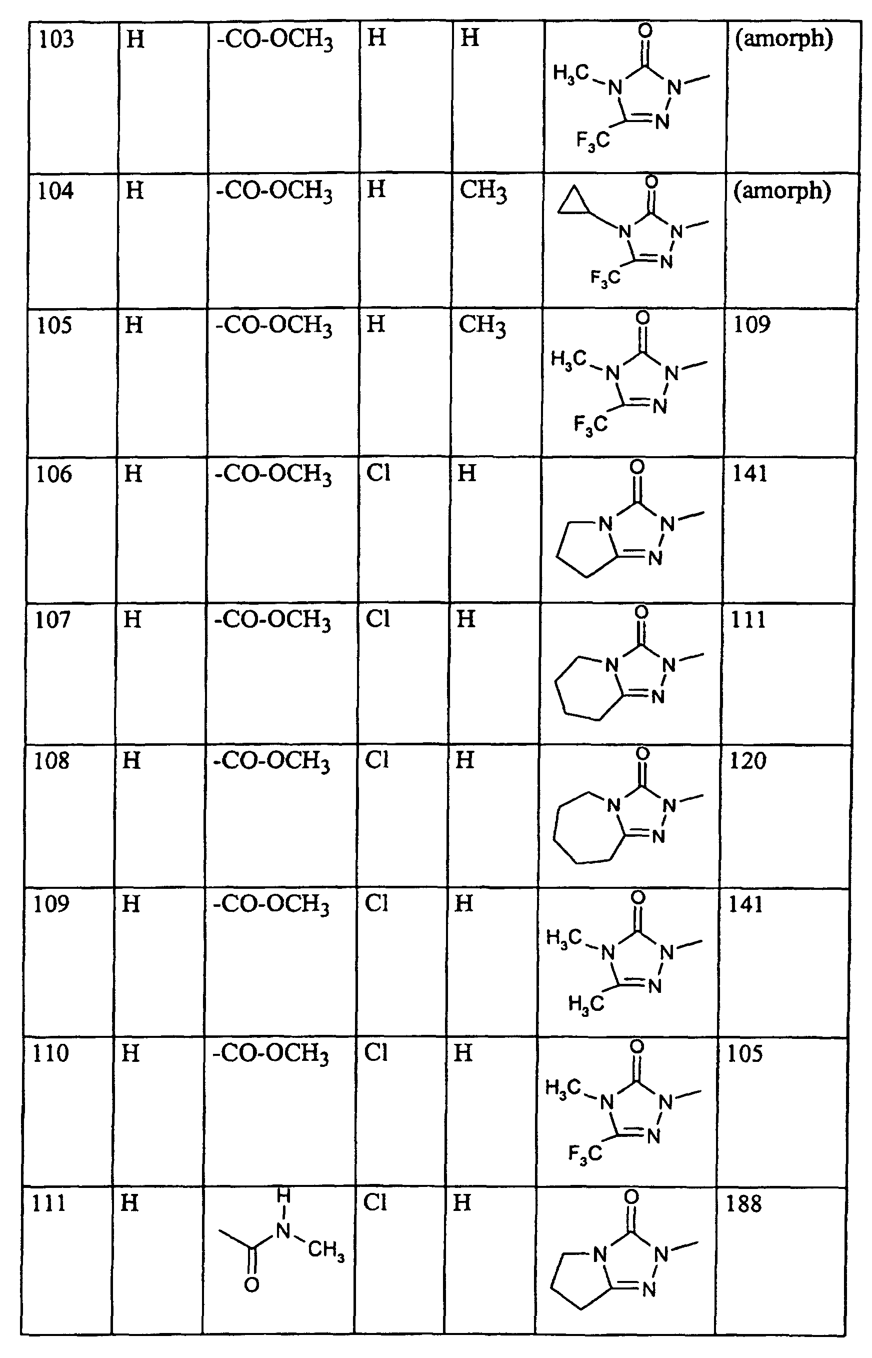

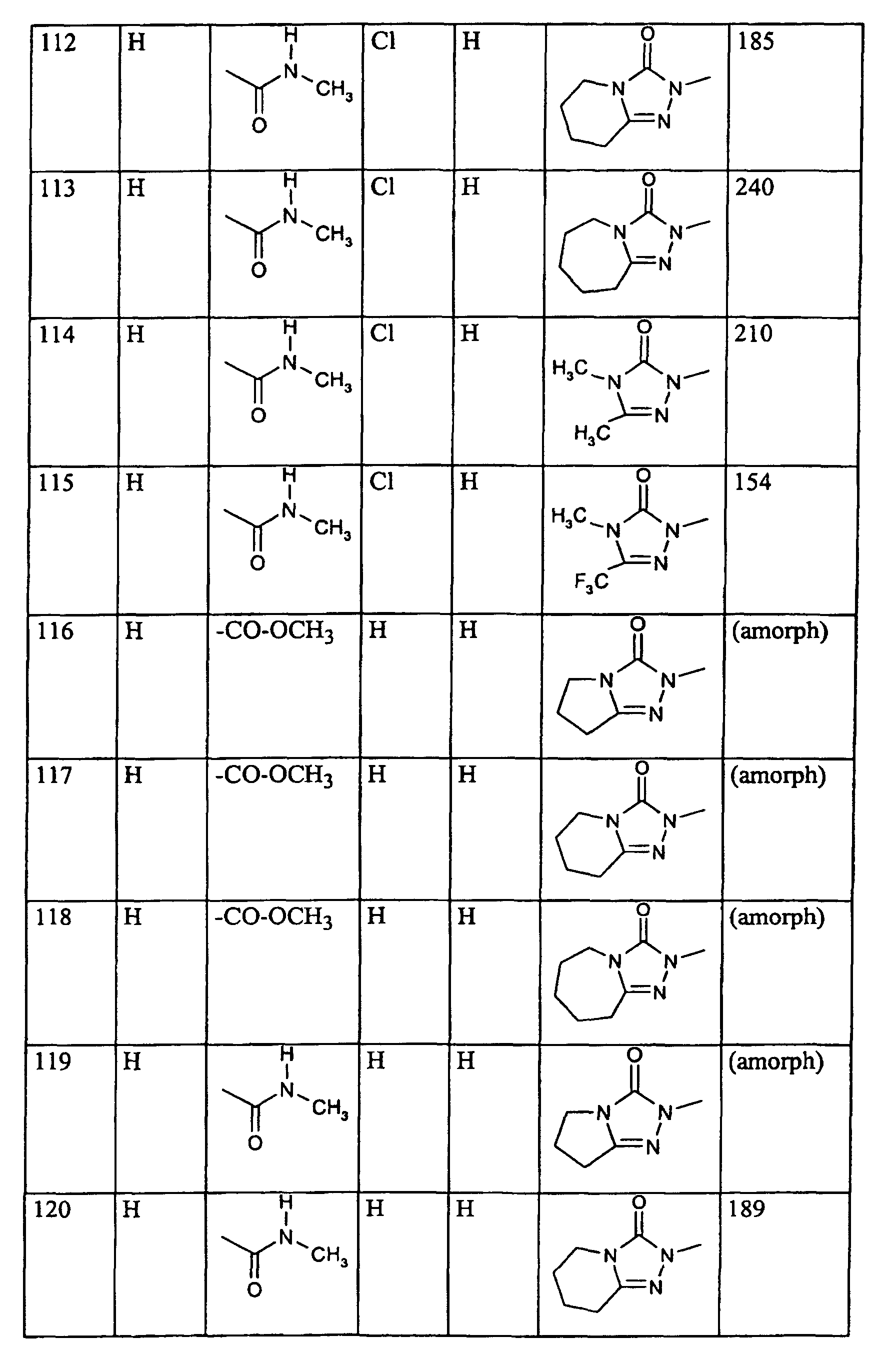

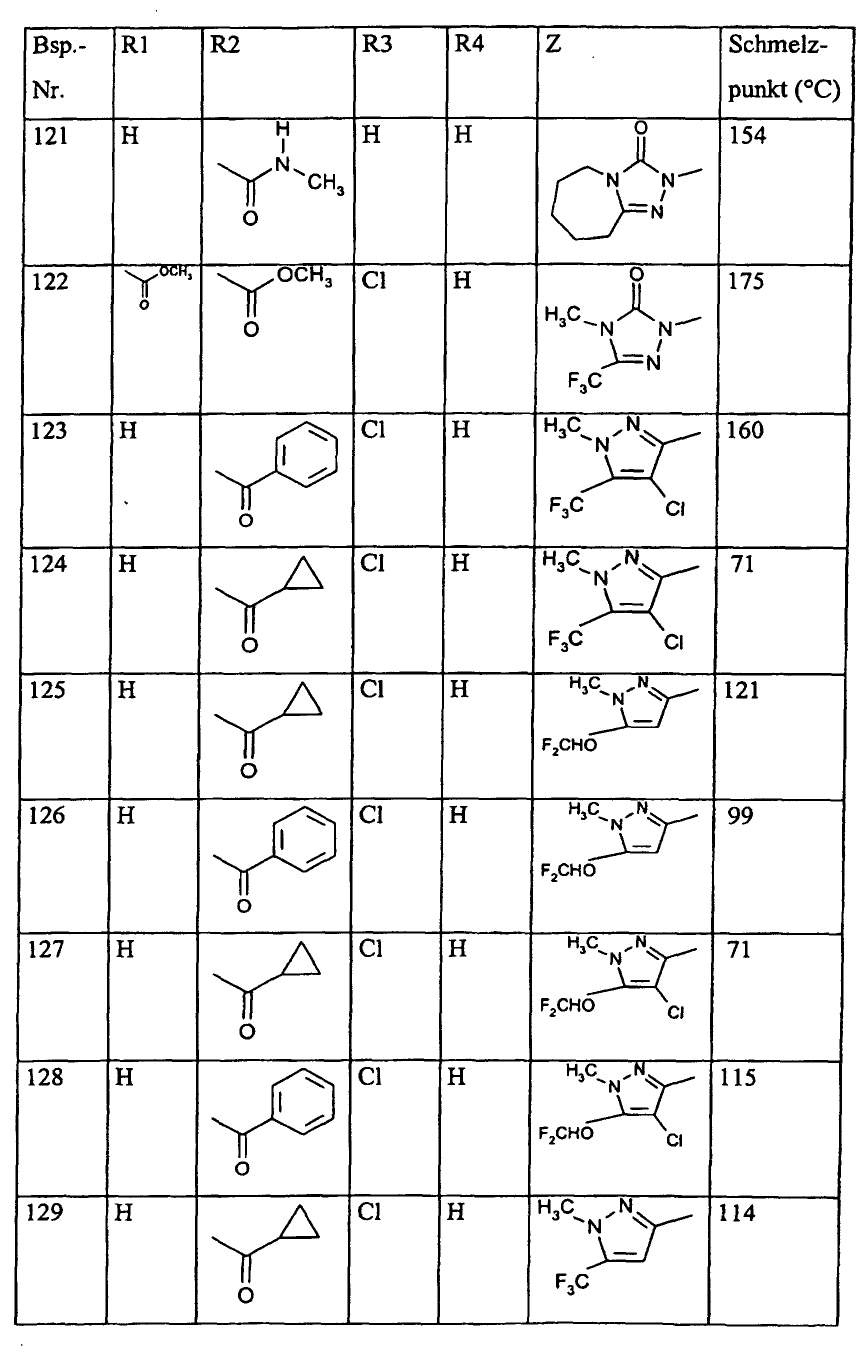

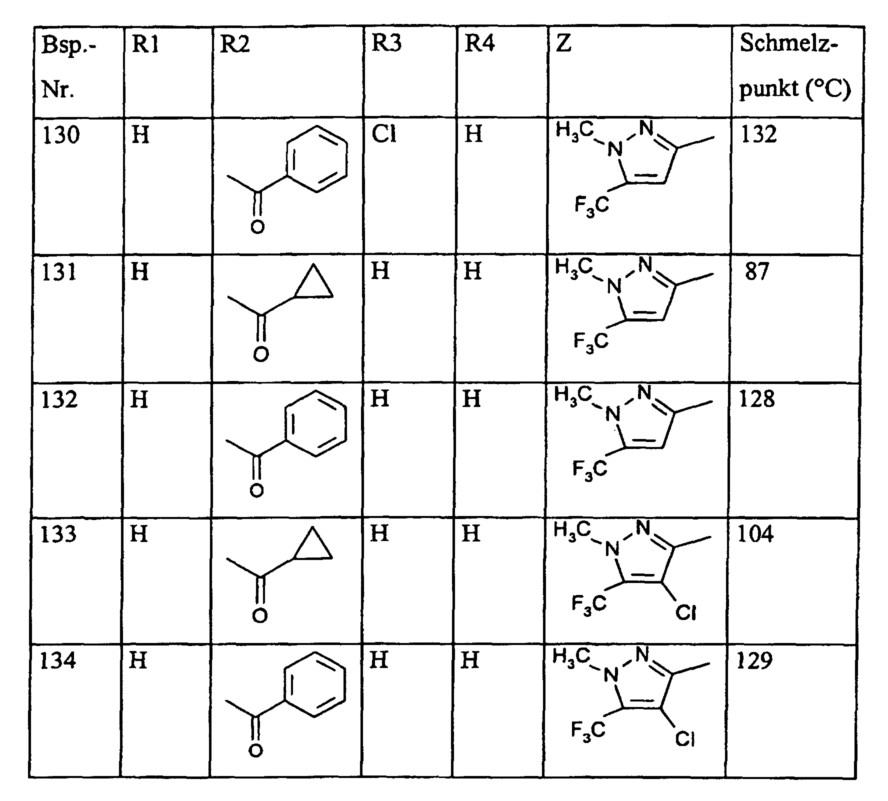

Ein weiterer Teil der erfindungsgemäßen Wirkstoffe kann durch die nachstehende allgemeine Formel (Ib) wiedergegeben werden:

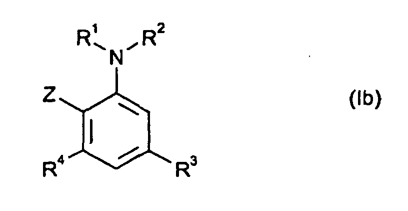

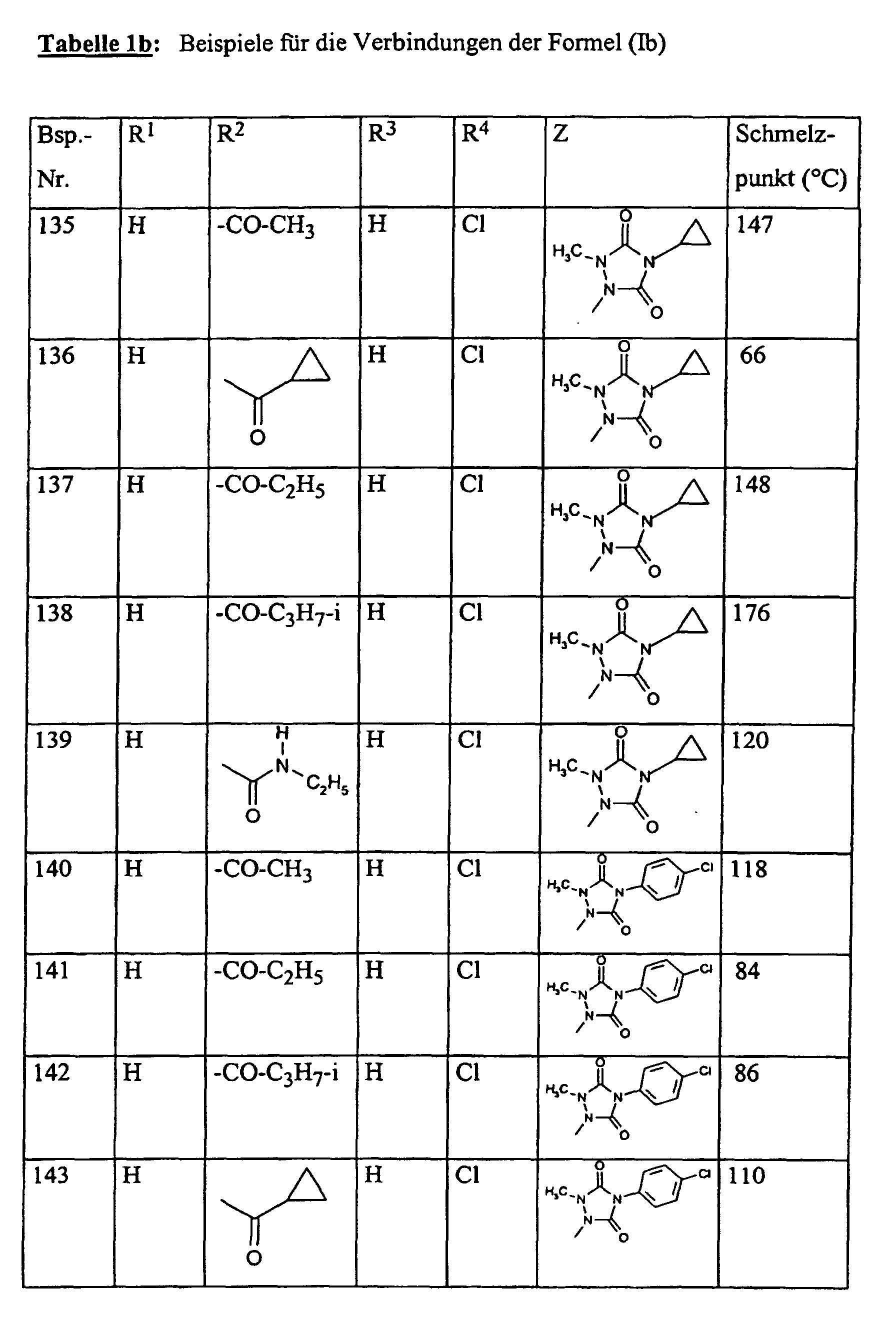

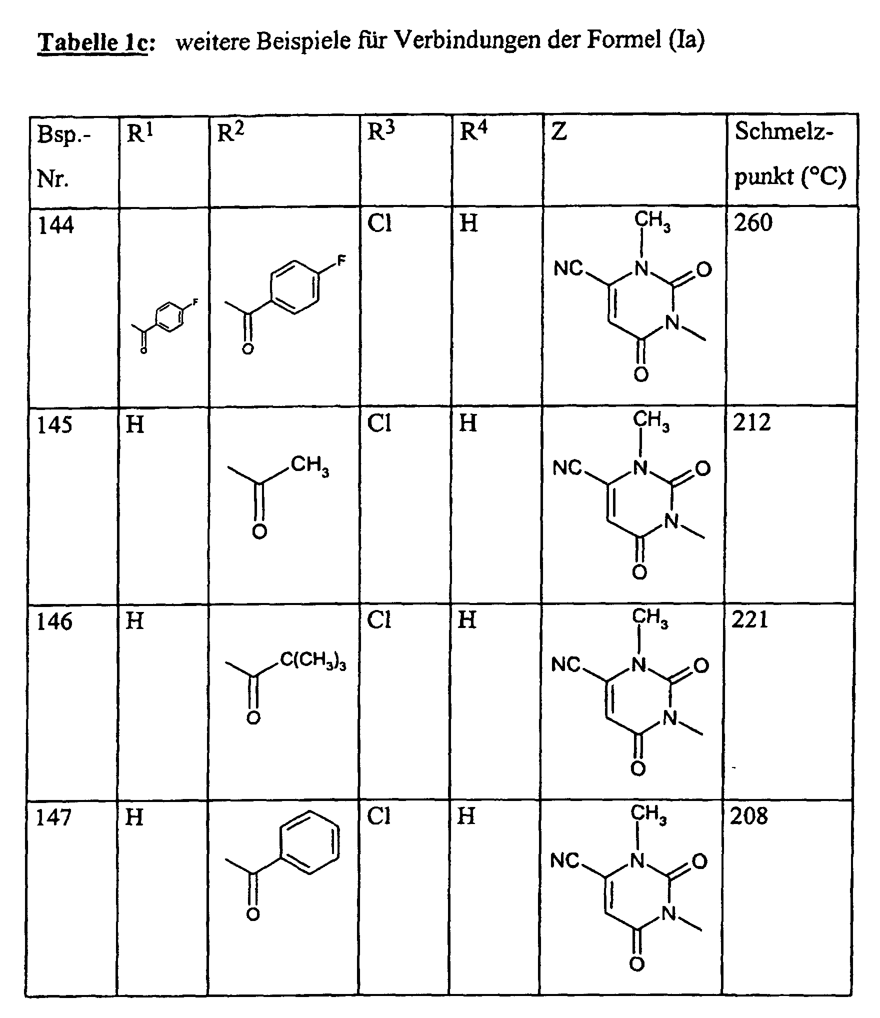

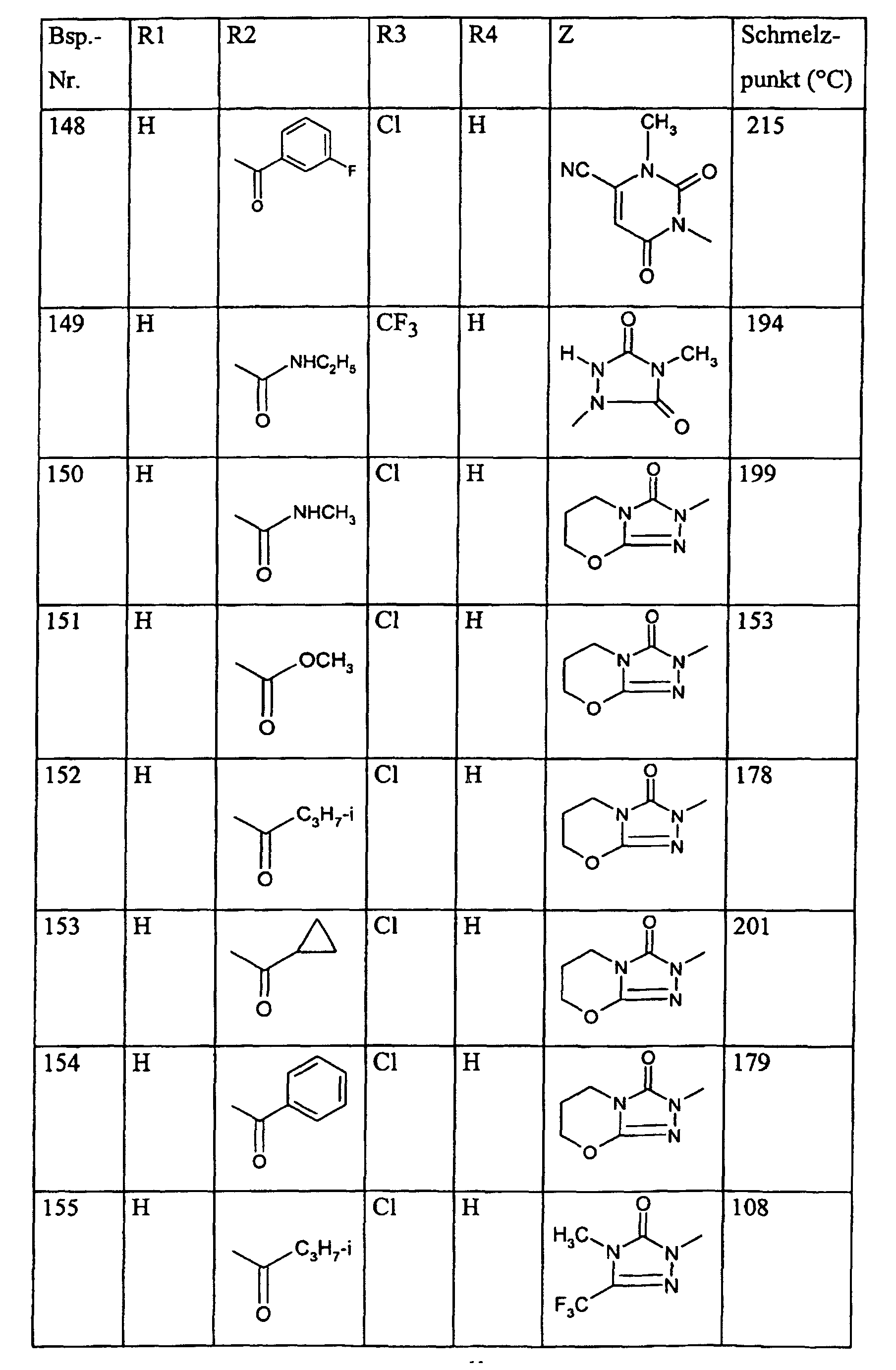

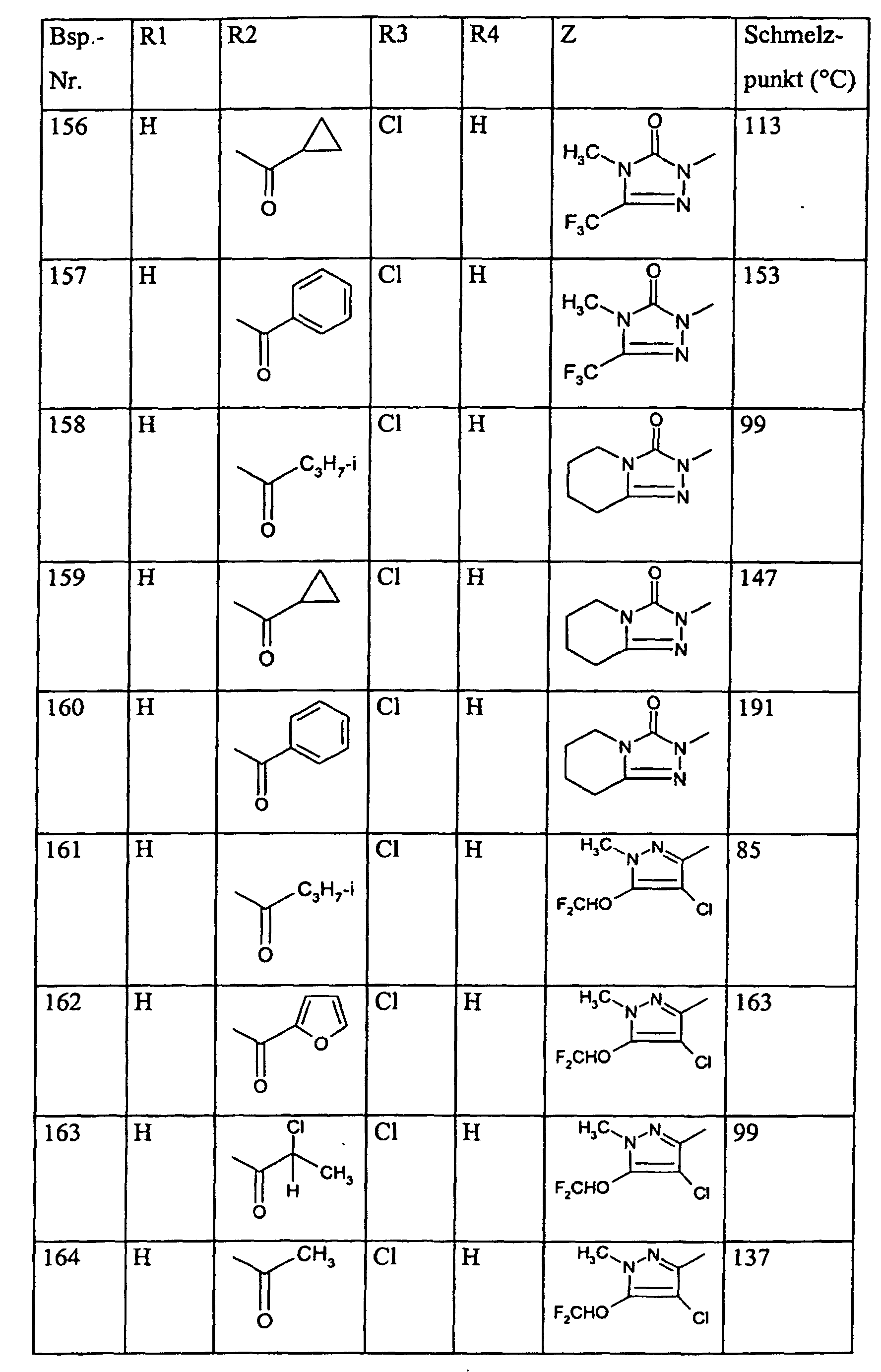

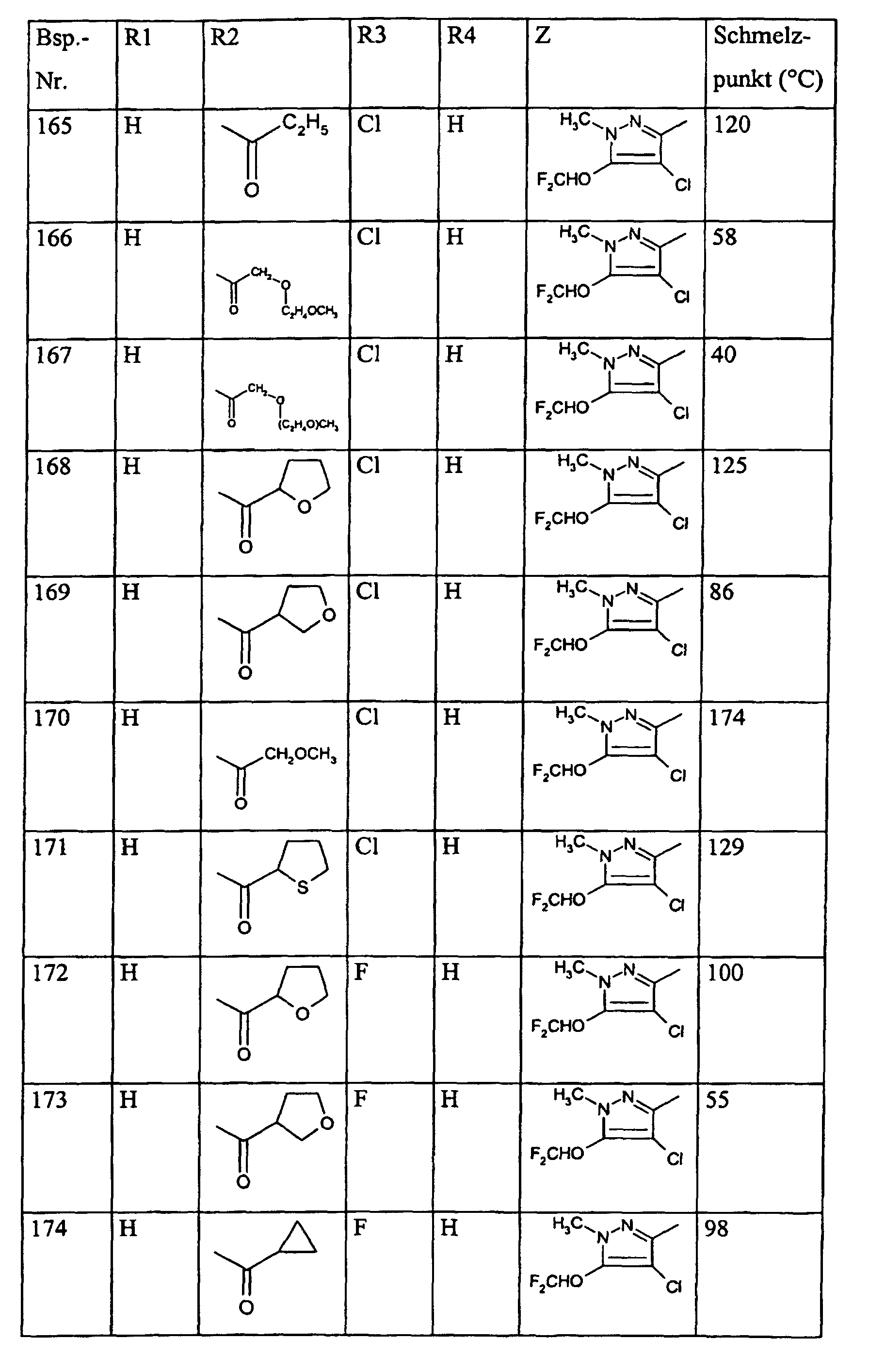

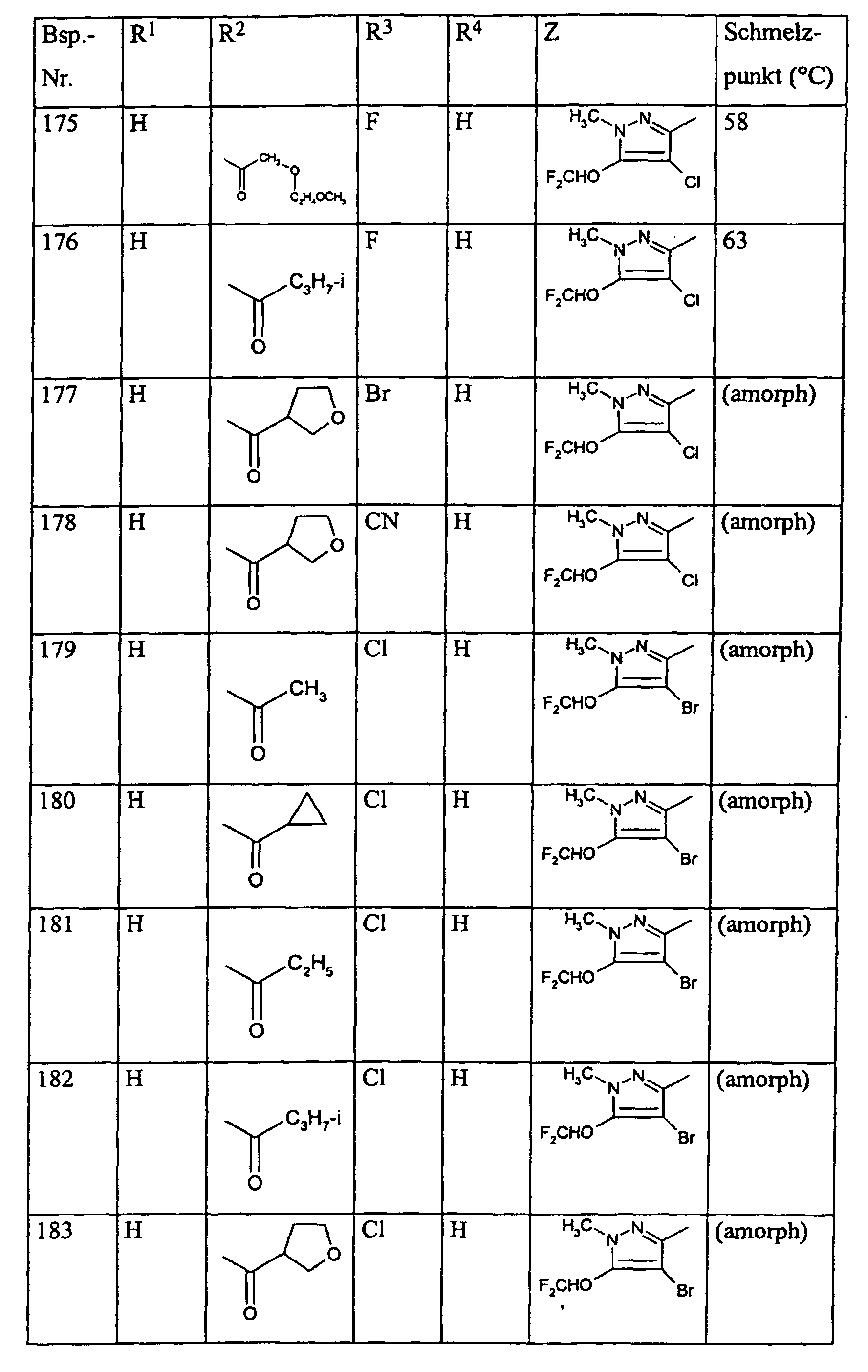

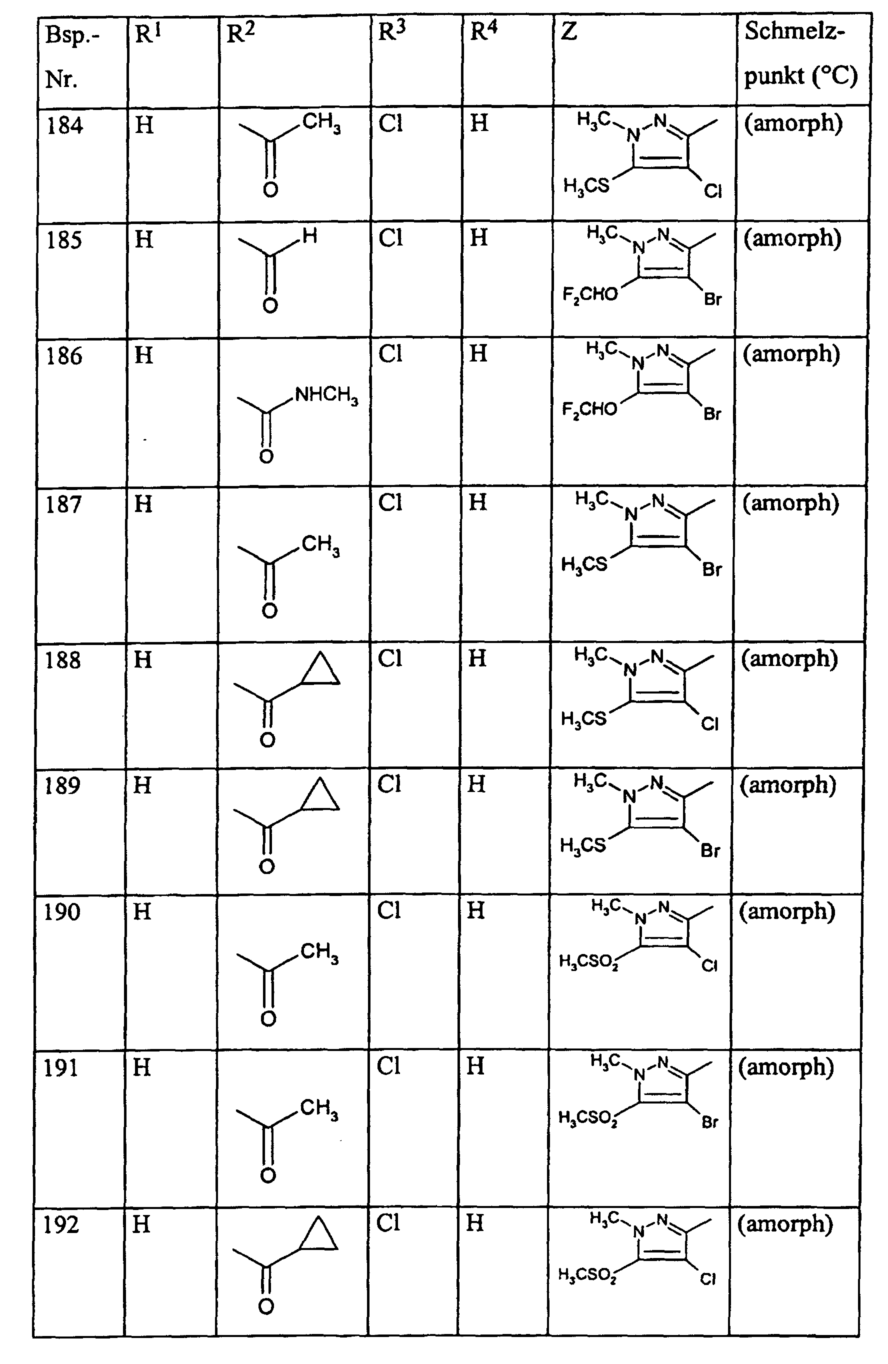

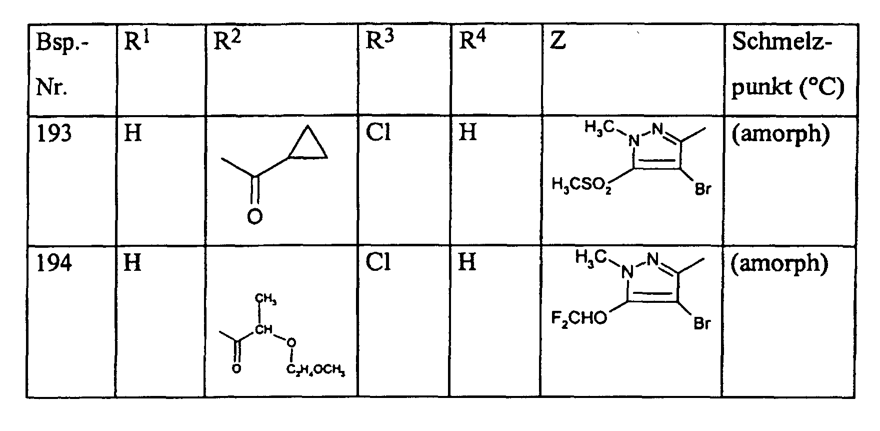

### Ausgangsstoffe der Formel (II):

### Beispiel (II-1)

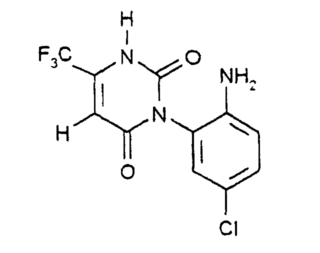

Eine Mischung aus 15,0 g (44,7 mMol) 1-(5-Chlor-2-nitro-phenyl)-3,6-dihydro-2,6-dioxo-4-trifluormethyl-1(2H)-pyrimidin, 30.25 g Zinn(II)-chlorid-Dihydrat, 20 ml Wasser und 100 ml 33%iger Salzsäure wird 45 Minuten bei 80°C gerührt und anschließend im Wasserstrahlvakuum eingeengt. Der Rückstand wird dann in Wasser aufgenommen und auf eine 5%ige wässrige Lösung von Natriumdihydrogenphosphat und auf Essigsäureethylester gegeben. Die organische Phase wird abgetrennt, mit 5%iger wässriger Lösung von Natriumdihydrogenphosphat gewaschen, mit Natriumsulfat getrocknet und filtriert. Das Filtrat wird im Wasserstrahlvakuum eingeengt, der Rückstand mit Diethylether/Petrolether digeriert und das kristallin anfallende Produkt durch Absaugen isoliert.

Man erhält 11,6 g (85% der Theorie) 1-(2-Amino-5-chlor-phenyl)-3,6-dihydro-2,6-dioxo-4-trifluormethyl-1(2H)-pyrimidin vom Schmelzpunkt 293°C.

### Beispiel (II-2)

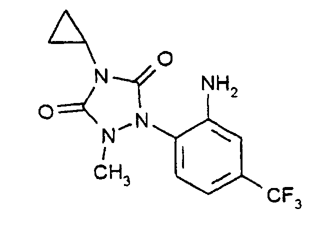

Eine Mischung aus 2,3 g (7 mMol) 1-(2-Nitro-4-trifluormethyl-phenyl)-5-cyclopropyl-2-methyl-3,5-dioxo-1,2,4-triazol, 2,4 g (42 mMol) Eisen und 100 ml 50%igem wässrigem Ethanol wird unter Rückfluß erhitzt und dabei wird eine Lösung von 0,2 ml konz. Salzsäure in 10 ml 50%igem wässrigem Ethanol tropfenweise zur Mischung gegeben. Die Reaktionsmischung wird 60 Minuten unter Rückfluß erhitzt und dann über Kieselgel abgesaugt. Vom Filtrat wird das Lösungsmittel im Wasserstrahlvakuum sorgfältig abdestilliert.

Man erhält 2,4 g (98% der Theorie) 1-(2-Amino-4-trifluormethyl-phenyl)-5-cyclopropyl-2-methyl-3,5-dioxo-1,2,4-triazol als kristallinen Rückstand vom Schmelzpunkt 154°C.

### Beispiel (II-3)

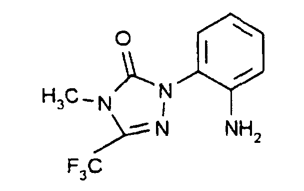

12 g (41 mMol) 4-Methyl-2-(2-nitro-phenyl)-5-trifluormethyl-2,4-dihydro-3H-1,2,4-triazol-3-on werden in 100 ml Methanol gelöst und in Gegenwart von 3 g Raney-Nickel 4 Stunden lang bei 50°C und 50 bar Wasserstoffdruck hydriert. Der erkaltete Reaktionsansatz wird filtriert und das Filtrat wird im Wasserstrahlvakuum eingeengt. Der Rückstand wird dann mit Toluol azeotrop getrocknet. Nach erneutem Einengen wird der Rückstand mit Petrolether digeriert und das kristallin anfallende Produkt durch Absaugen isoliert.

Man erhält 10 g (94% der Theorie) 2-(2-Amino-phenyl)-4-methyl-5-trifluormethyl-2,4-dihydro-3H-1,2,4-triazol-3-on vom Schmelzpunkt 101°C.

Analog zu den Herstellungsbeispielen (II-1) bis (II-3) können beispielsweise auch die in den nachstehenden Tabellen (2a und 2b) aufgeführten Verbindungen der Formel (II) hergestellt werden.

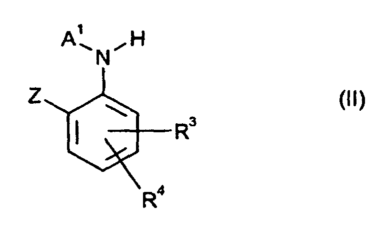

Ein großer Teil der erfindungsgemäßen Vorprodukte kann durch die nachstehende allgemeine Formel (IIa) wiedergegeben werden:

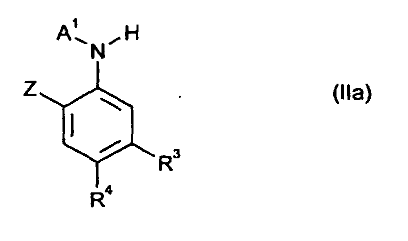

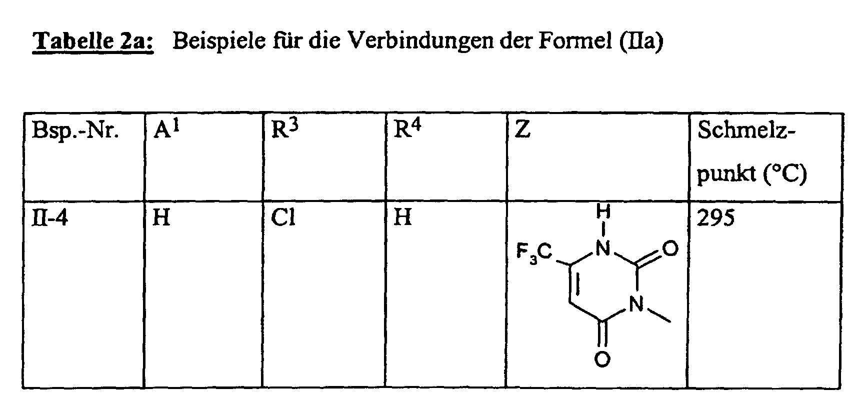

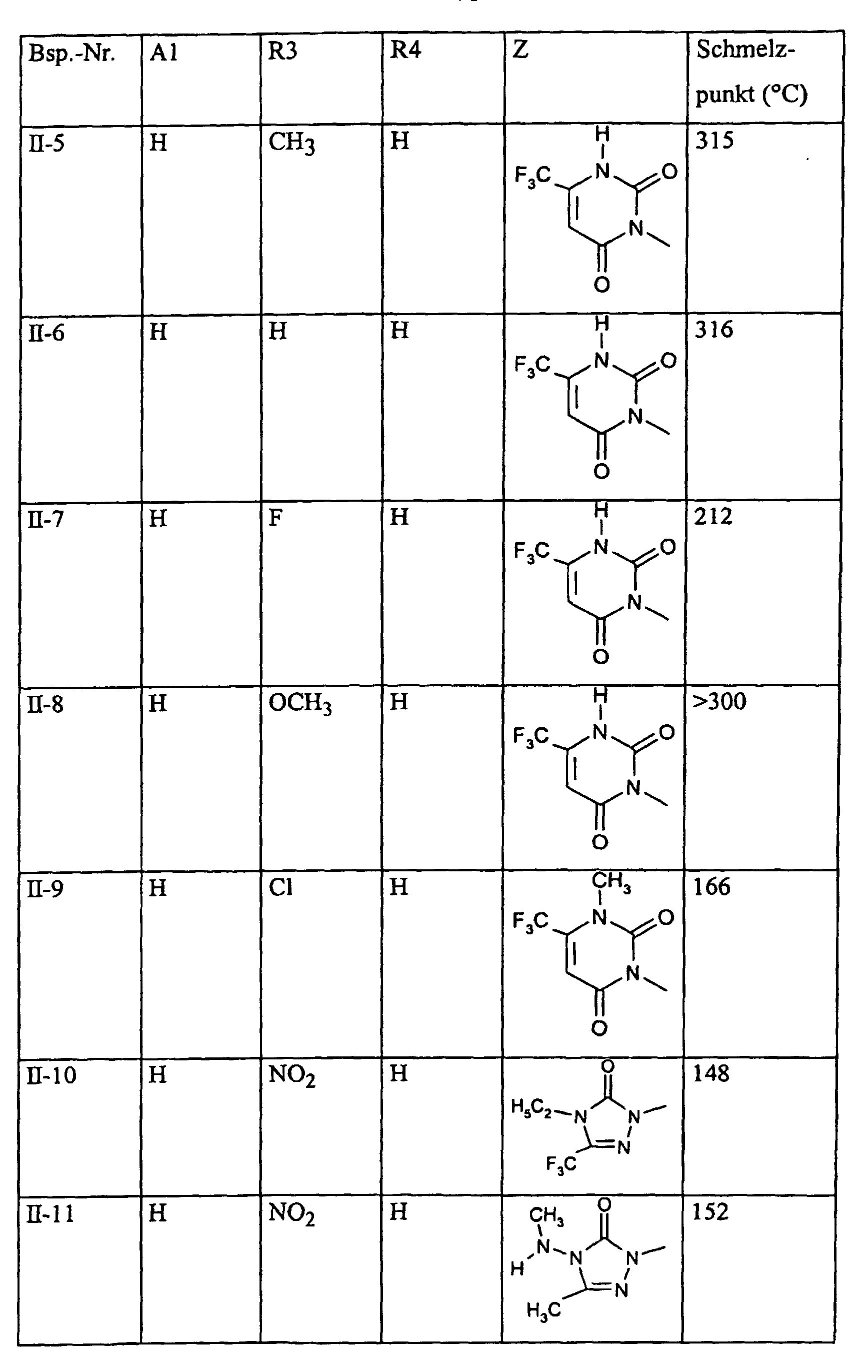

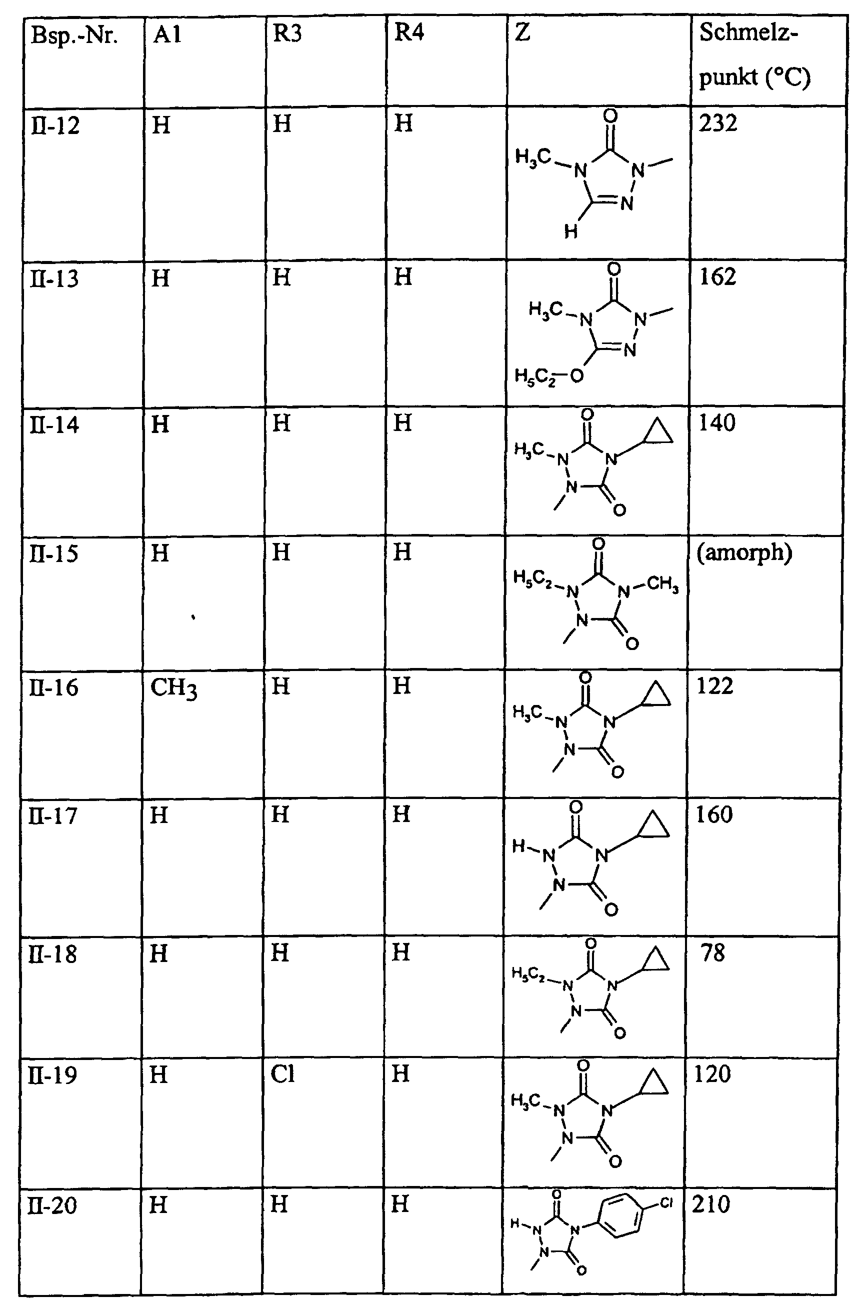

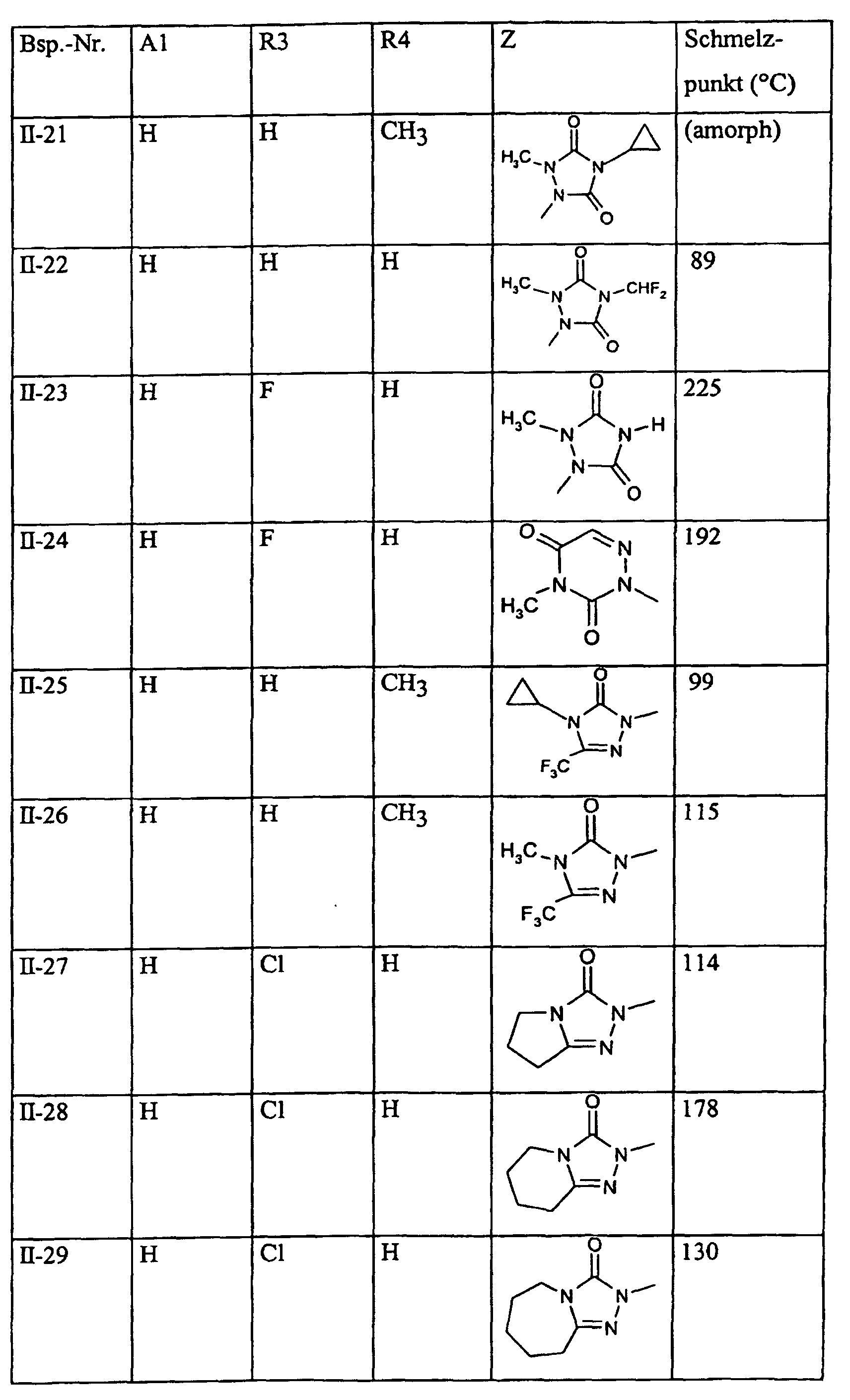

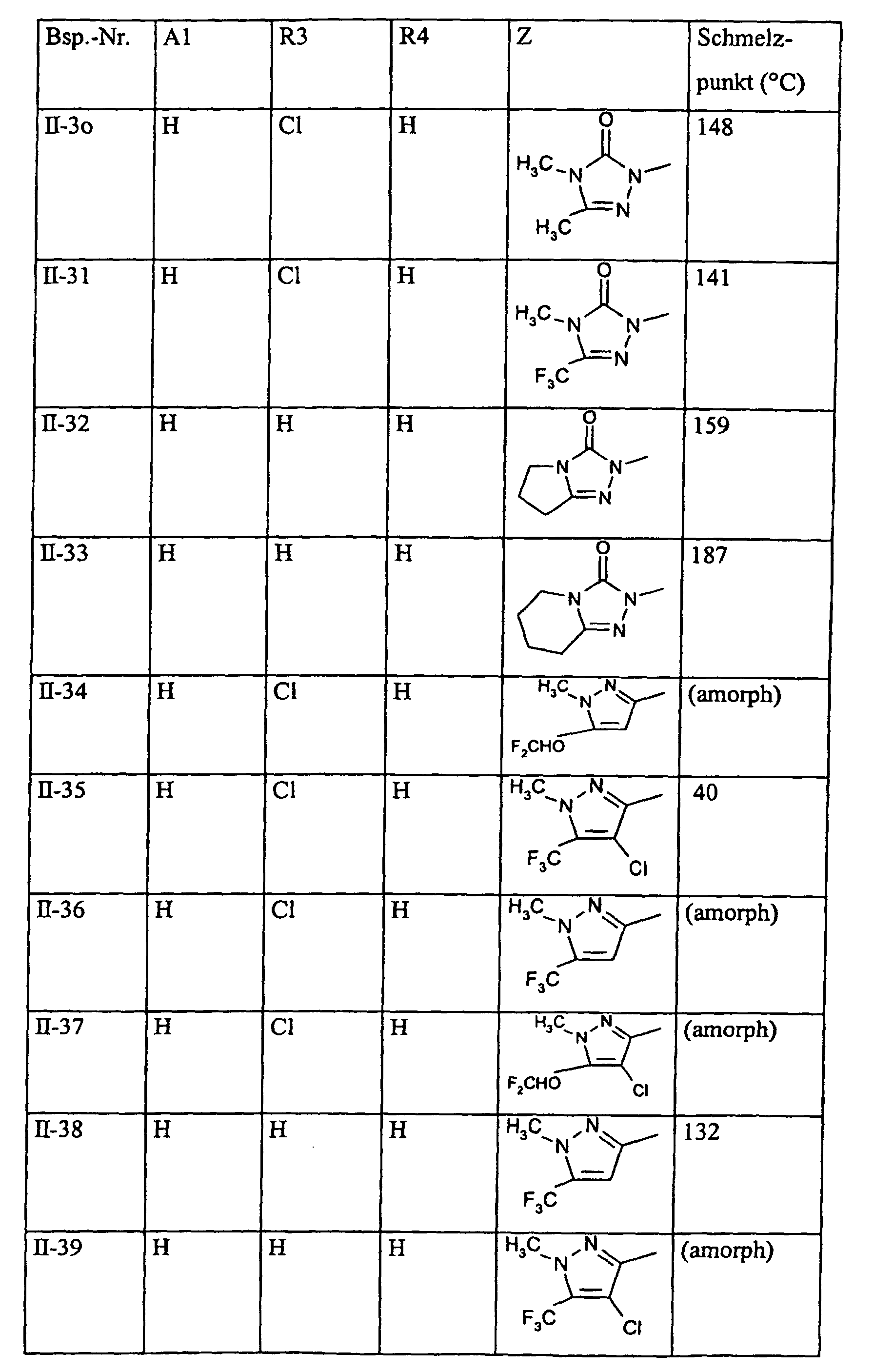

Ein weiterer Teil der erfindungsgemäßen Vorprodukte kann durch die nachstehende allgemeine Formel (IIb) wiedergegeben werden:

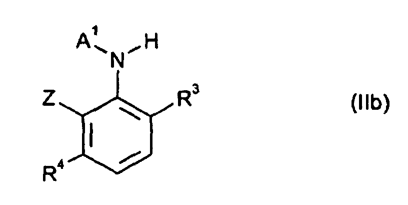

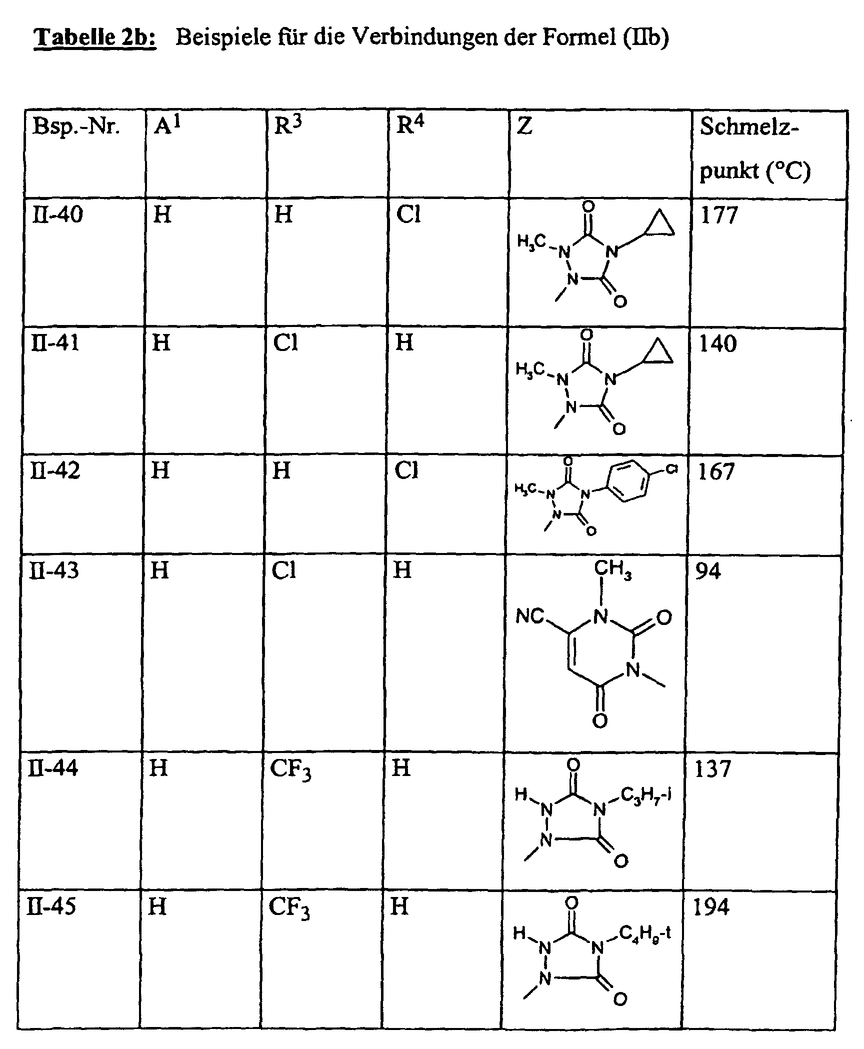

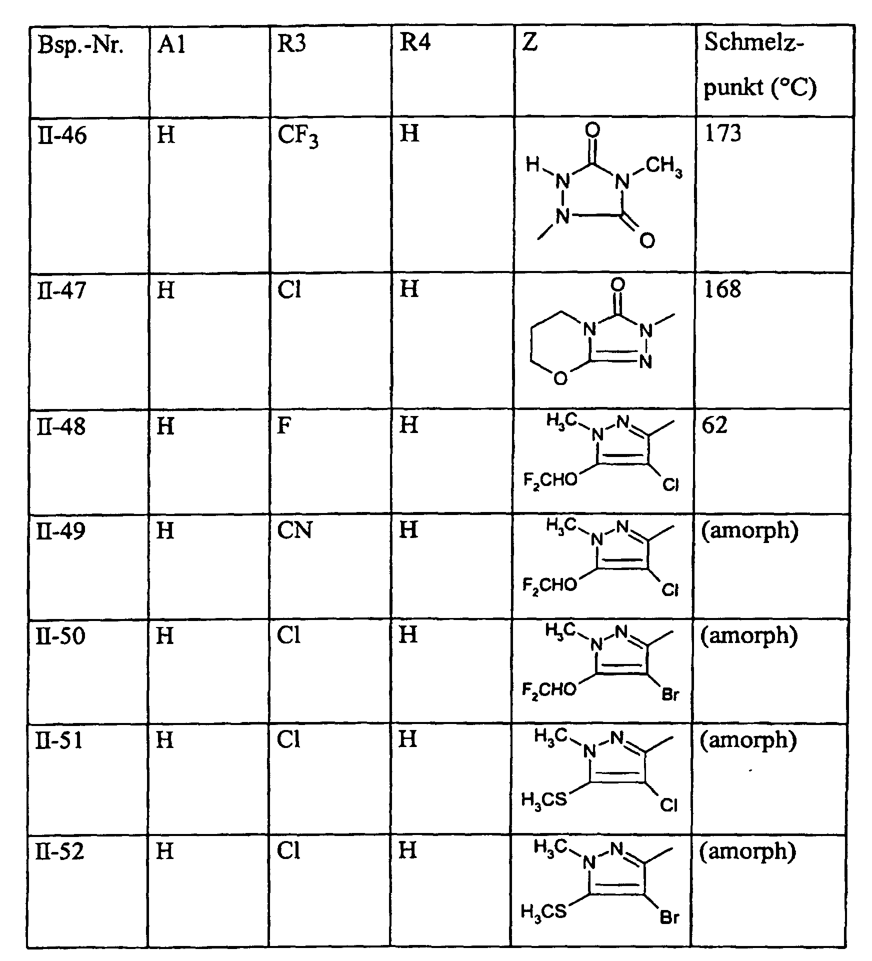

### Ausgangsstoffe der Formel (IV):

### Beispiel (IV-1)

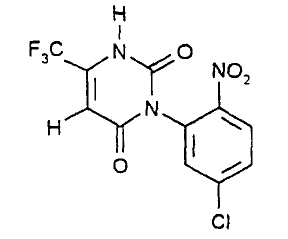

### Stufe 1

118,9 g (109,5 mMol) 5-Chlor-2-nitro-anilin werden in 150 ml Aceton gelöst und zu dieser Lösung werden bei Raumtemperatur (ca. 20°C) unter Rühren 27 g (128 mMol) Chlorameisensäure-trichlormethylester ("Diphosgen") tropfenweise gegeben. Die Reaktionsmischung wird 30 Minuten gerührt und dann tropfenweise mit 150 ml Ethanol versetzt. Nach weiteren 30 Minuten wird im Wasserstrahlvakuum eingeengt, der Rückstand mit Wasser/Petrolether digeriert und das kristallin anfallende Produkt durch Absaugen isoliert.

Man erhält 22,4 g (84% der Theorie) N-(5-Chlor-2-nitro-phenyl)-O-ethyl-urethan vom Schmelzpunkt 86°C.

### Stufe 2

25 g (0,10 Mol) 3-Amino-4,4,4-trifluor-crotonsäure-ethylester werden in 100 ml N,N-Dimethyl-formamid vorgelegt. Nach Zugabe von 3,5 g (0,15 Mol) Natriumhydrid wird die Mischung 30 Minuten bei Raumtemperatur (ca. 20°C) gerührt und dann mit 24,5 g (0,10 Mol) N-(5-Chlor-2-nitro-phenyl)-O-ethyl-urethan (vgl. Stufe 1) versetzt. Die Reaktionsmischung wird dann 150 Minuten auf 130°C erhitzt und anschließend auf etwa das gleiche Volumen Eiswasser gegeben. Man schüttelt zweimal mit Essigsäureethylester, säuert dann die wässrige Phase mit 2N-Salzsäure an und schüttelt erneut mit Essigsäureethylester. Die organische Phase wird mit Wasser gewaschen, mit Natriumsulfat getrocknet und filtriert. Das Filtrat wird im Wasserstrahlvakuum eingeengt, der Rückstand mit 5 ml Essigsäureethylester, 50 ml Diethylether und 50 ml Petrolether digeriert und das kristallin anfallende Produkt durch Absaugen isoliert.

Man erhält 21 g (62,5% der Theorie) 1-(5-Chlor-2-nitro-phenyl)-3,6-dihydro-2,6-dioxo-4-trifluormethyl-1(2H)-pyrimidin vom Schmelzpunkt 214°C.

### Beispiel (IV-2)

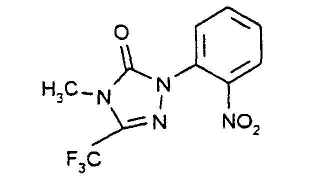

8,4 g (0,05 Mol) 4-Methyl-5-trifluormethyl-2,4-dihydro-3H-1,2,4-triazol-3-on werden in 50 ml Dimethylsulfoxid gelöst und mit 6,9 g (0,05 Mol) Kaliumcarbonat (Pulver) und 7,0 g (0,05 Mol) 2-Fluor-nitrobenzol versetzt. Die Reaktionsmischung wird dann unter Rühren 16 Stunden lang auf 80°C erhitzt und nach Abkühlen auf 200 ml Wasser gegossen. Man extrahiert zweimal mit Methylenchlorid, wäscht die vereinigten organischen Phasen mit Wasser, trocknet mit Magnesiumsulfat und filtriert. Das Filtrat wird im Wasserstrahlvakuum eingeengt, der Rückstand mit Diethylether digeriert und das kristallin anfallende Produkt durch Absaugen isoliert.

Man erhält 12,7 g (88% der Theorie) 4-Methyl-2-(2-nitro-phenyl)-5-trifluormethyl-2,4-dihydro-3H-1,2,4-triazol-3-on vom Schmelzpunkt 83°C.

Analog zu den Herstellungsbeispielen (IV-1) und (IV-2) können beispielsweise auch die in den nachstehenden Tabellen (3a und 3b) aufgeführten Verbindungen der Formel (IV) hergestellt werden.

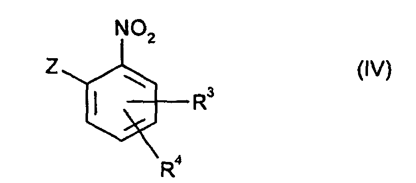

Ein großer Teil der erfindungsgemäßen Vorprodukte kann durch die nachstehende allgemeine Formel (IVa) wiedergegeben werden:

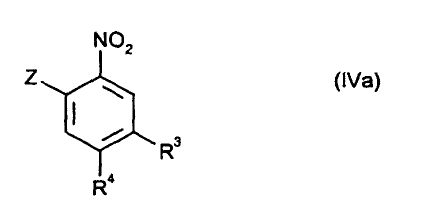

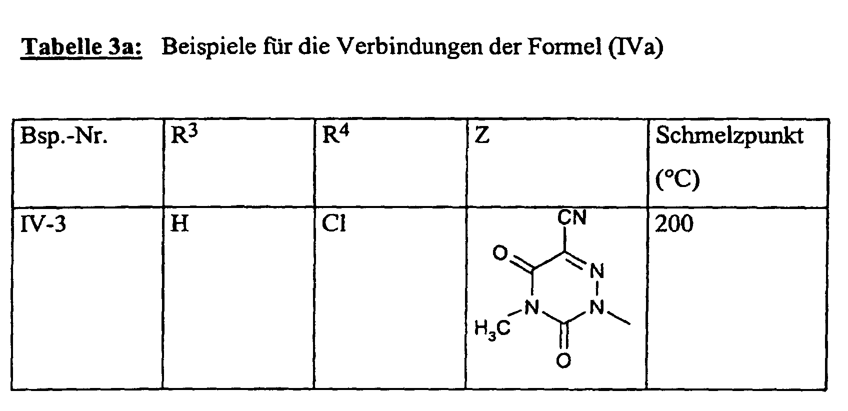

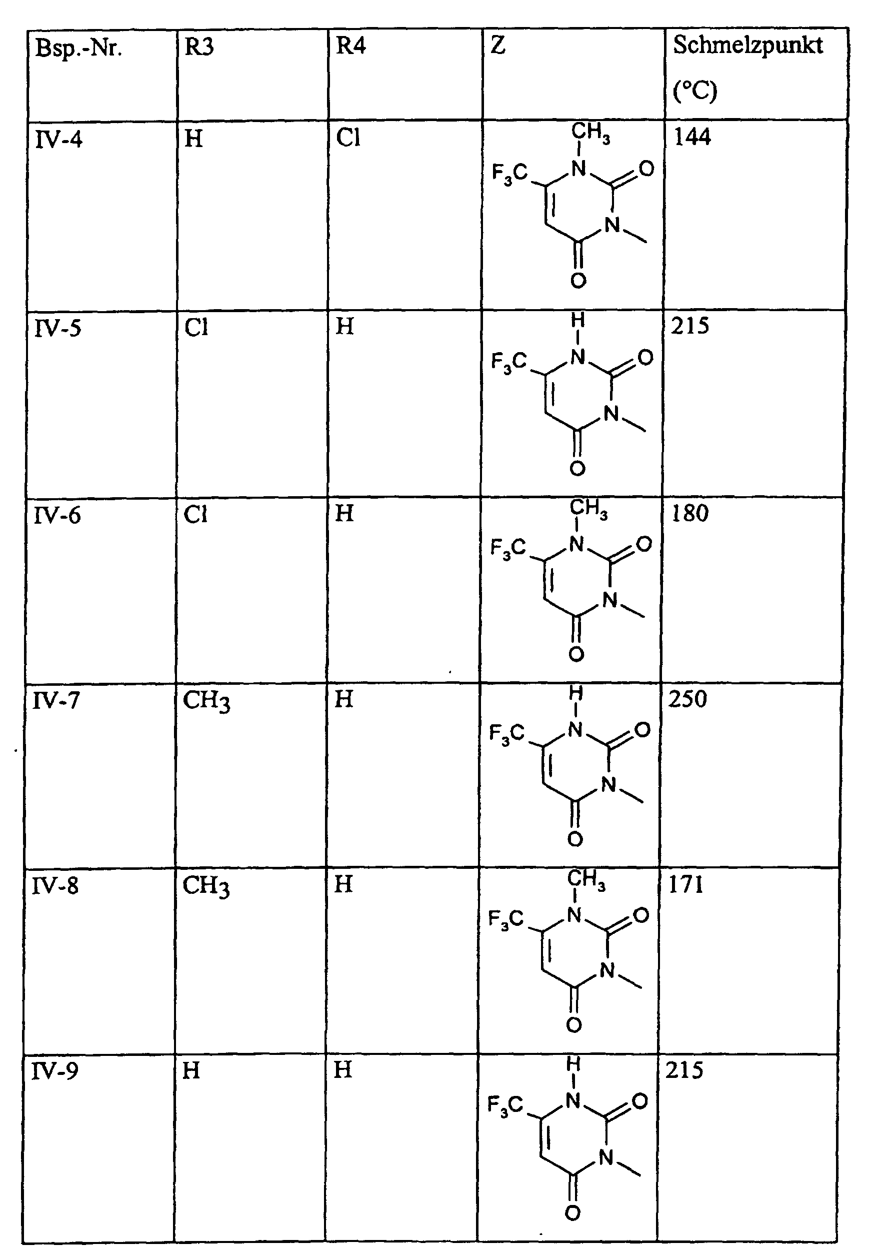

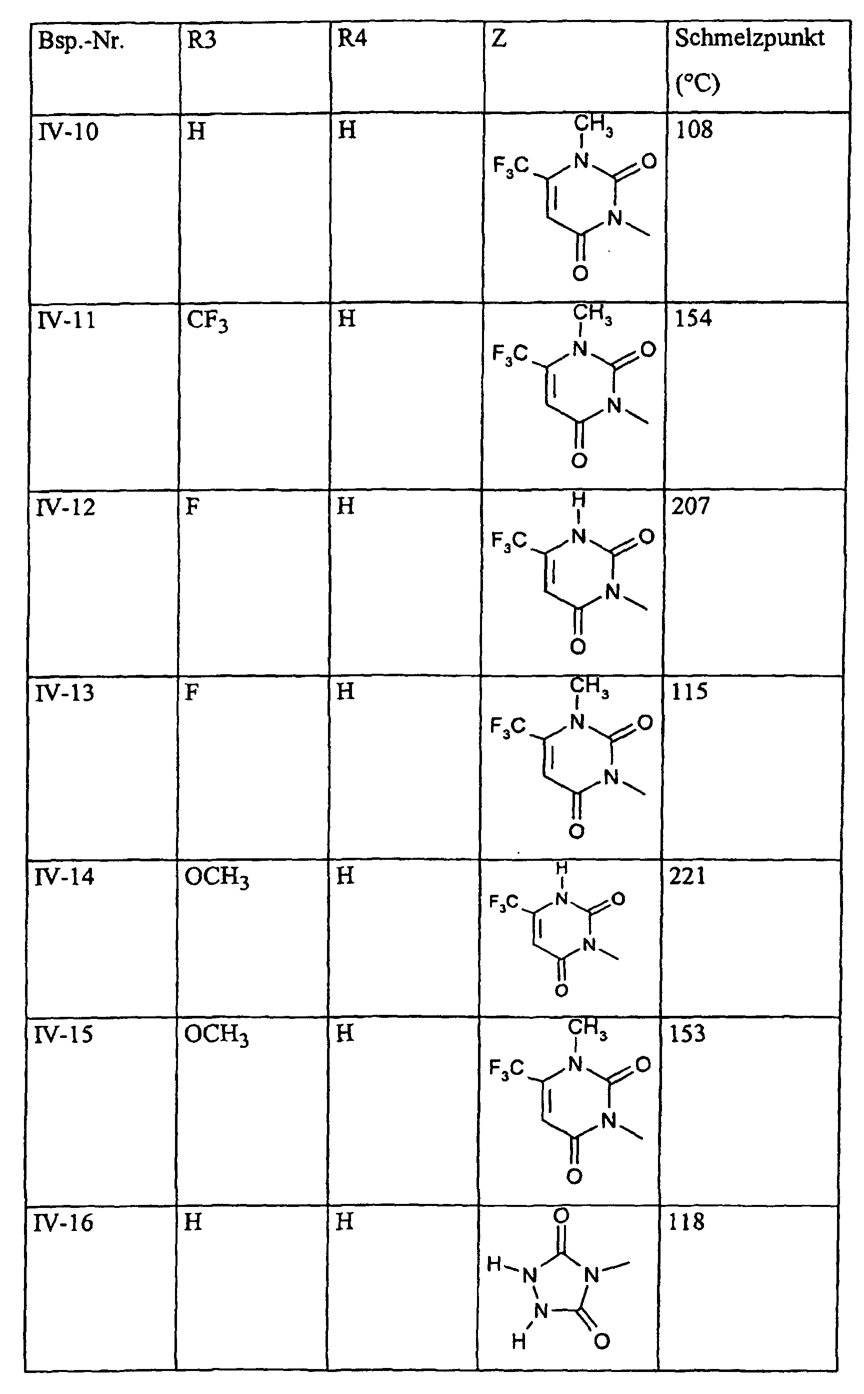

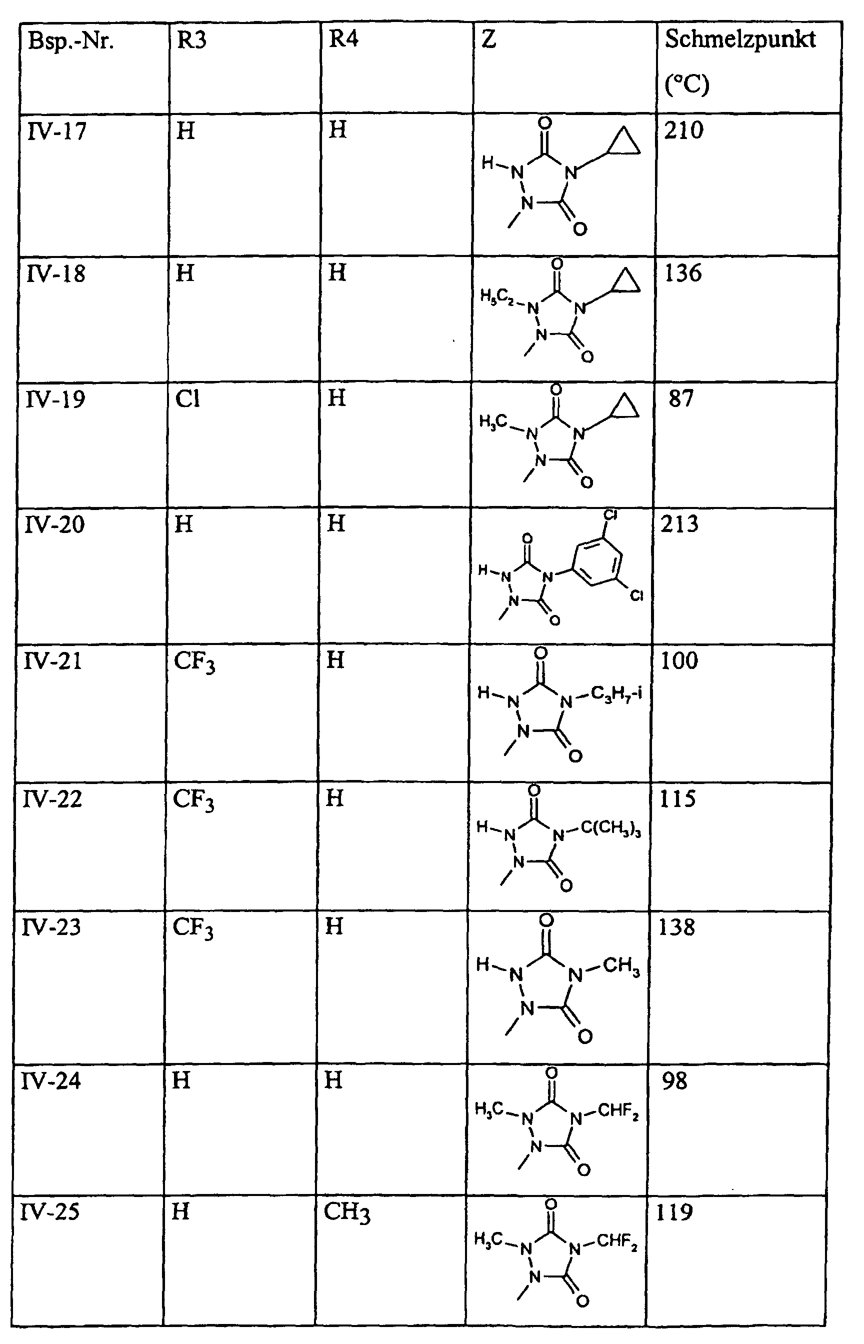

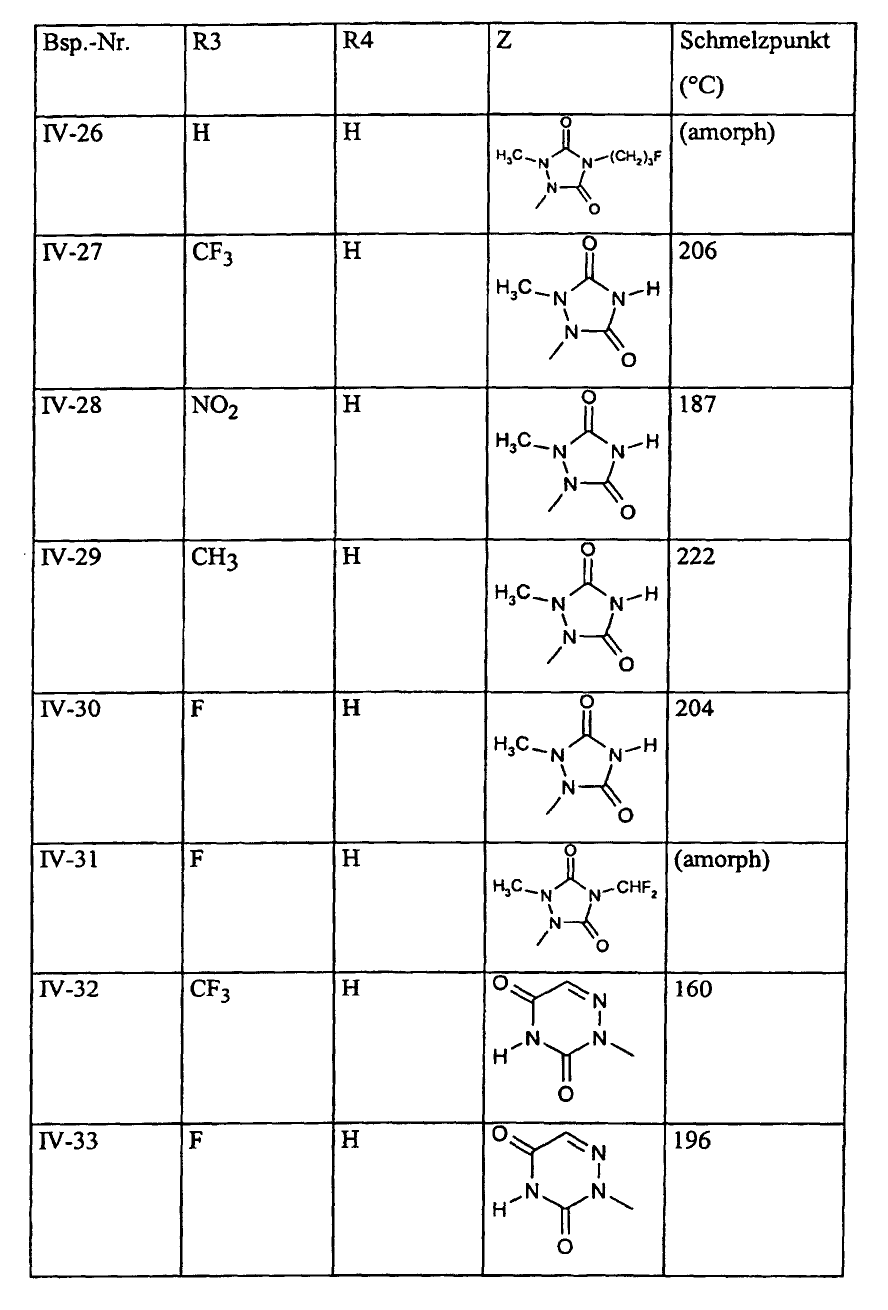

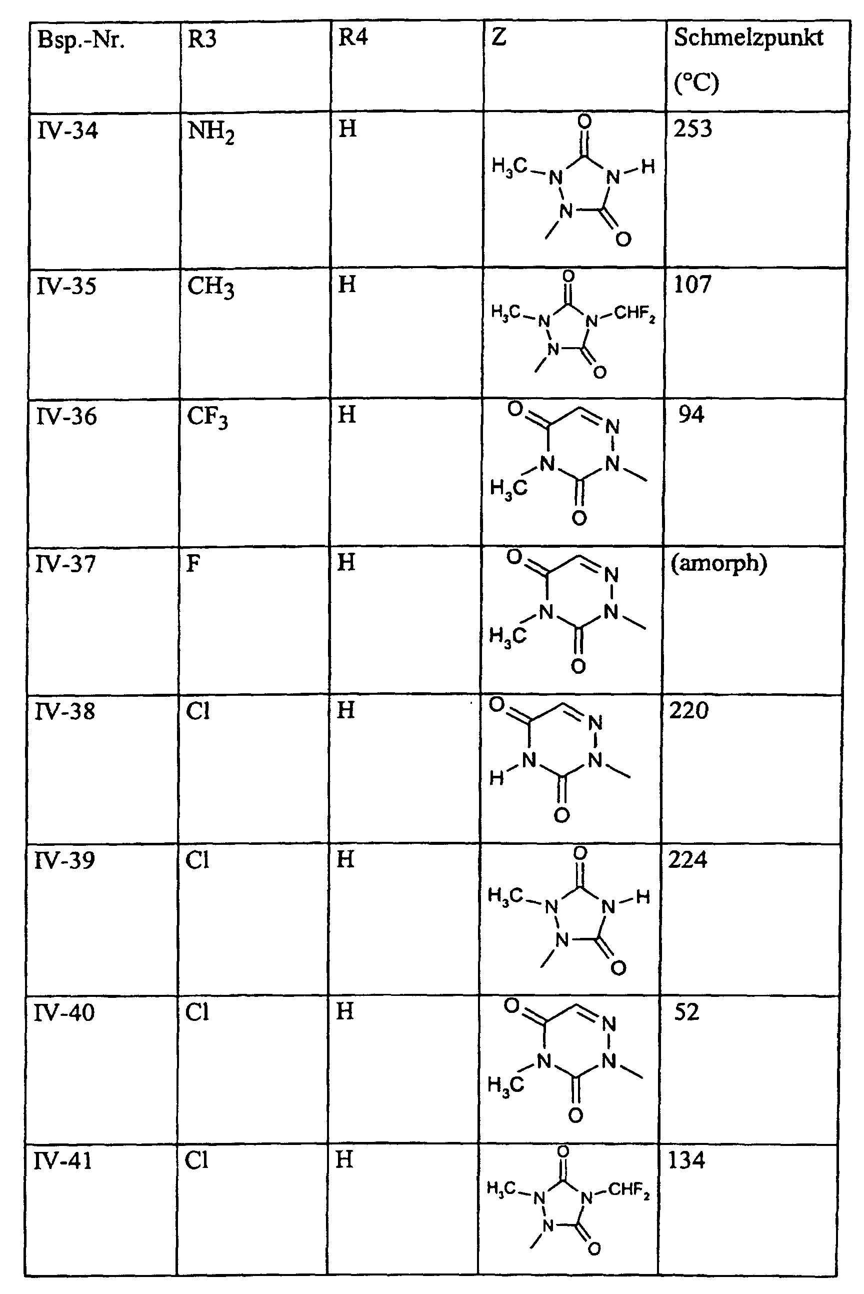

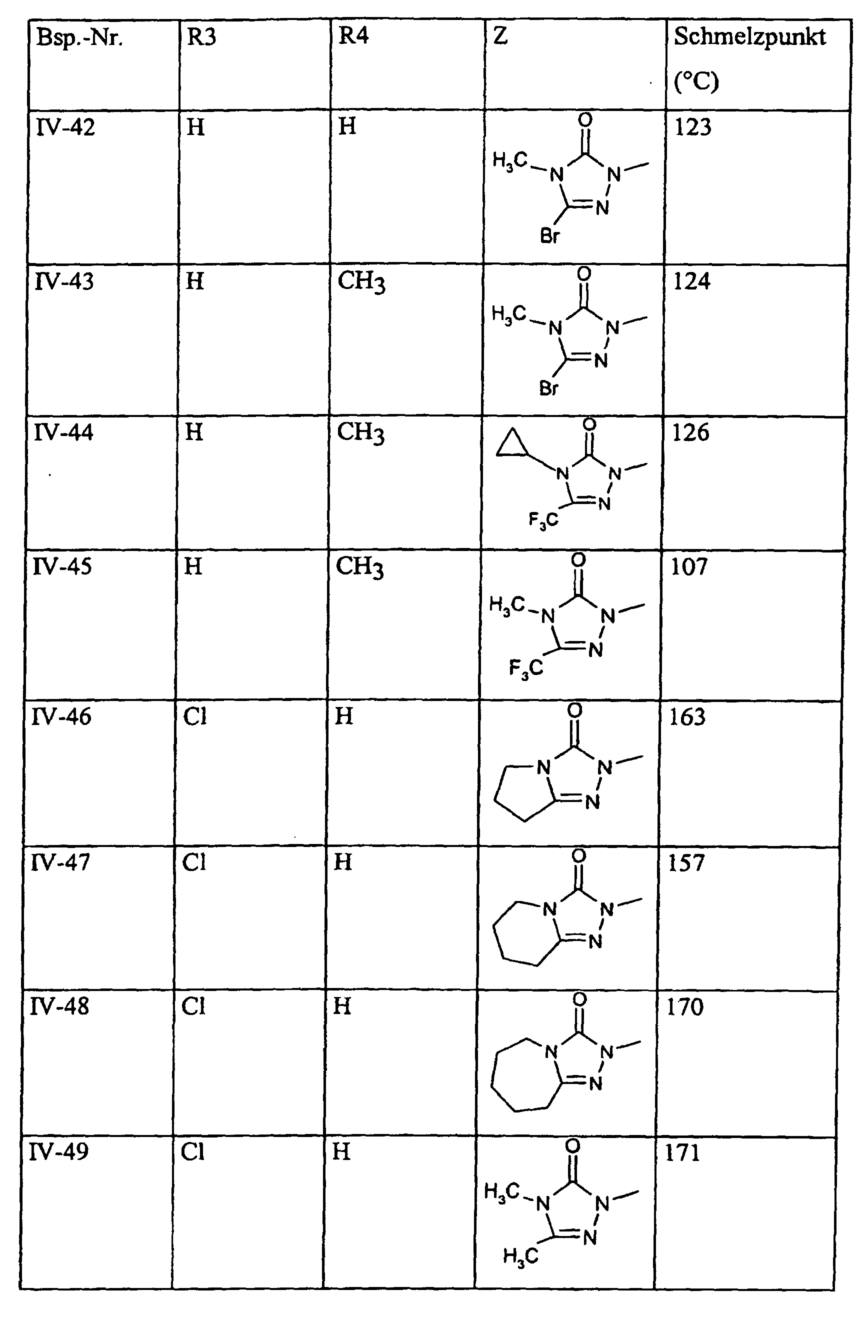

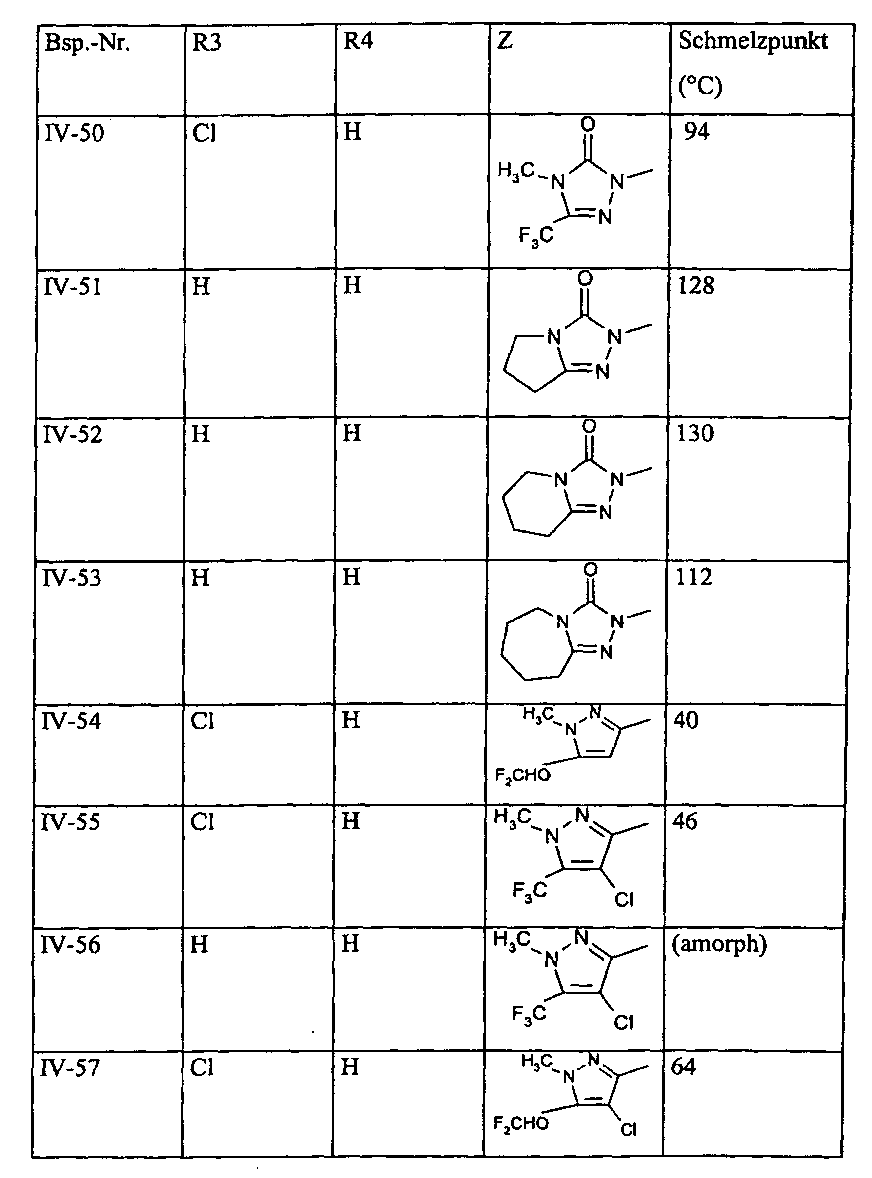

Ein weiterer Teil der erfindungsgemäßen Vorprodukte kann durch die nachstehende allgemeinen Formel (IVb) wiedergegeben werden:

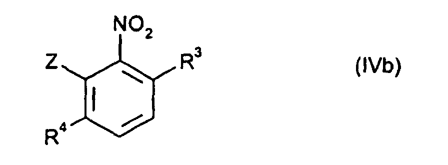

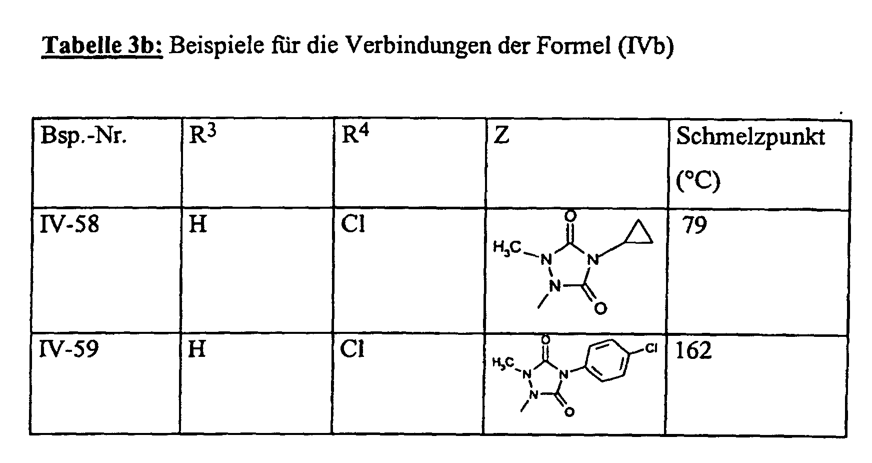

### Anwendungsbeispiele:

### Beispiel A

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät. Nach ca. 24 Stunden wird der Boden mit der Wirkstoffzubereitung so gespritzt, daß die jeweils gewünschten Wirkstoffmengen pro Flächeneinheit ausgebracht werden. Die Konzentration der Spritzbrühe wird so gewählt, daß in 1 000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen bei Aufwandmengen zwischen 250 und 750 g/ha beispielsweise die Verbindungen gemäß Herstellungsbeispiele 18, 19, 20, 23, 24 und 81 bei guter Verträglichkeit gegenüber Kulturpflanzen, wie z.B. Weizen (0 %), starke Wirkung gegen Unkräuter, wie Avena fatua (100 %), Cyperus (70-100 %), Setaria (90-100 %), Abutilon (70-100 %), Amaranthus (90-100 %), Galium (90-100 %), Sinapis (95-100 %), Lolium (100 %), Ipomoea (95-100 %), Matricaria (100 %) und Solanum (100%).

### Beispiel B

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen bei Aufwandmengen von 15-750 g/ha beispielsweise die Verbindungen gemäß Herstellungsbeispiele 18, 19, 20, 23, 24 und 81 bei guter Verträglichkeit gegenüber Kulturpflanzen, wie z.B. Weizen (5-10 %), starke Wirkung gegen Unkräuter wie Avena fatua (80-95 %), Cyperus (90-95 %), Setaria (95-100 %), Abutilon (100 %), Amaranthus (95-100 %), Galium (100 %), Sinapis (100 %), Chenopodium (95-100 %), Matricaria (95-100 %), Polygonum (95-100 %) und Viola (90-100 %).

## Patentansprüche

1. Aromatische Aminoverbindungen der allgemeinen Formel (I), 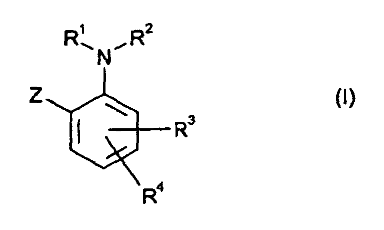 in welcher
R¹ für Wasserstoff, Hydroxy, Amino, für Alkyl, Alkoxy, Alkylamino oder Dialkylamino mit jeweils 1 bis 6 Kohlenstoffatomen in den Alkylgruppen, oder für eine der nachstehenden Gruppierungen steht,
-CQ¹-R⁵, -CQ¹-Q²-R⁶,
R² für eine der nachstehenden Gruppierungen steht,
-CQ¹-R⁵, -CQ¹-Q²-R⁶,
R³ für Wasserstoff, Hydroxy, Amino, Carboxy, Cyano, Carbamoyl, Thiocarbamoyl, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in den Alkylgruppen, oder für eine der nachstehenden Gruppierungen steht,
-SO₂-NH-R⁵, -NH-SO₂-R⁷, -N(SO₂-R⁷)₂, -N(SO₂-R⁷)(CO-R⁵),
R⁴ für Wasserstoff, Hydroxy, Amino, Carboxy, Cyano, Carbamoyl, Thiocarbamoyl, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in den Alkylgruppen, oder für eine der nachstehenden Gruppierungen steht,
-SO₂-NH-R⁵, -NH-SO₂-R⁷, -N(SO₂-R⁷)₂, -N(SO₂-R⁷)(CO-R⁵),
n für die Zahlen 0, 1 oder 2 steht,
Q¹ für O oder S steht und
Q² für O, S, NH oder N-Alkyl mit 1 bis 4 Kohlenstoffatomen steht,
R⁵ für Wasserstoff, für gegebenenfalls durch Cyano, Halogen oder C₁-C₄-Alkoxy substituiertes Alkyl mit 1 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Aryl oder Arylalkyl mit 6 oder 10 Kohlenstoffatomen in der Arylgruppe und gegebenenfalls 1 bis 4 Kohlenstoffatomen im Alkylteil, oder für gegebenenfalls durch Halogen, C₁-C₄-Alkyl, C₁-C₄-Halogenalkyl, C₁-C₄-Alkoxy oder C₁-C₄-Halogenalkoxy substituiertes Heterocyclyl steht, wobei als Heterocyclylgruppen Furyl, Tetrahydrofuryl, Thienyl, Pyridyl und Pyrimidinyl bevorzugt sind,
R⁶ für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, oder für jeweils gegebenenfalls durch Cyano, Nitro, Halogen, C₁-C₄-Alkyl, C₁-C₄-Halogenallcyl, C₁-C₄-Allcoxy oder C₁-C₄-Halogenalkoxy substituiertes Aryl oder Arylalkyl mit 6 oder 10 Kohlenstoffatomen in der Arylgruppe und gegebenenfalls 1 bis 4 Kohlenstoffatomen im Alkylteil steht,
R⁷ für gegebenenfalls durch Halogen substituiertes Alkyl mit 1 bis 6 Kohlenstoffatomen, für jeweils gegebenenfalls durch Halogen substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl, C₁-C₄-Alkylsulfonyl oder C₁-C₄-Alkoxy-carbonyl substituiertes Aryl oder Arylalkyl mit 6 oder 10 Kohlenstoffatomen in der Arylgruppe und gegebenenfalls 1 bis 4 Kohlenstoffatomen im Alkylteil, oder für gegebenenfalls durch Halogen, C₁-C₄-Alkyl, C₁-C₄-Halogenalkyl, C₁-C₄-Alkoxy oder C₁-C₄-Halogenalkoxy substituiertes Heterocyclyl steht, wobei als Heterocyclylgruppen Pyridyl und Pyrimidinyl bevorzugt sind, und
Z für eine der nachstehenden heterocyclischen Gruppierungen steht, 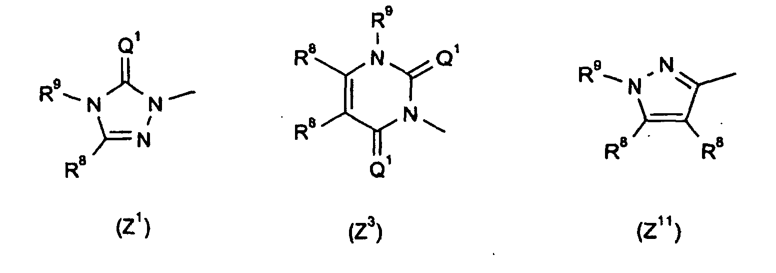 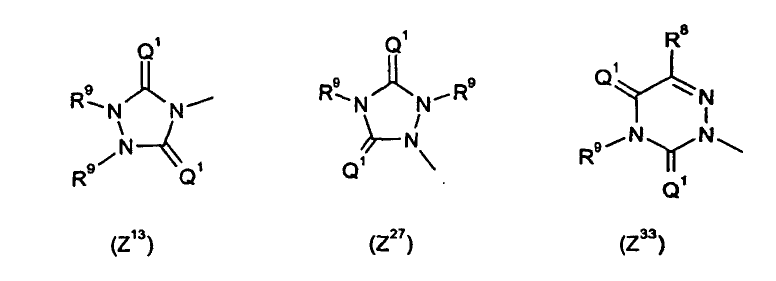
wobei jeweils
Q¹ und Q² die oben angegebene Bedeutung haben,
R⁸ für Wasserstoff, Amino, Nitro, Cyano, Carboxy, Carbamoyl, Thiocarbamoyl, Halogen, für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkoxy substituiertes Alkoxy oder Alkoxycarbonyl mit jeweils 1 bis 6 Kohlenstoffatomen in den Alkylgruppen, für jeweils gegebenenfalls durch Halogen substituiertes Alkenyloxy oder Alkinyloxy mit jeweils 3 bis 6 Kohlenstoffatomen, für gegebenenfalls durch Cyano, Halogen oder C₁-C₄-Alkoxy substituiertes Alkylthio mit 1 bis 6 Kohlenstoffatomen, für jeweils gegebenenfalls durch Halogen substituiertes Alkenylthio oder Alkinylthio mit jeweils 3 bis 6 Kohlenstoffatomen, für jeweils gegebenenfalls durch Cyano, Halogen oder C₁-C₄-Alkoxy substituiertes Alkylamino oder Dialkylamino mit jeweils 1 bis 6 Kohlenstoffatomen in den Alkylgruppen, oder für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht, und
R⁹ für Wasserstoff Hydroxy, Amino, Cyano, für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für gegebenenfalls durch Cyano, Halogen oder C₁-C₄-Alkoxy substituiertes Alkoxycarbonyl, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Cyano, Nitro, Halogen, C₁-C₄-Alkyl, C₁-C₄-Halogenalkyl, C₁-C₄-Alkoxy und/oder C₁-C₄-Halogenalkoxy substituiertes Phenyl oder Phenyl-C₁-C₄-alkyl steht,
wobei gegebenenfalls zwei benachbarte Reste - R⁸ und R⁸, R⁹ und R⁹ oder R⁸ und R⁹ - zusammen für Alkandiyl (Alkylen) oder Oxaalkandiyl mit jeweils 2 bis 6 Kohlenstoffatomen stehen, und
wobei die einzelnen Reste R⁸ und R⁹ - soweit sie mehr als einmal in der gleichen heterocyclischen Gruppierung stehen, die gleiche oder verschiedene Bedeutungen im Rahmen der obigen Definition haben können.

2. Aromatische Aminoverbindungen der allgemeinen Formel (I) gemäß Anspruch 1, in welcher
R¹ für Wasserstoff, Hydroxy, Amino, für Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino oder Dimethylamino, oder für eine der nachstehenden Gruppierungen steht,
-CQ¹-R⁵, -CQ¹-Q²-R⁶,
R² für eine der nachstehenden Gruppierungen steht,
-CQ¹-R⁵, -CQ¹-Q²-R⁶,
R³ für Wasserstoff, Hydroxy, Amino, Carboxy, Cyano, Carbamoyl, Thiocarbamoyl, Nitro, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methoxycarbonyl, Ethoxycarbonyl, Methylthio, Ethylthio, n- oder i-Propylthio, n-, i-, s- oder t-Butylthio, Methylsulfinyl, Ethylsulfinyl, Methylsulfonyl oder Ethylsulfonyl, oder für eine der nachstehenden Gruppierungen steht,
-SO₂-NH-R⁵, -NH-SO₂-R⁷, -N(SO₂-R⁷)₂, -N(SO₂-R⁷)(CO-R⁵),
R⁴ für Wasserstoff, Hydroxy, Amino, Carboxy, Cyano, Carbamoyl, Thiocarbamoyl, Nitro, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methoxycarbonyl, Ethoxycarbonyl, Methylthio, Ethylthio, n- oder i-Propylthio, n-, i-, s- oder t-Butylthio, Methylsulfinyl, Ethylsulfinyl, Methylsulfonyl oder Ethylsulfonyl, oder für eine der nachstehenden Gruppierungen steht,
-SO₂-NH-R⁵, -NH-SO₂-R⁷, -N(SO₂-R⁷)₂, -N(SO₂-R⁷)(CO-R⁵),
n für die Zahlen 0, 1 oder 2 steht,
Q¹ für O oder S steht und
Q² für O, S, NH oder N-Methyl steht,
R⁵ für Wasserstoff, für gegebenenfalls durch Cyano, Fluor, Chlor, Brom, Methoxy oder Ethoxy substituiertes Methyl, Ethyl, n- oder i-Propyl, n-, i-, s- oder t-Butyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Phenyl oder Benzyl, oder für gegebenenfalls durch Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Furyl, Tetrahydrofuryl, Thienyl oder Pyridyl steht,
R⁶ für gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Phenyl oder Benzyl steht,
R⁷ für gegebenenfalls durch Fluor, Chlor oder Brom substituiertes Methyl, Ethyl, n- oder i-Propyl, n-, i-, s- oder t-Butyl, für jeweils gegebenenfalls durch Fluor, Chlor oder Brom substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, n-, i-, s- oder t-Butylthio, Methylsulfinyl, Ethylsulfinyl, Methylsulfonyl, Ethylsulfonyl, Methoxycarbonyl oder Ethoxy-carbonyl substituiertes Phenyl oder Benzyl, oder für gegebenenfalls durch Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Trifluormethoxy substituiertes Pyridyl oder Pyrimidinyl steht und
Z für eine der nachstehenden heterocyclischen Gruppierungen steht. 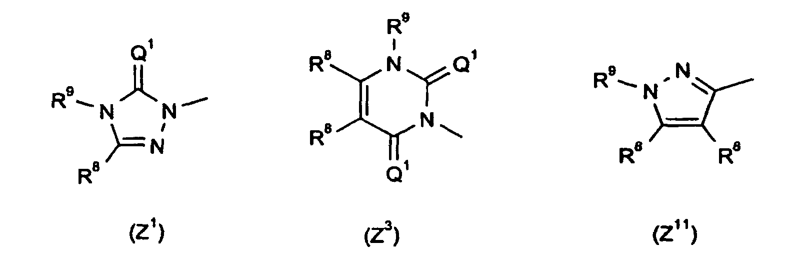 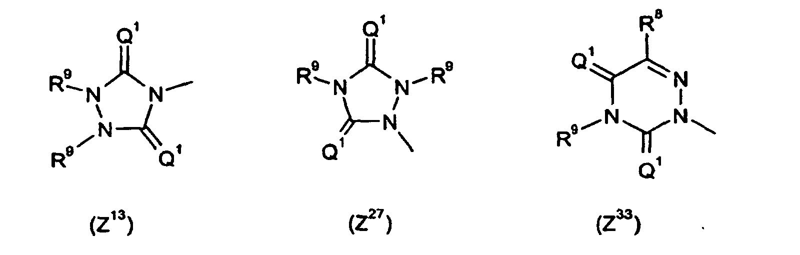
wobei jeweils
Q¹ und Q² die oben angegebene Bedeutung haben,
R⁸ für Wasserstoff, Amino, Nitro, Cyano, Carboxy, Carbamoyl, Thiocarbamoyl, Fluor, Chlor, Brom, für gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, Methoxycarbonyl oder Ethoxycarbonyl, für jeweils gegebenenfalls durch Fluor oder Chlor substituiertes Propenyloxy, Butenyloxy, Propinyloxy oder Butinyloxy, für gegebenenfalls durch Cyano, Fluor, Chlor, Methoxy oder Ethoxy substituiertes Methylthio, Ethylthio, n- oder i-Propylthio, n-, i-, s- oder t-Butylthio, für jeweils gegebenenfalls durch Fluor oder Chlor substituiertes Propenylthio, Butenylthio, Propinylthio oder Butinylthio, für jeweils gegebenenfalls durch Cyano, Fluor, Chlor, Methoxy oder Ethoxy substituiertes Methylamino, Ethylamino, n- oder i-Propylamino, n-, i-, s- oder t-Butylamino, Dimethylamino oder Diethylamino, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht, und
R⁹ für Wasserstoff, Hydroxy, Amino, Cyano, für gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für gegebenenfalls durch Cyano, Fluor, Chlor, Methoxy oder Ethoxy substituiertes Methoxycarbonyl oder Ethoxycarbonyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Cyano, Nitro, Fluor, Chlor, Brom, Methyl, Ethyl, n- oder iPropyl, n-, i-, s- oder t-Butyl, Trifluormethyl, Methoxy, Ethoxy, n- oder i-Propoxy, n-, i-, s- oder t-Butoxy, Difluormethoxy und/oder Trifluormethoxy substituiertes Phenyl oder Benzyl steht,
wobei gegebenenfalls zwei benachbarte Reste - R⁸ und R⁸, R⁹ und R⁹ oder R⁸ und R⁹ - zusammen für Ethan-1,2-diyl (Dimethylen), Propan-1,3-diyl (Trimethylen), Butan-1,4-diyl (Tetramethylen) oder 1-Oxabutan-1,4-diyl stehen, und
wobei die einzelnen Reste R⁸ und R⁹ - soweit sie mehr als einmal in der gleichen heterocyclischen Gruppierung stehen, die gleiche oder verschiedene Bedeutungen im Rahmen der obigen Definition haben können.

3. Verfahren zur Herstellung der aromatischen Aminoverbindungen der allgemeinen Formel (I), 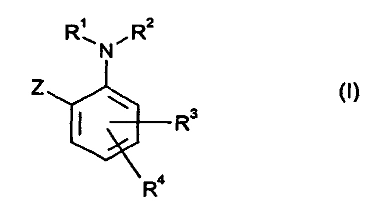 in welcher
R¹, R², R³, R⁴ und Z die in Anspruch 1 genannten Bedeutungen haben,
**dadurch gekennzeichnet, dass** man aromatische Aminoverbindungen der allgemeinen Formel (II), 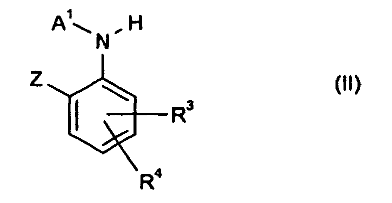 in welcher
R³, R⁴ und Z die in Anspruch 1 angegebene Bedeutung haben und
A¹ für Wasserstoff, Hydroxy, Amino, Alkyl, Alkoxy, Alkylamino oder Dialkylamino steht,
mit elektrophilen Verbindungen der allgemeinen Formel (III),
X-R² (III)
in welcher
R² die in Anspruch 1 angegebene Bedeutung hat und
X für Halogen steht,
umsetzt,
und die auf diese Weise erhaltenen Verbindungen der allgemeinen Formel (I), gegebenenfalls nach üblichen Methoden, in andere Verbindungen der allgemeinen Formel (I) gemäß der obigen Definition umwandelt.

4. Aromatische Aminoverbindungen der allgemeinen Formel (II), 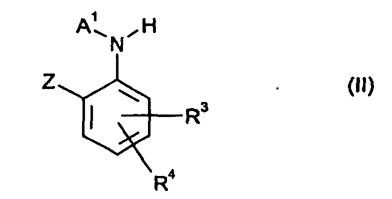 **dadurch gekennzeichnet, dass**
R³, R⁴ und Z die in Anspruch 1 genannten Bedeutungen haben und
A¹ für Wasserstoff, Hydroxy, Amino, Alkyl, Alkoxy, Alkylamino oder Dialkylamino steht.

5. Herbizide Mittel, **gekennzeichnet durch** einen Gehalt an mindestens einer aromatischen Aminoverbindung der Formel (I) gemäß dem Anspruch 1.

6. Verfahren zur Bekämpfung von unerwünschten Pflanzen, **dadurch gekennzeichnet, dass** man aromatische Aminoverbindungen der Formel (I) gemäß dem Anspruch 1 auf unerwünschte Pflanzen und/oder ihren Lebensraum einwirken läßt.

7. Verwendung von aromatischen Aminoverbindungen der Formel (I) gemäß dem Anspruch 1 zur Bekämpfung von unerwünschten Pflanzen.

8. Verfahren zur Herstellung von herbiziden Mitteln, **dadurch gekennzeichnet, dass** man aromatische Aminoverbindungen der Formel (I) gemäß dem Anspruch 1 mit Streckmitteln und/oder oberflächenaktiven Substanzen vermischt.

## Claims

1. Aromatic amino compounds of the general formula (I), 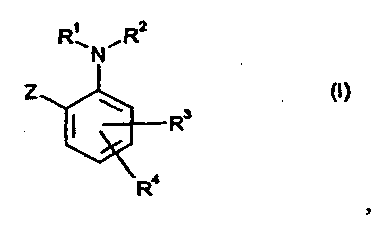 in which
R¹ represents hydrogen, hydroxyl, amino, or represents alkyl, alkoxy, alkylamino or dialkylamino having in each case 1 to 6 carbon atoms in the alkyl groups, or represents one of the groups which follow,
-CQ¹-R⁵, -CQ1-Q²-R⁶,
R² represents one of the groups which follow,
-CQ¹-R⁵, -CQ¹-Q²-R⁶,
R³ represents hydrogen, hydroxyl, amino, carboxyl, cyano, carbamoyl, thiocarbamoyl, nitro, halogen or represents alkyl, alkoxy, alkoxycarbonyl, alkylthio, alkylsulphinyl or alkylsulphonyl, each of which has 1 to 6 carbon atoms in the alkyl groups and each of which is optionally substituted by cyano, halogen, or C₁-C₄-alkoxy, or one of the groups which follow,
-SO₂-NH-R⁵, -NH-SO₂-R⁷, -N(SO₂-R⁷)₂, -N(SO₂-R⁷)(CO-R⁵),
R⁴ represents hydrogen, hydroxyl, amino, carboxyl, cyano, carbamoyl, thiocarbamoyl, nitro, halogen or represents alkyl, alkoxy, alkoxycarbonyl, alkylthio, alkylsulphinyl or alkylsulphonyl, each of which has 1 to 6 carbon atoms in the alkyl groups and each of which is optionally substituted by cyano, halogen, or C₁-C₄-alkoxy,or one of the groups which follow,
-SO₂-NH-R⁵, -NH-SO₂-R⁷, -N(SO₂-R⁷)₂, -N(SO₂-R⁷)(CO-R⁵),
n represents the numbers 0, 1 or 2,
Q¹ represents O or S and
Q² represents O, S, NH or N-alkyl having 1 to 4 carbon atoms,
R⁵ represents hydrogen, or represents alkyl which has 1 to 6 carbon atoms and which is optionally substituted by cyano, halogen, or C₁-C₄-alkoxy, or represents cycloalkyl or cycloalkylalkyl, each of which has 3 to 6 carbon atoms in the cycloalkyl group and if appropriate 1 to 4 carbon atoms in the alkyl moiety and each of which is optionally substituted by cyano, halogen, or C₁-C₄-alkyl, or represents aryl or arylalkyl, each of which has 6 or 10 carbon atoms in the aryl group and, if appropriate, 1 to 4 carbon atoms in the alkyl moiety and each of which is optionally substituted by cyano, nitro, halogen, C₁-C₄-alkyl C₁-C₄-haloalkyl, C₁-C₄-alkoxy or C₁₋C₄-haloalkoxy, or represents heterocyclyl which is optionally substituted by halogen, C₁-C₄-alkyl, C₁-C₄-haloalkyl, C₁-C₄-alkoxy or C₁-C₄-haloalkoxy, preferred heterocyclyl groups being furyl, tetrahydrofuryl, thienyl, pyridyl and pyrimidinyl,
R⁶ represents alkyl which has 1 to 6 carbon atoms and which is optionally substituted by cyano, halogen or C₁-C₄-alkoxy, or represents alkenyl or alkynyl, each of which has 2 to 6 carbon atoms and each of which is optionally substituted by halogen, or represents cycloalkyl or cycloalkylalkyl, each of which has 3 to 6 carbon atoms in the cycloalkyl group and, if appropriate, I to 4 carbon atoms in the alkyl moiety and each of which is optionally substituted by cyano, halogen or C₁-C₄-alkyl, or represents aryl or arylalkyl, each of which has 6 or 10 carbon atoms in the aryl group and, if appropriate, 1 to 4 carbon atoms in the alkyl moiety and each of which is optionally substituted by cyano, nitro, halogen, C₁₋C₄-alkyl, C₁-C₄-haloalkyl, C₁-C₄-alkoxy or C₁-C₄-haloalkoxy,
R⁷ represents alkyl which has 1 to 6 carbon atoms and which is optionally substituted by halogen, or represents cycloalkyl or cycloalkylalkyl, each of which has 3 to 6 carbon atoms in the cycloalkyl group and, if appropriate, 1 to 4 carbon atoms in the alkyl moiety and each of which is optionally substituted by halogen, or represents aryl or arylalkyl, of which has 6 or 10 carbon atoms in the aryl group and, if appropriate, 1 to 4 carbon atoms in the alkyl moiety and each of which is optionally substituted by cyano, nitro, halogen, C₁-C₄-alkyl, C₁-C₄-haloalkyl, C₁-C₄-alkoxy, C₁-C₄-haloalkoxy, C₁-C₄-alkylthio, C₁-C₄-alkylsulphinyl, C₁₋C₄₋alkylsulphonyl or C₁-C₄-alkoxycarbonyl, or represents heterocyclyl which is optionally substituted by halogen, C₁-C₄-alkyl, C₁-C₄-haloalkyl, C₁-C₄-alkoxy or C₁₋C₄-haloalkoxy, preferred heterocyclyl groups being pyridyl and pyrimidinyl, and
Z represents one of the heterocyclic groups which follow 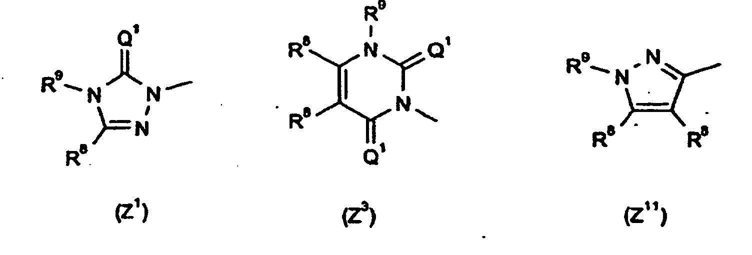 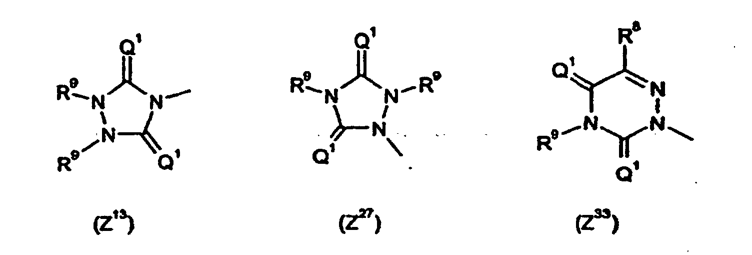 where in each case
Q¹ and Q² have the abovementioned meaning,
R⁸ represents hydrogen, amino, nitro, cyano, carboxyl, carbamoyl, thiocarbamoyl, halogen, or represents alkyl which has 1 to 6 carbon atoms and which is optionally substituted by cyano, halogen or C₁-C₄-alkoxy, or represents alkenyl or alkynyl, each of which has 2 to 6 carbon atoms and each of which is optionally substituted by halogen, or represents alkoxy or alkoxycarbonyl, each of which has 1 to 6 carbon atoms in the alkyl groups and each of which is optionally substituted by cyano, halogen or C₁-C₄-alkoxy, or represents alkenyloxy or alkynyloxy, each of which has 3 to 6 carbon atoms and each of which is optionally substituted by halogen, or represents alkylthio which has 1 to 6 carbon atoms and which is optionally substituted by cyano, halogen or C₁-C₄-alkoxy, or represents alkylthio or alkynylthio, each of which has 3 to 6 carbon atoms and each of which is optionally substituted by halogen, or represents alkylamino or dialkylamino, each of which has 1 to 6 carbon atoms in the alkyl groups and each of which is optionally substituted by cyano, halogen or C₁-C₄-alkoxy, or represents cycloalkyl or cycloalkylalkyl, each of which has 3 to 6 carbon atoms in the cycloalkyl groups and, if appropriate, 1 to 4 carbon atoms in the alkyl moiety and each of which is optionally substituted by cyano, halogen or C₁-C₄-alkyl, and
R⁹ represents hydrogen, hydroxyl, amino, cyano, or represents alkyl which has 1 to 6 carbon atoms and which is optionally substituted by cyano, halogen or C₁-C₄-alkoxy, or represents alkenyl or alkynyl, each of which has 2 to 6 carbon atoms and each of which is optionally substituted by halogen, or represents alkoxycarbonyl which is optionally substituted by cyano, halogen or C₁-C₄-alkoxy, or represents cycloalkyl or cycloalkylalkyl, each of which has 3 to 6 carbon atoms in the cycloalkyl groups and, if appropriate, 1 to 4 carbon atoms in the alkyl moiety and each of which is optionally substituted by cyano, halogen or C₁-C₄-alkyl, or represents phenyl or phenyl-C₁-C₄-alkyl, each of which is optionally substituted by cyano, nitro, halogen, C₁-C₄-alkyl, C₁-C₄-haloalkyl, C₁-C₄-alkoxy and/or C₁-C₄-haloalkoxy,
where, if appropriate, two adjacent radicals - R⁸ and R⁸, R⁹ and R⁹ or R⁸ and R⁹ - together represent alkanediyl (alkylene) or oxaalkanediyl having in each case 2 to 6 carbon atoms, and
where the individual radicals R⁸ and R⁹ - if they occur more than once in the same heterocyclic group, may have identical or different meanings within the scope of the above definition.

2. Aromatic amino compounds of the general formula (I) according to Claim 1, in which
R¹ represents hydrogen, hydroxyl, amino, or represents methyl, ethyl, n- or i-propyl n-, i-, s- or t-butyl, methoxy, ethoxy, n- or i-propoxy, n-, i-, s- or t-butoxy, methylamino, ethylamino, n- or i-propylamino, n-, i-, s- or t-butylamino or dimethylamino, or represents one of the groups which follow,
-CQ¹-R⁵, -CQ¹-Q²-R⁶,
R² represents one of the groups which follow,
-CQ¹-R⁵, -CQ¹-Q²-R⁶,
R³ represents hydrogen, hydroxyl, amino, carboxyl, cyano, carbamoyl, thiocarbamoyl, nitro, fluorine, chlorine, bromine, or represents methyl, ethyl, n- or i-propyl, n-, i-, s- or t-butyl, methoxy, ethoxy, n- or i-propoxy, n-, i-, s- or t-butoxy, methoxycarbonyl, ethoxycarbonyl, methylthio, ethylthio, n- or i-propylthio, n-, i-, s- or t-butylthio, methylsulphinyl, ethylsulphinyl, methylsulphonyl or ethylsulphonyl, each of which is optionally substituted by cyano, fluorine, chlorine, methoxy or ethoxy, or represents one of the groups which follow,
-SO₂-NH-R⁵, -NH-SO₂-R⁷, -N(SO₂-R²)₂, -N(SO₂-R⁷)(CO-R⁵),
R⁴ represents hydrogen, hydroxyl, amino, carboxyl, cyano, carbamoyl, thiocarbamoyl, nitro, fluorine, chlorine, bromine, or represents methyl, ethyl, n- or i-propyl, n-, i-, s- or t-butyl, methoxy, ethoxy, n- or i-propoxy, n-, i-, s- or t-butoxy, methoxycarbonyl, ethoxycarbonyl, methylthio, ethylthio, n- or i-propylthio, n-, i-, s- or t-butylthio, methylsulphinyl, ethylsulphinyl, methylsulphonyl or ethylsulphonyl, each of which is optionally substituted by cyano, fluorine, chlorine, methoxy or ethoxy, or represents one of the groups which follow,
-SO₂-NH-R⁵, -NH-SO₂-R⁷, -N(SO₂-R⁷)₂, -N(SO₂-R⁷)(CO-R⁵),
n represents the numbers 0, 1 or 2,
Q¹ represents O or S and
Q² represents O, S, NH or N-methyl,
R⁵ represents hydrogen, or represents methyl, ethyl, n- or i-propyl, n-, i-, s- or t-butyl, each of which is optionally substituted by cyano, fluorine, chlorine, bromine, methoxy or ethoxy, or represents cyclopropyl, cyclobutyl, cyclopentyl, cyclohexyl, cyclopropylmethyl, cyclobutylmethyl, cyclopentylmethyl or cyclohexylmethyl, each of which is optionally substituted by cyano, fluorine, chlorine, bromine, methyl or ethyl, or represents phenyl or benzyl, each of which is optionally substituted by cyano, nitro, fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxyl, difluoromethoxy or trifluoromethoxy, or represents furyl, tetrahydrofuryl, thienyl or pyridyl, each of which is optionally substituted by fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy or trifluoromethoxy,
R⁶ represents methyl, ethyl, n- or i-propyl, n-, i-, s- or t-butyl, each of which is optionally substituted by cyano, fluorine, chlorine, methoxy or ethoxy, or represents propenyl, butenyl, propynyl or butynyl, each of which is optionally substituted by fluorine, chlorine or bromine, or represents cyclopropyl, cyclobutyl, cyclopentyl, cyclohexyl, cyclopropylmethyl, cyclobutylmethyl, cyclopentylmethyl or cyclohexylmethyl, each of which is optionally substituted by cyano, fluorine, chlorine, bromine, methyl or ethyl, or represents phenyl or benzyl, each of which is optionally substituted by cyano, nitro, fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy or trifluoromethoxy,
R⁷ represents methyl, ethyl, n- or i-propyl, n-, i-, s- or t-butyl, each of which is optionally substituted by fluorine, chlorine or bromine, or represents cyclopropyl, cyclobutyl, cyclopentyl, cyclohexyl, cyclopropylmethyl, cyclobutylmethyl, cyclopentylmethyl or cyclohexylmethyl, each of which is optionally substituted by fluorine, chlorine or bromine, or represents phenyl or benzyl, each of which is optionally substituted by cyano, nitro, fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy, trifluoromethoxy, methylthio, ethylthio, n- or i-propylthio, n-, i-, s- or t-butylthio, methylsulphinyl, ethylsulphinyl, methylsulphonyl, ethylsulphonyl, methoxycarbonyl or ethoxycarbonyl, or represents pyridyl or pyrimidinyl, each of which is optionally substituted by fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy or trifluoromethoxy, and
Z represents one of the heterocyclic groups which follow, 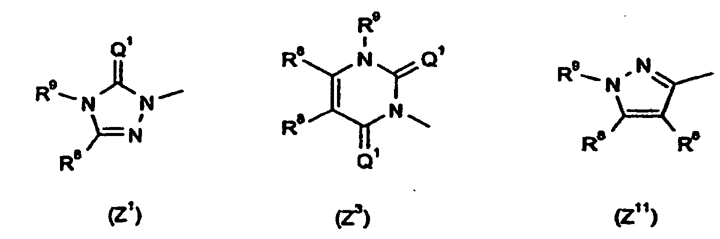 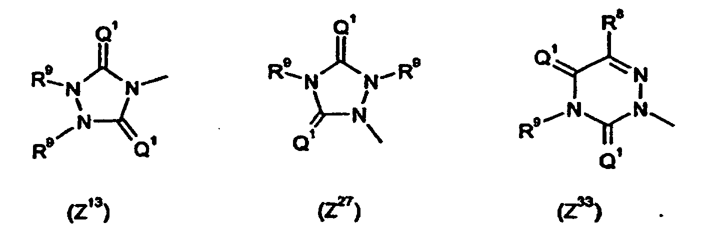 where in each case
Q¹ and Q² have the abovementioned meaning,
R⁸ represents hydrogen, amino, nitro, cyano, carboxyl, carbamoyl, thiocarbamoyl, fluorine, chlorine, bromine, or represents methyl, ethyl, n- or i-propyl, n-, i-, s- or t-butyl, each of which is optionally substituted by cyano, fluorine, chlorine, methoxy or ethoxy, or represents propenyl, butenyl, propynyl or butynyl, each of which is optionally substituted by fluorine, chlorine or bromine, or represents methoxy, ethoxy, n- or i-propoxy, n-, i-, s- or t-butoxy, methoxycarbonyl or ethoxycarbonyl, each of which is optionally substituted by cyano, fluorine, chlorine, methoxy or ethoxy, or represents propenyloxy, butenyloxy, propynyloxy or butynyloxy, each of which is optionally substituted by fluorine or chlorine, or represents methylthio, ethylthio, n- or i-propylthio, n-, i-, s- or t-butylthio, each of which is optionally substituted by cyano, fluorine, chlorine, methoxy or ethoxy, or represents propenylthio, butenylthio, propynylthio or butynylthio, each of which is optionally substituted by fluorine or chlorine, or represents methylamino, ethylamino, n- or i-propylamino, n-, i-, s- or t-butylamino, dimethylamino or diethylamino, each of which is optionally substituted by cyano, fluorine, chlorine, methoxy or ethoxy, or represents cyclopropyl, cyclobutyl, cyclopentyl cyclohexyl, cyclopropylmethyl, cyclobutylmethyl, cyclopentylmethyl or cyclohexylmethyl, each of which is optionally substituted by cyano, fluorine, chlorine, bromine, methyl or ethyl, and
R⁹ represents hydrogen, hydroxyl, amino, cyano, or represents methyl, ethyl, n- or i-propyl, n-, i-, s- or t-butyl, each of which is optionally substituted by cyano, fluorine, chlorine, methoxy or ethoxy, or represents propenyl, butenyl, propynyl or butynyl, each of which is optionally substituted by fluorine, chlorine or bromine, or represents methoxycarbonyl or ethoxycarbonyl, each of which is optionally substituted by cyano, fluorine, chlorine, methoxy or ethoxy, or represents cyclopropyl, cyclobutyl, cyclopentyl cyclohexyl, cyclopropylmethyl, cyclobutylmethyl, cyclopentylmethyl or cyclohexylmethyl, each of which is optionally substituted by cyano, fluorine, chlorine, bromine, methyl or ethyl, or represents phenyl or benzyl, each of which is optionally substituted by cyano, nitro, fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy and/or trifluoromethoxy,
where, if appropriate, two adjacent radicals - R⁸ and R⁹, R⁹ and R⁹ or R⁸ and R⁹ - together represent ethane-1,2-diyl (dimethylene), propane-1,3-diyl (trimethylene), butane-1,4-diyl (tetramethylene) or 1-oxabutane-1,4-diyl, and
where the individual radicals R⁸ and R⁹ - if they occur more than once in the same heterocyclic group, may have identical or different meanings within the scope of the above definition.

3. Process for the preparation of the aromatic amino compounds of the general formula (I), 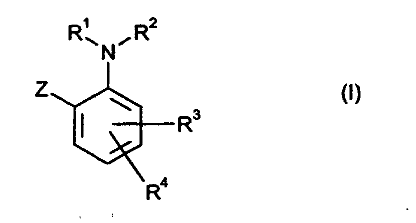 in which
R¹, R², R³, R⁴ and Z have the meanings given in Claim 1, **characterized in that**
aromatic amino compounds of the general formula (II) 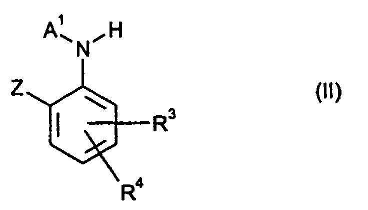 in which
R³, R⁴ and Z have the meaning given in claim 1 and
A¹ represents hydrogen, hydroxyl, amino, alkyl, alkoxy, alkylamino or dialkylamino
are reacted with electrophilic compounds of the general formula (III)
X-R² (III)
in which
R² has the meaning given in Claim 1 and
X represents halogen,
and the resulting compounds of the general formula (I) are converted into other compounds of the general formula (I) in accordance with the above definition, if appropriate by customary methods.

4. Aromatic amino compounds of the general formula (II), 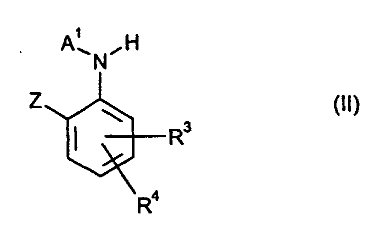 **characterized in that**
R³, R⁴ and Z have the meanings given in Claim 1 and
A¹ represents hydrogen, hydroxyl, amino, alkyl, alkoxy, alkylamino or dialkylamino.

5. Herbicidal compositions, **characterized in that** they comprise at least one aromatic amino compound of the formula (I) according to Claim 1.

6. Method of controlling undesired plants, **characterized in that** aromatic amino compounds of the formula (I) according to Claim 1 are allowed to act on undesired plants and/or their environment.

7. Use of aromatic amino compounds of the formula (I) according to Claim 1 for controlling undesired plants.

8. Process for the preparation of herbicidal compositions, **characterized in that** aromatic amino compounds of the formula (I) according to Claim 1 are mixed with extenders and/or surfactants.

## Revendications

1. Composés aromatiques aminés de formule générale (I), 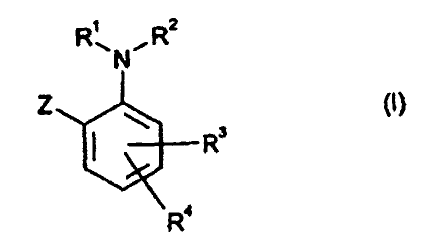 dans laquelle
R¹ représente l'hydrogène, un groupe hydroxy, amino, un reste alkyle, alkoxy, alkylamino ou dialkylamino ayant chacun 1 à 6 atomes de carbone dans les groupes alkyle, ou l'un des groupements suivants,
-CQ¹-R⁵, -CQ¹-Q²-R⁶,
R² représente l'un des groupements suivants,
-CQ¹-R⁵, -CQ¹-Q²-R⁶,
R³ représente l'hydrogène, un groupe hydroxy, amino, carboxy, cyano, carbamoyle, thiocarbamoyle, nitro, un halogène, un reste alkyle, alkoxy, alkoxycarbonyle, alkylthio, alkylsulfinyle ou alkylsulfonyle ayant chacun 1 à 6 atomes de carbone dans les groupes alkyle et chacun étant éventuellement substitué par un radical cyano, halogéno ou alkoxy en C₁ à C₄, ou l'un des groupements suivants,
-SO₂-NH-R⁵, -NH-SO₂-R⁷, -N(SO₂-R⁷)₂, -N(SO₂-R⁷)(CO-R⁵),
R⁴ représente l'hydrogène, un groupe hydroxy, amino, carboxy, cyano, carbamoyle, thiocarbamoyle, nitro, un halogène, un reste alkyle, alkoxy, alkoxycarbonyle, alkylthio, alkylsulfinyle ou alkylsulfonyle ayant chacun 1 à 6 atomes de carbone dans les groupes alkyle et chacun étant éventuellement substitué par un radical cyano, halogéno ou alkoxy en C₁ à C₄, ou l'un des groupements suivants,
SO₂-NH-R⁵, -NH-SO₂-R⁷, -N(SO₂-R⁷)₂, -N(SO₂-R⁷)(CO-R⁵),
n représente les nombres 0, 1 ou 2,
Q¹ représente O ou S, et
Q² représente O, S, NH ou un reste N-alkyle ayant 1 à 4 atomes de carbone,
R⁵ représente l'hydrogène, un reste alkyle ayant 1 à 6 atomes de carbone éventuellement substitué par un radical cyano, halogéno ou alkoxy en C₁ à C₄, un reste cycloalkyle ou cycloalkylalkyle ayant chacun 3 à 6 atomes de carbone dans le groupe cycloalkyle et, le cas échéant, 1 à 4 atomes de carbone dans la partie alkyle et chacun étant éventuellement substitué par un radical cyano, halogéno ou alkyle en C₁ à C₄, un reste aryle ou arylalkyle ayant 6 ou 10 atomes de carbone dans le groupe aryle et, le cas échéant, 1 à 4 atomes de carbone dans la partie alkyle et chacun étant éventuellement substitué par un radical cyano, nitro, halogéno, alkyle en C₁ à C₄, halogénalkyle en C₁ à C₄, alkoxy en C₁ à C₄ ou halogénalkoxy en C₁ à C₄, ou un reste hétérocyclyle éventuellement substitué par un radical halogéno, alkyle en C₁ à C₄, halogénalkyle en C₁ à C₄, alkoxy en C₁ à C₄ ou halogénalkoxy en C₁ à C₄, les restes hétérocyclyle appréciés étant les groupes furyle, tétrahydrofuryle, thiényle, pyridyle ou pyrimidinyle,
R⁶ représente un reste alkyle ayant 1 à 6 atomes de carbone éventuellement substitué par un radical cyano, halogéno ou alkoxy en C₁ à C₄, un reste alcényle ou alcynyle ayant chacun 2 à 6 atomes de carbone et chacun étant éventuellement substitué par un halogène, un reste cycloalkyle ou cycloalkylalkyle ayant chacun 3 à 6 atomes de carbone dans le groupe cycloalkyle et, le cas échéant, 1 à 4 atomes de carbone dans la partie alkyle et chacun étant éventuellement substitué par un radical cyano, halogéno ou alkyle en C₁ à C₄, ou bien un reste aryle ou arylalkyle ayant 6 ou 10 atomes de carbone dans le groupe aryle et, le cas échéant, 1 à 4 atomes de carbone dans la partie alkyle et chacun étant éventuellement substitué par un radical cyano, nitro, halogéno, alkyle en C₁ à C₄, halogénalkyle en C₁ à C₄, alkoxy en C₁ à C₄ ou halogénalkoxy en C₁ à C₄,
R⁷ est un reste alkyle ayant 1 à 6 atomes de carbone éventuellement substitué par un halogène, un reste cycloalkyle ou cycloalkylalkyle ayant chacun 3 à 6 atomes de carbone dans le groupe cycloalkyle et, le cas échéant, 1 à 4 atomes de carbone dans la partie alkyle et chacun étant éventuellement substitué par un radical halogéno, un reste aryle ou arylalkyle ayant 6 ou 10 atomes de carbone dans le groupe aryle et, le cas échéant, 1 à 4 atomes de carbone dans la partie alkyle et chacun étant éventuellement substitué par un radical cyano, nitro, halogéno, alkyle en C₁ à C₄, halogénalkyle en C₁ à C₄, alkoxy en C₁ à C₄, halogénalkoxy en C₁ à C₄, alkylthio en C₁ à C₄, alkylsulfinyle en C₁ à C₄, alkylsulfonyle en C₁ à C₄ ou (alkoxy en C₁ à C₄)-carbonyle, ou un reste hétérocyclyle éventuellement substitué par un radical halogéno, alkyle en C₁ à C₄, halogénalkyle en C₁ à C₄, alkoxy en C₁ à C₄ ou halogénalkoxy en C₁ à C₄, des restes hétérocyclyle appréciés étant les groupes pyridyle et pyrimidinyle, et
Z représente l'un des groupements hétérocycliques suivants : 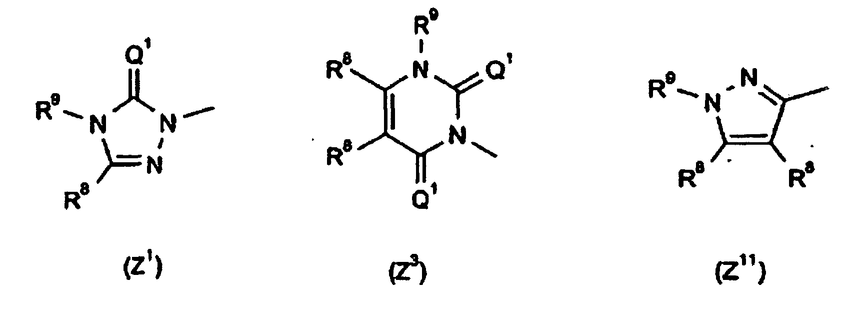 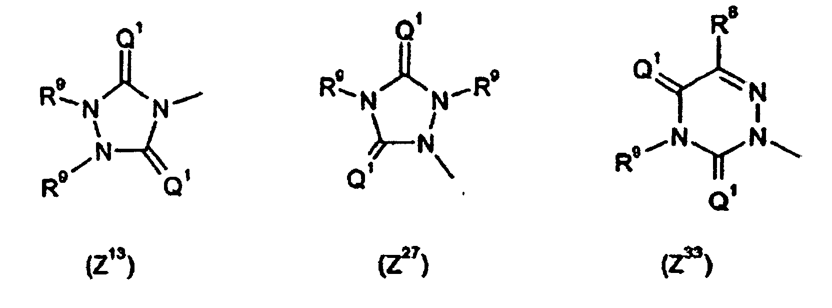
où, dans chaque cas
Q¹ et Q² ont la définition indiquée ci-dessus,
R⁸ représente l'hydrogène, un groupe amino, nitro, cyano, carboxy, carbamoyle, thiocarbamoyle, un halogène, un reste alkyle ayant 1 à 6 atomes de carbone éventuellement substitué par un radical cyano, halogéno ou alkoxy en C₁ à C₄, un reste alcényle ou alcynyle ayant chacun 2 à 6 atomes de carbone et chacun étant éventuellement substitué par un halogène, un reste alkoxy ou alkoxycarbonyle ayant chacun 1 à 6 atomes de carbone dans les groupes alkyle et chacun étant éventuellement substitué par un radical cyano, halogéno ou alkoxy en C₁ à C₄, un reste alcényloxy ou alcynyloxy ayant chacun 3 à 6 atomes de carbone et chacun étant éventuellement substitué par un radical halogéno, un reste alkylthio ayant 1 à 6 atomes de carbone éventuellement substitué par un radical cyano, halogèno ou alkoxy en C₁ à C₄, un reste alcénylthio ou alcynylthio ayant chacun 3 à 6 atomes de carbone et chacun étant éventuellement substitué par un halogène, un reste alkylamino ou dialkylamino ayant chacun 1 à 6 atomes de carbone dans les groupes alkyle et chacun étant éventuellement substitué par un radical cyano, halogéno ou alkoxy en C₁ à C₄, ou bien un reste cycloalkyle ou cycloalkylalkyle ayant chacun 3 à 6 atomes de carbone dans les groupes cycloalkyle et, le cas échéant, 1 à 4 atomes de carbone dans la partie alkyle et chacun étant éventuellement substitué par un radical cyano, halogéno ou alkyle en C₁ à C₄, et
R⁹ représente l'hydrogène, un groupe hydroxy, amino, cyano, un reste alkyle ayant 1 à 6 atomes de carbone éventuellement substitué par un radical cyano, halogéno ou alkoxy en C₁ à C₄, un reste alcényle ou alcynyle ayant 2 à 6 atomes de carbone et chacun étant éventuellement substitué par un halogène, un reste alkoxycarbonyle éventuellement substitué par un radical cyano, halogéno ou alkoxy en C₁ à C₄, un reste cycloalkyle ou cycloalkylalkyle ayant chacun 3 à 6 atomes de carbone dans les groupes cycloalkyle et, le cas échéant, 1 à 4 atomes de carbone dans la partie alkyle et chacun étant éventuellement substitué par un radical cyano, halogéno ou alkyle en C₁ à C₄, ou bien un reste phényle ou phényl-(alkyle en C₁ à C₄), chacun étant éventuellement substitué par un radical cyano, nitro, halogéno, alkyle en C₁ à C₄, halogénalkyle en C₁ à C₄, alkoxy en C₁ à C₄ et/ou halogénalkoxy en C₁ à C₄, deux restes contigus - R⁸ et R⁸, R⁹ et R⁹ ou R⁸ et R⁹ - formant éventuellement ensemble un reste alcanediyl(alkylène) ou oxaalcanediyle ayant chacun 2 à 6 atomes de carbone, et
les restes R⁸ et R⁹ individuels - dans la mesure où ils sont plus d'une fois présents dans le même groupement hétérocyclique - pouvant avoir la même signification ou des significations différentes dans le cadre de la définition donnée ci-dessus.

2. Composés aromatiques aminés de formule (I) suivant la revendication 1, formule dans laquelle
R¹ représente l'hydrogène, un groupe hydroxy, amino, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylamino, éthylamino, n-propylamino, isopropylamino, n-butylamino, isobutylamino, sec.-butylamino, tertiobutylamino ou diméthylamino, ou représente l'un des groupements suivants,
-CQ¹-R⁵, -CQ¹-Q²-R⁶,
R² représente l'un des groupements suivants,
-CQ¹-R⁵, -CQ¹-Q²-R⁶,
R³ représente l'hydrogène, un groupe hydroxy, amino, carboxy, cyano, carbamoyle, thiocarbamoyle, nitro, le fluor, le chlore, le brome, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthoxycarbonyle, éthoxycarbonyle, méthylthio, éthylthio, n-propylthio, isopropylthio, n-butylthio, isobutylthio, sec.-butylthio, tertiobutylthio, méthylsulfinyle, éthylsulfinyle, méthylsulfonyle ou éthylsulfonyle chacun éventuellement substitué par un radical cyano, fluoro, chloro, méthoxy ou éthoxy, ou l'un des groupements suivants,
-SO₂-NH-R⁵, -NH-SO₂-R⁷, -N(SO₂-R⁷)₂, -N(SO₂-R⁷)(CO-R⁵),
R⁴ représente l'hydrogène, un groupe hydroxy, amino, carboxy, cyano, carbamoyle, thiocarbamoyle, nitro, le fluor, le chlore, le brome, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthoxycarbonyle, éthoxycarbonyle, méthylthio, éthylthio, n-propylthio, isopropylthio, n-butylthio, isobutylthio, sec.-butylthio, tertiobutylthio, méthylsulfinyle, éthylsulfinyle, méthylsulfonyle ou éthylsulfonyle chacun éventuellement substitué par un radical cyano, fluoro, chloro, méthoxy ou éthoxy, ou représente l'un des groupements suivants,
-SO₂-NH-R⁵, -NH-SO₂-R⁷, -N(SO₂-R⁷)₂, -N(SO₂-R⁷)(CO-R⁵),
n représente les nombres 0, 1 ou 2,
Q¹ représente O ou S et
Q² représente O, S, NH ou un reste N-méthyle,
R⁵ représente l'hydrogène, un reste éthyle, n-propyle, isopropyle, n-butyle, isobutyle, sec.-butyle, tertiobutyle ou méthyle éventuellement substitué par un radical cyano, fluoro, chloro, bromo, méthoxy ou éthoxy, un reste cyclopropyle, cyclobutyle, cyclopentyle, cyclohexyle, cyclopropylméthyle, cyclobutylméthyle, cyclopentylméthyle ou cyclohexylméthyle chacun éventuellement substitué par un radical cyano, fluoro, chloro, bromo, méthyle ou éthyle, un reste phényle ou benzyle chacun éventuellement substitué par un radical cyano, nitro, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy ou trifluorométhy, ou un reste furyle, tétrahydrofuryle, thiényle ou pyridyle chacun éventuellement substitué par un radical fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy ou trifluorométhoxy,
R⁶ représente un reste éthyle, n-propyle, isopropyle, n-butyle, isobutyle, sec.-butyle, tertiobutyle ou méthyle éventuellement substitué par un radical cyano, fluoro, chloro, méthoxy ou éthoxy, un reste propényle, butényle, propinyle ou butinyle chacun éventuellement substitué par un radical fluoro, chloro ou bromo, un reste cyclopropyle, cyclobutyle, cyclopentyle, cyclohexyle, cyclopropylméthyle, cyclobutylméthyle, cyclopentylméthyle ou cyclohexylméthyle chacun éventuellement substitué par un radical cyano, fluoro, chloro, bromo, méthyle ou éthyle, ou un reste phényle ou benzyle chacun éventuellement substitué par un radical cyano, nitro, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy ou trifluorométhoxy,
R⁷ est un reste éthyle, n-propyle, isopropyle, n-butyle, isobutyle, sec.-butyle, tertiobutyle ou méthyle éventuellement substitué par un radical fluoro, chloro ou bromo, un reste cyclopropyle, cyclobutyle, cyclopentyle, cyclohexyle, cyclopropylméthyle, cyclobutylméthyle, cyclopentylméthyle ou cyclohexylméthyle chacun éventuellement substitué par un radical fluoro, chloro ou bromo, un reste phényle ou benzyle chacun éventuellement substitué par un radical cyano, nitro, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy, trifluorométhoxy, méthylthio, éthylthio, n-propylthio, isopropylthio, n-butylthio, isobutylthio, sec.-isobutylthio, tertiobutylthio, méthylsulfinyle, éthylsulfinyle, méthylsulfonyle, éthylsulfonyle, méthoxycarbonyle ou éthoxycarbonyle, ou un reste pyridyle ou pyrimidinyle chacun éventuellement substitué par un radical fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy ou trifluorométhoxy, et
Z représente l'un des groupements hétérocycliques suivants : 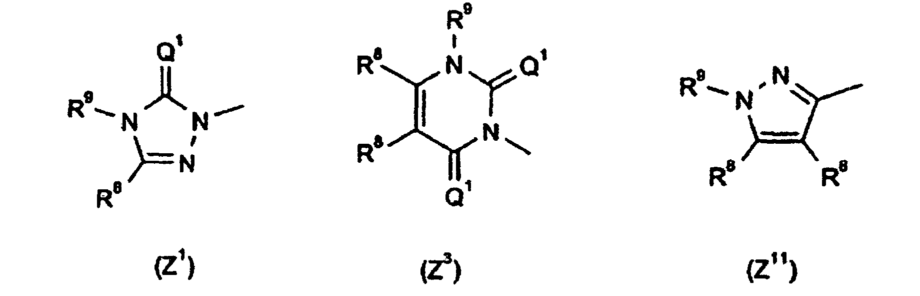 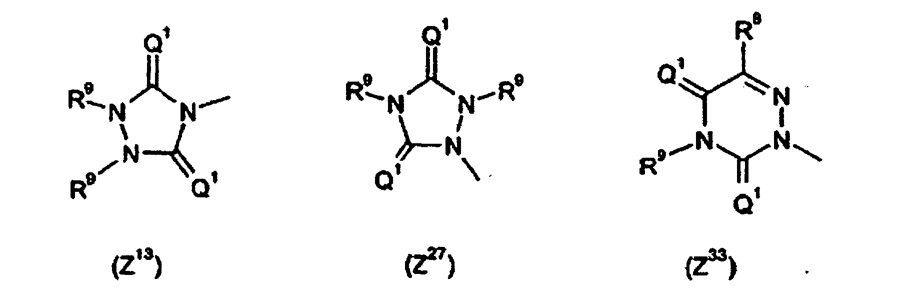
où, dans chaque cas
Q¹ et Q² ont la définition indiquée ci-dessus,
R⁸ représente l'hydrogène, un groupe amino, nitro, cyano, carboxy, carbamoyle, thiocarbamoyle, le fluor, le chlore, le brome, un reste éthyle, n-propyle, isopropyle, n-butyle, isobutyle, sec.-butyle, tertiobutyle ou méthyle éventuellement substitué par un radical cyano, fluoro, chloro, méthoxy ou éthoxy, un reste propényle, butényle, propinyle ou butinyle chacun éventuellement substitué par un radical fluoro, chloro ou bromo, un reste méthoxy, éthoxy, n-propoxy, isopropoxy, n-butoxy, isobutoxy, sec.-butoxy, tertiobutoxy, méthoxycarbonyle ou éthoxycarbonyle chacun éventuellement substitué par un radical cyano, fluoro, chloro, méthoxy ou éthoxy, un reste propényloxy, butényloxy, propinyloxy ou butinyloxy chacun éventuellement substitué par du fluor ou du chlore, un reste méthylthio, éthylthio, n-propylthio, isopropylthio, n-butylthio, isobutylthio, sec.-butylthio ou tertiobutylthio chacun éventuellement substitué par un radical cyano, fluoro, chloro, méthoxy ou éthoxy, un reste propénylthio, buténylthio, propinylthio ou butinylthio chacun éventuellement substitué par du fluor ou du chlore, un reste méthylamino, éthylamino, n-propylamino, isopropylamino, n-butylamino, isobutylamino, sec.-butylamino, tertiobutylamino, diméthylamino ou diéthylamino chacun éventuellement substitué par un radical cyano, fluoro, chloro, méthoxy ou éthoxy, ou un reste cyclopropyle, cyclobutyle, cyclopentyle, cyclohexyle, cyclopropylméthyle, cyclobutylméthyle, cyclopentylméthyle ou cyclohexylméthyle chacun éventuellement substitué par un radical cyano, fluoro, chloro, bromo, méthyle ou éthyle, et
R⁹ représente l'hydrogène, un groupe hydroxy, amino, cyano, un reste éthyle, n-propyle, isopropyle, n-butyle, isobutyle, sec.-butyle, tertiobutyle ou méthyle éventuellement substitué par un radical cyano, fluoro, chloro, méthoxy ou éthoxy, un reste propényle, butényle, propinyle ou butinyle chacun éventuellement substitué par du fluor, du chlore ou du brome, un reste éthoxycarbonyle ou méthoxycarbonyle éventuellement substitué par un radical cyano, fluoro, chloro, méthoxy ou éthoxy, un reste cyclopropyle, cyclobutyle, cyclopentyle, cyclohexyle, cyclopropylméthyle, cyclobutylméthyle, cyclopentylméthyle ou cyclohexylméthyle chacun éventuellement substitué par un radical cyano, fluoro, chloro, bromo, méthyle ou éthyle, ou un reste phényle ou benzyle chacun éventuellement substitué par un radical cyano, nitro, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy et/ou trifluorométhoxy,
deux restes contigus - R⁸ et R⁸, R⁹ et R⁹ ou R⁸ et R⁹ - formant éventuellement ensemble un reste éthane-1,2-diyl(diméthylène), propane-1,3-diyl(triméthylène), butane-1,4-diyl(tétraméthylène) ou 1-oxabutane-1,4-diyle, et
les restes R⁸ et R⁹ individuels - dans la mesure où ils sont présents plus d'une fois dans le même groupement hétérocyclique - pouvant avoir la même signification ou des significations différentes dans le cadre de la définition indiquée ci-dessus.

3. Procédé de production des composés aromatiques aminés de formule (I), 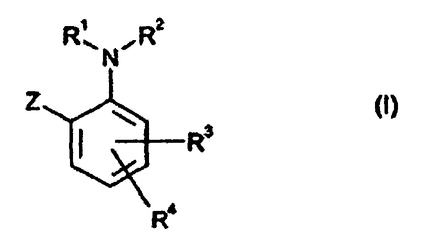 dans laquelle
R¹, R², R³, R⁴ et Z ont les définitions indiquées dans la revendication 1,
**caractérisé en ce qu'**on fait réagir des composés aromatiques aminés de formule (II), 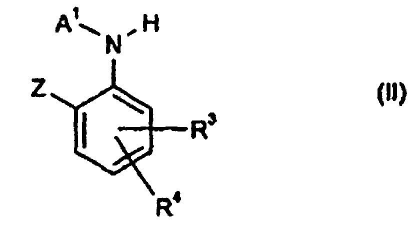 dans laquelle
R³, R⁴ et Z ont la définition indiquée dans la revendication 1, et
A¹ représente l'hydrogène, un groupe hydroxy, amino, un reste alkyle, alkoxy, alkylamino ou dialkylamino,
avec des composés électrophiles de formule (III),
**X-R**² **(III)**
dans laquelle
R² a la définition indiquée dans la revendication 1 et
X représente un halogène,
et on transforme éventuellement selon des modes opératoires usuels, les composés de formule (I) obtenues de la manière indiquée, en d'autres composés de formule (I) selon la définition ci-dessus.

4. Composés aromatiques aminés de formule (II), 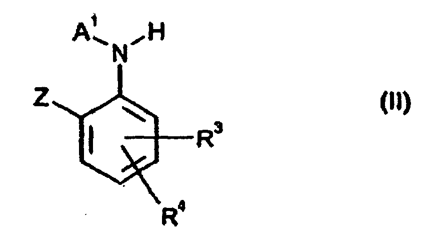 **caractérisés en ce que**
R³, R⁴ et Z ont les définitions indiquées dans la revendication 1 et
A¹ représente l'hydrogène, un groupe hydroxy, amino, un reste alkyle, alkoxy, alkylamino ou dialkylamino.

5. Compositions herbicides, **caractérisées par** une teneur en au moins un composé aromatique aminé de formule (I) suivant la revendication 1.

6. Procédé pour combattre des plantes indésirables, **caractérisé en ce qu'**on fait agir des composés aromatiques aminés de formule (I) suivant la revendication 1 sur des plantes indésirables et/ou sur leur milieu.

7. Utilisation de composés aromatiques aminés de formule (I) suivant la revendication 1 pour combattre des plantes indésirables.

8. Procédé de préparation de compositions herbicides, **caractérisé en ce qu'**on mélange des composés aromatiques aminés de formule (I) suivant la revendication 1 avec des diluants et/ou des substances tensioactives.
